# EUROPEAN PATENT SPECIFICATION

(11) **EP 2 114 158 B1**
(45) Date of publication and mention of the grant of the patent: **08.08.2012**
(21) Application number: 07868092.3
(22) Date of filing: 28.12.2007
(51) Int. Cl.: C07D 487/04, C07D 498/04, C07D 498/14, A61K 31/53, A61P 25/16, A61P 25/18, A61P 25/28

(54) **3-SUBSTITUTED-[1,2,3]BENZOTRIAZINONE COMPOUNDS FOR ENHANCING GLUTAMATERGIC SYNAPTIC RESPONSES**
3-SUBSTITUIERTE [1,2,3]BENZOTRIAZINON-VERBINDUNGEN FÜR DIE VERBESSERUNG DER GLUTAMATERGEN SYNAPSENREAKTIONEN
COMPOSÉS [1,2,3]BENZOTRIAZINONE SUBSTITUÉS EN POSITION 3 DESTINÉS À AMÉLIORER LES RÉPONSES SYNAPTIQUES GLUTAMATERGIQUES

(30) Priority: 03.01.2007 US 878626 P
(43) Date of publication of application: 11.11.2009
(73) Proprietor: CORTEX PHARMACEUTICALS, INC., Irvine, CA 92718 (US)
(72) Inventor: MUELLER, Rudolf, Foothill Ranch, CA 92610 (US); LEE, Stephen, San Gabriel, CA 91775 (US); O'HARE, Sean, Carlsbad, CA 92009 (US); ROGERS, Gary, Laguna Beach, CA 92651 (US); RACHWAL, Stan, Irvine, CA 92614 (US); STREET, Leslie, Newport Beach, CA 92660 (US)
(74) Representative: Dall'Olio, Giancarlo
(86) International application number: PCT/US2007/026415
(87) International publication number: WO 2008/085505

(56) References cited:
- WO-A1-97/36907
- WO-A1-99/33469
- WO-A1-03/099299
- WO-A2-2009/038752
- US-A- 3 723 436
- US-A- 5 962 477
- US-B1- 6 303 542
- GOUAUX ET AL.: 'Structure and function of AMPA receptors' J. PHYSIOL. vol. 554, 2003, pages 249 - 253, XP008111964
- GUEYRARD ET AL.: 'A new and rapid access to homochiral 2,3-dihydro-oxazolo[2,3-b]quinazolin-5-ones ' TETRAHEDRON: ASYMMETRY vol. 12, 2001, pages 337 - 340, XP004230916
- MURRAY ET AL.: 'LY503430, a novel AMPA receptor potentiator with functional, neuroprotective and neurotrophic effects in rodent models of Parkinson's disease' J. PHARMACOL. EXP. THER. vol. 306, 2003, pages 752 - 762, XP008111966
- RUSSELL: 'Increased AMPA Receptor Function in Slices Containing the Prefrontal Cortex of Spontaneously Hypertensive Rats' METABOLIC BRAIN DISEASE vol. 16, 2001, pages 143 - 149, XP008111965
- PONTARELLI: 'New drug that enhances glutamate transmission in brain being evaluated for fragile X', [Online] 2002, XP008116236 Retrieved from the Internet: <URL:http://www.innovations-report.com/html /reports/medicine-health/report-12386.html>
- REN: 'Ampakines Alleviate Respiratory Depression in Rats' AM. J. RESPIR. CRIT. CARE MED. vol. 174, 2006, pages 1384 - 1391, XP008104921

## Description

### Field of the Invention

This invention relates to compounds, pharmaceutical compositions as such and for use in the prevention and treatment of cerebral insufficiency, including enhancement of receptor functioning at synapses in brain networks responsible for higher order behaviors. These brain networks, which are involved in cognitive abilities, are related to memory impairment, such as is observed in aging and a variety of dementias, in imbalances in neuronal activity between different brain regions, as is suggested in disorders such as Parkinson's disease, schizophrenia, attention deficit and affective or mood disorders, and in disorders wherein a deficiency in neurotrophic factors is implicated. In a particular aspect, the present invention relates to compounds useful for treatment of such conditions, and for use in such treatment.

### Background of the Invention

The release of glutamate at synapses at many sites in mammalian forebrain stimulates two classes ofpostsynaptic, ionotropic receptors. These classes are usually referred to as AMPA/quisqualate and N-methyl-D-aspartic acid (NMDA) receptors. AMPA/quisqualate receptors mediate a voltage independent fast excitatory post-synaptic current (the fast EPSC), whereas NMDA receptors generate a voltage-dependent, slow excitatory current. Studies carried out in slices of hippocampus or cortex, indicate that the AMPA receptor mediated fast EPSC is generally the dominant component by far at most glutamatergic synapses.

AMPA receptors are not evenly distributed across the brain but rather are largely restricted to the telencephalon and cerebellum. These receptors are found in high concentrations in the superficial layers of neocortex, in each of the major synaptic zones of hippocampus, and in the striatal complex, as reported by Monaghan et al., in Brain Research 324:160-164 (1984). Studies in animals and humans indicate that these structures organize complex perceptual-motor processes and provide the substrates for higher-order behaviors. Thus, AMPA receptors mediate transmission in those brain networks responsible for a host of cognitive activities.

For the reasons set forth above, drugs that modulate and thereby enhance the functioning of AMPA receptors could have significant benefits for cognitive and intellectual performance. Such drugs should also facilitate memory encoding. Experimental studies, such as those reported by Arai and Lynch, Brain Research 598:173-184 (1992), indicate that increasing the size of AMPA receptor-mediated synaptic response(s) enhances the induction of long-term potentiation (LTP). LTP is a stable increase in the strength of synaptic contacts that follows repetitive physiological activity of a type known to occur in the brain during learning.

Compounds that enhance the functioning of the AMPA form of glutamate receptors facilitate the induction of LTP and the acquisition of learned tasks in rodents and humans as measured in a number of paradigms. See, for example, Granger et al., Synapse 15:326-329 (1993); Staubli et al., PNAS 91:777-781 (1994); Arai et al., Brain Res. 638:343-346 (1994); Staubli et al., PNAS 91:11158-11162 (1994); Shors et al., Neurosci. Let. 186:153-156 (1995); Larson et al., J. Neurosci. 15:8023-8030 (1995); Granger et al., Synapse 22:332-337 (1996); Arai et al., JPET 278:627- 638 (1996); Lynch et al., Internat. Clin. Psychopharm. 11: 13-19 (1996); Lynch et al., Exp. Neurology 145:89-92 (1997); Ingvar et al., Exp. Neurology 146:553-559 (1997); Hampson, et al., J. Neurosci. 18:2748-2763 (1998); Porrino et al., PLoS Biol 3(9):1-14 (2006) and Lynch and Rogers, US Patent 5,747,492. There is a considerable body of evidence showing that LTP is a substrate of memory. For example, compounds that block LTP interfere with memory formation in animals, and certain drugs that disrupt learning in humans antagonize the stabilization of LTP, as reported by del Cerro and Lynch, Neuroscience 49: 1-6 (1992). Learning a simple task induces LTP in hippocampus that occludes LTP generated by high frequency stimulation (Whitlock et al., Science 313:1093-1097 (2006)) and a mechanism that maintains LTP sustains spatial memory (Pastalkova, et al., Science 313:1141-1144 (2006)). Of significant importance to the field of learning is the finding that *in vivo* treatments with a positive AMPA-type glutamate receptor modulator restores stabilization of basal dendritic LTP in middle-aged animals (Rex, et al., J. Neurophysiol. 96:677-685 (2006)).
Excitatory synaptic transmission provides a major pathway by which neurotrophic factors are increased within specific brain regions. As such, potentiation of AMPA receptor function by modulators has been found to increase levels of neurotrophins, particularly brain derived neurotrophic factor, or BDNF. See, for example, Lauterborn, et al., J. Neurosci. 20:8-21 (2000); Gall, et al., U.S. Patent 6,030,968; Lauterborn, et al., JPET 307:297-305 (2003); and Mackowiak, et al., Neuropharmacology 43:1-10 (2002). Other studies have linked BDNF levels to a number of neurological disorders, such as Parkinson's disease, Attention Deficit Hyperactivity Disorder (ADHD), autism, Fragile-X Syndrome, and Rett Syndrome (RTT). See, for example, O'Neill, et al., Eur. J. Pharmacol. 486:163-174 (2004); Kent, et al., Mol. Psychiatry 10:939-943 (2005); Riikonen, et al., J. Child Neurol. 18:693-697 (2003) and Chang, et al., Neuron 49:341-348 (2006). Thus, AMPA receptor potentiators may be useful for the treatment of these, as well as other, neurological diseases that are the result of a glutamatergic imbalance or a deficit in neurotrophic factors.

A prototype for a compound that increases AMPA receptor function was described by Ito et al., J. Physiol. 424:533-543 (1990). These authors found that the nootropic drug aniracetam (N-anisoyl-2-pyrrolidinone) increases currents mediated by brain AMPA receptors expressed in Xenopus oocytes without affecting responses by γ-aminobutyric acid (GABA), kainic acid (KA), or NMDA receptors. Infusion of aniracetam into slices of hippocampus was also shown to substantially increase the size of fast synaptic potentials without altering resting membrane properties. It has since been confirmed that aniracetam enhances synaptic responses at several sites in hippocampus, and that it has no effect on NMDA-receptor mediated potentials (Staubli et al., Psychobiology 18:377-381 (1990) and Xiao et al., Hippocampus 1:373-380 (1991)).

Aniracetam has been found to have an extremely rapid onset and washout, and can be applied repeatedly with no apparent lasting effects, which are desirable features for behaviorally-relevant drugs. Aniracetam does present several disadvantages, however. The peripheral administration of aniracetam is not likely to influence brain receptors. The drug works only at high concentrations (approx. 1000 µM), and about 80% of the drug is converted to anisoyl-GABA following peripheral administration in humans (Guenzi and Zanetti, J. Chromatogr. 530:397-406 (1990)). The metabolite, anisoyl-GABA, has been found to have less synaptic activity than aniracetam. In addition to these issues, aniracetam has putative effects on a plethora of other neurotransmitter and enzymatic targets in the brain, which makes uncertain the mechanism of any claimed therapeutic drug effect. See, for example, Himori, et al., Pharmacology Biochemistry and Behavior 47:219-225 (1994); Pizzi et al., J. Neurochem. 61:683-689 (1993); Nakamura and Shirane, Eur. J. Pharmacol. 380: 81-89 (1999); Spignoli and Pepeu, Pharmacol. Biochem. Behav. 27:491-495 (1987); Hall and Von Voigtlander, Neuropharmacology 26:1573-1579(1987); and Yoshimoto et al., J. Pharmacobiodyn. 10:730-735(1987).

A class of AMPA receptor-modulating compounds that does not display the low potency and inherent instability characteristic of aniracetam has been described (Lynch and Rogers, US Patent 5,747,492). These compounds, termed "Ampakines"^{®}, can be substituted benzamides, which include, for example, 1-(quinoxaline-6-ylcarbonyl)piperidine (CX516; Ampalex^{®}). Typically, they are chemically more stable than aniracetam and show improved bioavailability. CX516 is active in animal tests used to detect efficacious drugs for the treatment of memory disorders, schizophrenia, and depression. In three separate clinical trials, CX516 showed evidence for efficacy in improving various forms of human memory (Lynch et al., Internat. Clin. Psychopharm. 11:13-19 (1996); Lynch et al., Exp. Neurology 145:89-92 (1997); Ingvar et al., Exp. Neurology 146:553-559 (1997)).

Another class of Ampakines, benzoxazines, has been discovered to have very high activity in *in vitro* and *in vivo* models for assessing the probability of producing cognition enhancement (Rogers and Lynch; US Patent # 5,736,543). The substituted benzoxazines are rigid benzamide analogues with different receptor modulating properties from the flexible benzamide, CX516.

Certain substituted benzofurazan and benzothiadiazole compounds have been found to be significantly and surprisingly more potent in the animal model of schizophrenia than previous compounds, and are also effective in cognition enhancement. These compounds are structurally similar to those disclosed in Lynch and Rogers, US Patent # 5,736,543.

Previously disclosed structures that contained the 1,3-benzoxazine-4-one pharmacophore were substituted on the benzene portion by heteroatoms, such as nitrogen or oxygen (US Patents # 5,736,543 and 5,962,447), by substituted alkyl groups (US Patents # 5,650,409 and 5,783,587), or unsubstituted (WO 99/42456). Yet another class of 1,3-benzoxazine compounds contained a carbonyl external to the oxazine ring (US Patent # 6,124,278), but not as a substituent on the benzene ring structure.

WO 97/36907 and WO 99/33469 disclose compounds having a 1,3-benzooxazine-3-one scaffold that are useful as AMPA modulators.
WO 2009038752 discloses compounds having the following scaffold: 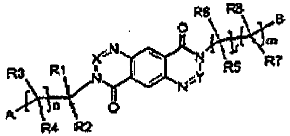 wherein:
X = C-H or N,
Y = C-H or N, with the proviso that Y cannot be carbon when X = N and the group 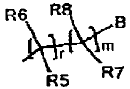 represents H, alkyl or cycloalkyl,
These compounds are useful in the treatment of a mammalian subject, wherein the subject suffers from a hypoglutamatergic condition or a deficiency in the number or strength of excitatory synapses or in the number of AMPA receptors, such that memory or other cognitive functions are impaired or such that a cortical/striatal imbalance occurs leading to schizophrenia or schizophreniform behavior.

Now, a new class of triazinone compounds has been discovered that display significant activity on hippocampal synaptic responses and neuronal whole cell currents mediated by AMPA receptors. 3-Substituted benzo-[1,2,3]-triazin-4-one compounds are potent AMPA receptor modulators that are significantly more metabolically stable than the corresponding bis-benzoxazinones.

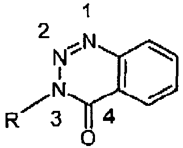

The biological activity of the triazinone compounds of the invention was unexpected and the potency at the AMPA receptor is surprisingly high; the most potent compounds in this class double AMPA receptor currents at concentrations as low as 3 nM. These compounds are disclosed herein.

### Summary of the Invention

The present invention includes, in one aspect, a compound as shown by structure I, and described in Section II of the Detailed Description, which follows. Administration of compounds of this class has been found to increase synaptic responses mediated by AMPA receptors. The compounds of the present invention are significantly and unexpectedly more potent than previously described compounds in increasing AMPA receptor function in primary neuronal cultures and in slices of rat hippocampus, and in enhancing cognitive performance, such as performance in a delayed match to sample task. This unexpected activity translates into pharmaceutical compounds as such and for uses in treatment methods, which utilize significantly lower concentrations (on a mole-to-mole basis) of the present compounds compared to prior art compositions.

The ability of the compounds of the invention to increase AMPA receptor-mediated responses makes the compounds useful for a variety of purposes. These include facilitating the learning of behaviors dependent upon glutamate receptors, treating conditions in which AMPA receptors or synapses utilizing these receptors are reduced in numbers or efficiency, and enhancing excitatory synaptic activity in order to restore an imbalance between brain subregions or increase the levels of neurotrophic factors.

In another aspect, the invention includes compounds for use in the treatment of a mammalian subject suffering from a hypoglutamatergic condition, or from a deficiency in the number or strength of excitatory synapses, or in the number of AMPA receptors, such that memory or other cognitive functions are impaired. Such conditions may also cause a cortical/striatal imbalance, leading to schizophrenia or schizophreniform behavior. According to the claimed compounds for use in the method, such a subj ect is treated with an effective amount of a compound as shown by structure I, and described in Section II of the Detailed Description, following, in a pharmaceutically acceptable carrier.

In another aspect, the invention includes claimed compounds for use in reducing or inhibiting respiratory depression in a subject having respiratory depression, comprising administering to the subject an amount of a compound of the invention, the amount being sufficient to reduce or inhibit respiratory depression. In one embodiment of the invention, the subject is a mammal. In another embodiment, the subject is a human.

These and other objects and features of the invention will become more fully apparent when the following detailed description of the invention is read in conjunction with the accompanying drawings.

### Detailed Description of the Invention

### I. Definitions

The terms below have the following definitions unless indicated otherwise. Other terms that are used to describe the present invention have the same definitions as those terms are generally used by those skilled in the art.

The term "alkyl," is used herein to refer to a fully saturated monovalent radical containing up to 12 carbons (preferably up to 7 carbons) and hydrogen, and which may be a straight chain or branched. Examples of alkyl groups are methyl, ethyl, n-butyl, n-heptyl, isopropyl, 2-methylpropyl.

The term "cycloalkyl" is used herein to refer to a fully saturated monovalent radical containing up to 8 carbons and hydrogen in a ring. Examples of cycloalkyl groups are cyclopropyl, cyclobutyl, cyclopentyl, cyclohexyl and cycloheptyl.

The term "alkenyl" is used herein to refer to a monovalent radical containing carbon and hydrogen that contains one or two sites of unsaturation, and which may be a straight chain, branched or cyclic. Examples of alkenyl groups are ethenyl, n-butenyl, n-heptenyl, and isopropenyl. Examples of cycloalkenyl are cyclopentenyl, cyclopentenylethyl and cyclohexenyl.

The term "alkynyl," is used herein to refer to a monovalent radical containing carbon and hydrogen that contains a triple bond. Examples of alkynyl groups are ethynyl, n-butynyl, n-heptynyl, propargyl, propynyl.

The term "substituted alkyl" refers to alkyl as just described including one or more functional groups such as lower alkyl (containing 1-6 carbon atoms), aryl, substituted aryl, acyl, halogen (i.e., alkyl halos, e.g., CF₃), hydroxy, alkoxy, alkoxyalkyl, amino, alkyl and dialkyl amino, acylamino, acyloxy, aryloxy, aryloxyalkyl, carboxyalkyl, carboxamido, thio, thioethers, both saturated and unsaturated cyclic hydrocarbons, heterocycles and the like.

The term "aryl" refers to a substituted or unsubstituted monovalent aromatic radical having a single ring (e.g., phenyl) or multiple condensed rings (e.g., naphthyl). Other examples include heterocyclic aromatic ring groups having one or more nitrogen, oxygen, or sulfur atoms in the ring, such as oxazolyl, isoxazolyl, pyrazolyl, thiazolyl, thiadiazolyl, tetrazolyl, pyridazinyl, pyrimidyl, benzofuryl, benzothienyl, benzimidazolyl, benzoxazolyl, benzothiazolyl, quinolyl, isoquinolyl, imidazolyl, furyl, pyrrolyl, pyridyl, thienyl and indolyl.

The term "substituted" as used in the term "substituted aryl, substituted aromatic, substituted heteroaryl, or substituted heteroaromatic" herein signifies that one or more substituents other than H may be present, said substituents being selected from atoms and groups, which when present do not prevent the compound from functioning as a potentiator of AMPA receptor function. Examples of substituents that may be present in a substituted aromatic or heteroaromatic group include, but are not limited to, groups such as (C₁-C₇) alkyl, (C₁-C₇) acyl, aryl, heteroaryl, substituted aryl and heteroaryl, halogen, cyano, nitro, (C₁-C₇) alkylhalos (e.g., CF₃), hydroxy, (C₁-C₇) alkoxy, (C₂-C₁₄, preferably C₂-C₇) alkoxyalkyl, amino, alkyl and dialkyl amino, acylamino, acyloxy, aryloxy, aryloxyalkyl, carboxyalkyl, carboxamido, thio, thioethers, both saturated and unsaturated (C₃-C₈) cyclic hydrocarbons, (C₃-C₈) heterocycles and the like.

"Heterocycle" or "heterocyclic" refers to a carbocylic ring wherein one or more carbon atoms have been replaced with one or more heteroatoms such as nitrogen, oxygen or sulfur. Examples of heterocycles include, but are not limited to, piperidine, pyrrolidine, morpholine, thiomorpholine, piperazine, tetrahydrofuran, tetrahydropyran, 2-pyrrolidinone, δ-velerolactam, δ-velerolactone and 2-ketopiperazine.

The term "substituted heterocycle" refers to a heterocycle as just described that contains one or more functional groups (other than H) such as lower alkyl, acyl, aryl, cyano, halogen, hydroxy, alkoxy, alkoxyalkyl, amino, alkyl and dialkyl amino, acylamino, acyloxy, aryloxy, aryloxyalkyl, carboxyalkyl, carboxamido, thio, thioethers, both saturated and unsaturated cyclic hydrocarbons, heterocycles and the like.

The term "compound" is used herein to refer to any specific chemical compound disclosed herein. Within its use in context, the term generally refers to a single compound, but in certain instances may also refer to stereoisomers and/or optical isomers (including racemic mixtures, enantiomerically enriched mixtures or enantiomerically pure compounds) of disclosed compounds. Where relevant, the term compound also includes pharmaceutically acceptable salts and solvates, thereof.

The term "effective amount" refers to the amount of a compound or a component which is provided within the context of its use, and, with respect to preferred embodiments including a selected compound of formula I, the amount that is used to enhance glutamatergic synaptic response by increasing AMPA receptor activity. The precise amount used will vary depending upon the particular compound selected and its intended use, the age and weight of the subject, route of administration, and so forth, but may be easily determined by routine experimentation. In the case of the treatment of a condition or disease state, an effective amount is that amount which is used to effectively treat the particular condition or disease state.

The term "pharmaceutically acceptable carrier" refers to a carrier or excipient which is not unacceptably toxic to the subject to which it is administered. Pharmaceutically acceptable excipients are described at length by E.W. Martin, in "Remington's Pharmaceutical Sciences."

A "pharmaceutically acceptable salt" of an amine compound, such as those contemplated in the current invention, is an ammonium salt having as counterion an inorganic anion such as chloride, bromide, iodide, sulfate, sulfite, nitrate, nitrite, phosphate, and the like, or an organic anion such as acetate, malonate, pyruvate, propionate, fumarate, cinnamate, tosylate, and the like.

The term "patient" or "subject" is used throughout the specification to describe an animal, generally a mammalian animal, including a human, to whom treatment or use with the compounds or compositions according to the present invention is provided. For treatment or use with/or of those conditions or disease states which are specific for a specific animal (especially, for example, a human subject or patient), the term patient or subject refers to that particular animal.

The term "sensory motor problems" is used to describe a problem which arises in a patient or subject from the inability to integrate external information derived from the five known senses in such a way as to direct appropriate physical responses involving movement and action.

The term "cognitive task" or "cognitive function" is used to describe an endeavor or process by a patient or subject that involves thought or knowing. The diverse functions of the association cortices of the parietal, temporal and frontal lobes, which account for approximately 75% of all human brain tissue, are responsible for much of the information processing that goes on between sensory input and motor output. The diverse functions of the association cortices are often referred to as cognition, which literally means the process by which we come to know the world. Selectively attending to a particular stimulus, recognizing and identifying these relevant stimulus features and planning and experiencing the response are some of the processes or abilities mediated by the human brain which are related to cognition.

The term "brain network" is used to describe different anatomical regions of the brain that communicate with one another via the synaptic activity of neuronal cells.

The term "AMPA receptor" refers to an aggregate of proteins found in some membranes, which allows positive ions to cross the membrane in response to the binding of glutamate or AMPA (DL-α-amino-3-hydroxy-5-methyl-4-isoxazolepropionic acid), but not NMDA.

The term "excitatory synapse" is used to describe a cell-cell junction at which release of a chemical messenger by one cell causes depolarization of the external membrane of the other cell. An excitatory synapse describes a postsynaptic neuron which has a reversal potential that is more positive than the threshold potential and consequently, in such a synapse, a neurotransmitter increases the probability that an excitatory post synaptic potential will result (a neuron will fire producing an action potential). Reversal potentials and threshold potentials determine postsynaptic excitation and inhibition. If the reversal potential for a post synaptic potential ("PSP") is more positive than the action potential threshold, the effect of a transmitter is excitatory and produces an excitatory post synaptic potential ("EPSP") and the firing of an action potential by the neuron. If the reversal potential for a post synaptic potential is more negative than the action potential threshold, the transmitted is inhibitory and may generate inhibitory post synaptic potentials (IPSP), thus reducing the likelihood that a synapse will fire an action potential. The general rule for postsynaptic action is: if the reversal potential is more positive than threshold, excitation results; inhibition occurs if the reversal potential is more negative than threshold. See, for example, Chapter 7, NEUROSCIENCE, edited by Dale Purves, Sinauer Associates, Inc., Sunderland, MA 1997.

The term "motor task" is used to describe an endeavor taken by a patient or subject that involves movement or action.

The term "perceptual task" is used to describe an act by a patient or subj ect of devoting attention to sensory inputs.

The term "synaptic response" is used to describe biophysical reactions in one cell as a consequence of the release of chemical messengers by another cell with which it is in close contact.

The term "hypoglutamatergic condition" is used to describe a state or condition in which transmission mediated by glutamate (or related excitatory amino acids) is reduced to below normal levels. Transmission consists of the release of glutamate, binding to post synaptic receptors, and the opening of channels integral to those receptors. The end point of the hypoglutamatergic condition is reduced excitatory post synaptic current. It can arise from any of the three above noted phases of transmission. Conditions or disease states which are considered hypoglutamatergic conditions and which can be treated using the compounds, compositions and compounds for use in treatments according to the present invention include, for example, loss of memory, dementia, depression, attention disorders, sexual dysfunction, movement disorders, including Parkinson's disease, schizophrenia or schizophreniform behavior, memory and learning disorders, including those disorders which result from aging, trauma, stroke and neurodegenerative disorders, such as those associated with drug-induced states, neurotoxic agents, Alzheimer's disease, and aging. These conditions are readily recognized and diagnosed by those of ordinary skill in the art.

The term "cortico-striatal imbalance" is used to describe a state in which the balance of neuronal activities in the interconnected cortex and underlying striatal complex deviates from that normally found. 'Activity' can be assessed by electrical recording or molecular biological techniques. Imbalance can be established by applying these measures to the two structures or by functional (behavioral or physiological) criteria.

The term "affective disorder" or "mood disorder" describes the condition when sadness or elation is overly intense and continues beyond the expected impact of a stressful life event, or arises endogenously. As used herein, the term "effective disorder" embraces all types of mood disorders as described in, for example; Diagnostic and Statistical Manual of Mental Disorders, Fourth Edition (DSM IV), pages 317-391.

The term "schizophrenia" is used to describe a condition which is a common type of psychosis, characterized by a disorder in the thinking processes, such as delusions and hallucinations, and extensive withdrawal of the individual's interest from other people and the outside world, and the investment of it in his or her own. Schizophrenia is now considered a group of mental disorders rather than a single entity, and distinction is made between reactive and process schizophrenias. As used herein, the term schizophrenia or "schizophreniform" embraces all types of schizophrenia, including ambulatory schizophrenia, catatonic schizophrenia, hebephrenic schizophrenia, latent schizophrenia, process schizophrenia, pseudoneurotic schizophrenia, reactive schizophrenia, simple schizophrenia, and related psychotic disorders which are similar to schizophrenia, but which are not necessarily diagnosed as schizophrenia *per se.* Schizophrenia and other psychotic disorders may be diagnosed using guidelines established in, for example, Diagnostic and Statistical Manual of Mental Disorders, Fourth Edition (DSM IV) Sections 293.81, 293.82, 295.10, 295.20, 295.30, 295.40, 295.60, 295.70, 295.90, 297.1, 297.3, 298.8.

The term "brain function" is used to describe the combined tasks of perceiving, integrating, filtering and responding to external stimuli and internal motivational processes.

The term "impaired" is used to describe a function working at a level that is less than normal. Impaired functions can be significantly impacted such that a function is barely being carried out, is virtually non-existent or is working in a fashion that is significantly less than normal. Impaired functions may also be sub-optimal. The impairment of function will vary in severity from patient to patient and the condition to be treated.

The term "respiratory depression" as used herein refers to a variety of conditions characterized by reduced respiratory frequency and inspiratory drive to cranial and spinal motor neurons. Specifically, respiratory depression refers to conditions where the medullary neural network associated with respiratory rhythm generating activity does not respond to accumulating levels of PCO₂ (or decreasing levels of PO₂) in the blood and subsequently understimulates motorneurons controlling lung musculature.

### II. Compounds of the Present Invention

The present invention is directed, in one aspect, to compounds having the property of enhancing AMPA receptor functions. These are compounds having the structure of formula I, below: 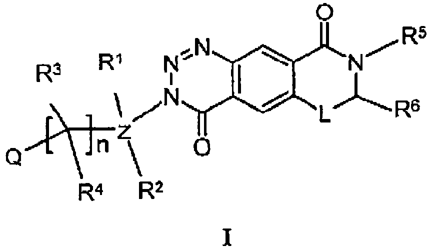 wherein:
R¹ and R² are independently hydrogen, alkyl, substituted alkyl, cycloalkyl, alkynyl, substituted alkynyl, cyano, alkoxy, carboxamido, substituted carboxamido, and if R¹ and R² are alkyl, R¹ and R² may be joined with a bond or -(CH₂)ₘ- to produce a cycloalkane ring,
R³ and R⁴ are independently hydrogen, alkyl, hydroxyl, alkoxy, cyano, fluoro, and if R³ and R⁴ are alkyl, R³ and R⁴ may be joined with a bond or -(CH₂)ₘ to produce a cycloalkane ring,
Q may be hydrogen, alkyl, cycloalkyl, cycloalkenyl, alkoxy, substituted alkoxy, alkynyl, substituted alkynyl, substituted thio, cyano, thionitrile, sulfonamide, substituted sulfonamide, substituted sulfonyl, aromatic, substituted aromatic, heteroaromatic, substituted heteroaromatic, or bicycloheteroaromatic,
R⁵ is hydrogen, alkyl, cycloalkyl, or when R⁵ is alkyl, together with R⁶ may form a heterocycloalkane ring,
R⁶ may be hydrogen, alkyl, substituted alkyl, or together with R⁵, when R⁵ is alkyl, may form heterocycloalkane ring or -OR⁷,
R⁷ is alkyl or, when R⁵ is alkyl, together with R⁵ forms a 5-, 6-, or 7-membered ring,
L may be -O-, -S-, -N= or absent,
Z may be carbon or nitrogen or absent,
m = 1, 2 or 3,
n = 0, 1 or 2, and when n = 0,
Q may be directly bonded to Z;
with the provisos that when the compounds of the formula wherein R⁵ and R⁶ together form a morpholino ring and L is absent, then neither R¹, nor R² may be -C≡C-H; and
when the compounds of the formula wherein R⁵ is cyclopropyl, R¹, R², R³, R⁴,
and R⁶ may not all be hydrogen, or Q may not be meta-fluorophenyl
or a pharmaceutically acceptable addition salt of an acid or base thereof.

Alternative preferred embodiments include compounds according to formula I above wherein:
R⁵ is hydrogen, alkyl, cycloalkyl or together with R⁶ may form a heterocycloalkane ring, and L may be -O-, -S-, or -N=;
with the proviso that when R⁵ is cyclopropyl, R¹, R², R³, R⁴, and R⁶ may not all be hydrogen, or Q may not be meta-fluorophenyl.

Other preferred embodiments include compounds according to formula I above wherein:
R⁵ is hydrogen, alkyl, cycloalkyl or together with R⁶ may form a heterocycloalkane ring, and L is absent;
with the proviso that when R⁵ and R⁶ together form a morpholino ring, neither R¹, nor R² may be -C≡C-H.

Additional preferred embodiments include compounds according to formula I as described above wherein:
L may be -O- or N=.

Other preferred embodiments include compounds according to formula I as described above wherein:
n = 1.

Still additional preferred embodiments include compounds according to formula I as described above wherein:
Z is carbon, n = 0, and Q is directly bonded to Z.

Other preferred embodiments include compounds according to formula I as described above wherein:
L is -O-, Z is carbon, and n = 1 or 2.

Still further preferred embodiments include compounds according to formula I above wherein:
L is -N=, Z is carbon, and n = 1 or 2.

Other preferred embodiments include compounds according to formula I above wherein:
R¹ and R² are independently hydrogen, alkyl, substituted alkyl, cycloalkyl, alkynyl, substituted alkynyl, cyano, and if R¹ and R² are alkyl, R¹ and R² may be joined with a bond or -(CH₂)ₘ- to produce a cycloalkane ring,
R³ and R⁴ are independently hydrogen, alkyl, hydroxyl, fluoro, and if R³ and R⁴ are alkyl, R³ and R⁴ may be joined with a bond or -CH₂)ₘ- to produce a cycloalkane ring,
Q may be hydrogen, alkyl, cycloalkyl, cycloalkenyl, aromatic, substituted aromatic, heteroaromatic, substituted heteroaromatic, or bicycloheteroaromatic, R⁵ is alkyl, cycloalkyl or together with R⁶ may form a heterocycloalkane ring,
R⁶ may be alkyl, substituted alkyl, or -OR⁷, L may be -O-, -S- or -N=, Z may be carbon or nitrogen,
m = 1 , 2 or 3, n = 0, 1 or 2, and when n = 0, Q may be directly bonded to Z.

Still additional preferred embodiments include compounds of formula I as described above wherein:
Z = carbon, and n = 1 or 2.

Other preferred embodiments include compounds according to formula I as described above wherein:
R⁵ and R⁶ together form a heterocycloalkane ring, and
L is -O- or -N=.

Other preferred embodiments include compounds according to formula I as described above wherein:
L is -O-.

Still other preferred embodiments include compounds according to formula I as described above wherein:
R⁵ is alkyl, R⁶ is -OR⁷, R⁷ together with R⁵ forms a 5- or 6-membered ring, and L is -O- or -N=.

Other preferred embodiments include compounds according to formula I as described above wherein:
R¹ is hydrogen, R² is alkyl, substituted alkyl, cycloalkyl, alkynyl, substituted alkynyl, or cyano, and
Q is aromatic, substituted aromatic, heteroaromatic or substituted heteroaromatic.

Additional preferred embodiments include compounds according to formula I as described above wherein:
Q is substituted aromatic, heteroaromatic or substituted heteroaromatic.

Still other preferred embodiments include compounds according to formula I as described above wherein:
R² is alkyl, cycloalkyl, alkynyl, or cyano, R³ is hydrogen,
R⁴ is alkyl, hydroxyl, fluoro, and Q is aromatic, substituted aromatic, heteroaromatic or substituted heteroaromatic.

Yet still other preferred embodiments include compounds according to formula I as described above wherein:
R² is alkyl, cycloalkyl, alkynyl, or cyano, R³ and R⁴ are independently alkyl, hydroxyl, fluoro, and if R³ and R⁴ are alkyl, R³ and R⁴ may be joined with a bond or -CH₂)ₘ- to produce a cycloalkane ring.

Further preferred embodiments include compounds according to formula I as described above wherein:
R² is alkyl, cycloalkyl, alkynyl, or cyano, R³ and R⁴ are independently alkyl, hydroxyl, fluoro, and if R³ and R⁴ are alkyl, R³ and R⁴ may be joined with a bond or -CH₂)ₘ- to produce a cycloalkane ring.

Other embodiments may be readily gleaned from the further description of the present invention which follows and in particular, the examples which appear in the present specification.

### III. Synthesis

The synthesis of 3-substituted benzo[1,2,3]-triazin-4-one compounds are preferably carried out by the following Schemes 1-7, wherein the syntheses of substituted salicylamides (1) are well-known in the field of organic synthesis. Alternative syntheses by analogy replying on well-known methodology which exists in the art also may be used.

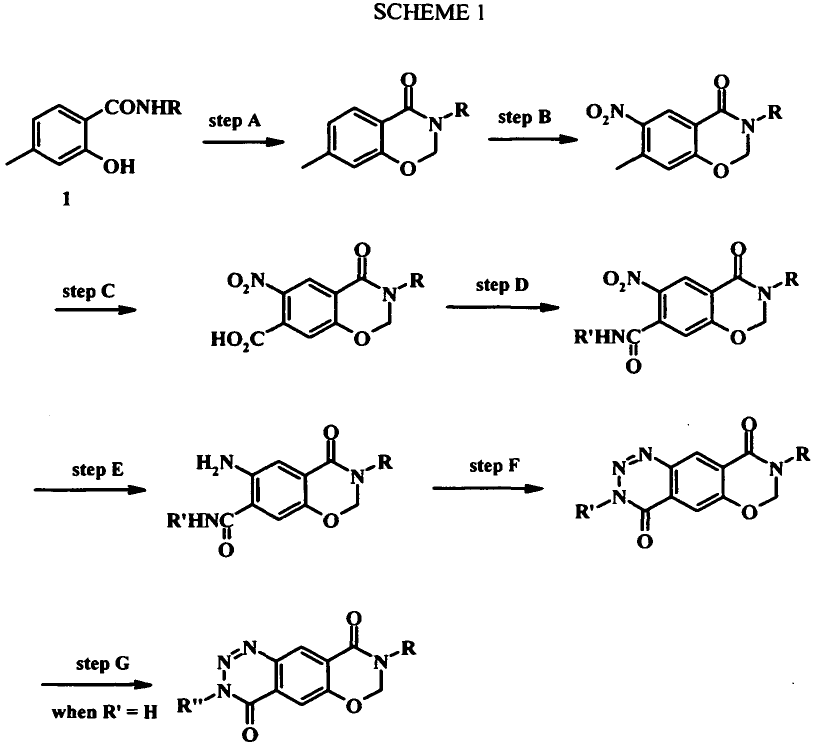

In Scheme 1, step A may be carried out under standard conditions, among them acid catalyzed insertion of a formaldehyde synthon. For example, the salicylamide (1) is dissolved and heated in a suitable organic solvent together with trioxane and sulfuric or hydrochloric acid. Alternatively, if R contains an aldehyde functionality, ring closure to a fused cycloalkylbenzoxazine structure can be carried out under acidic conditions e.g. with 6N hydrochloric acid at ambient temperature as shown in Scheme 2.

In Schemes 1 and 2, step B is a nitration reaction that can be carried out under mild conditions known to those skilled in organic synthesis and detailed in such volumes as Reagents for Organic Synthesis (Fieser and Fieser) and Organic Syntheses (see web site at http://www.orgsyn.orgn. Step C describes the 2-step oxidation of the methyl group of compound 3 to a carboxylic acid via an intermediate aldehyde employing N,N-dimethylformamide dimethyl acetal and sodium periodate followed by oxone or potassium permanganate. Step D involves coupling of a primary amine to the nitro acid, which can be accomplished using a variety of coupling reagents known to a skilled chemist. Some nonlimiting examples commonly used are thionyl chloride, oxalyl chloride or carbonyl diimidazole. Step E is a reduction of the aryl nitro to an aniline and can be effected by hydrogenation using a variety of catalysts including, but not limited to, Pd, Pt or Raney Ni, or Zn/Cu. Step F forms the triazinone structure and can be carried out by the addition of isoamyl nitrite in DMF. When R' = H, the group R" may be introduced by reaction with R"-X where X = halogen e.g. bromide or mesylate in a suitable solvent for example DMF or DME, in the presence of an organic base e.g. DBU.

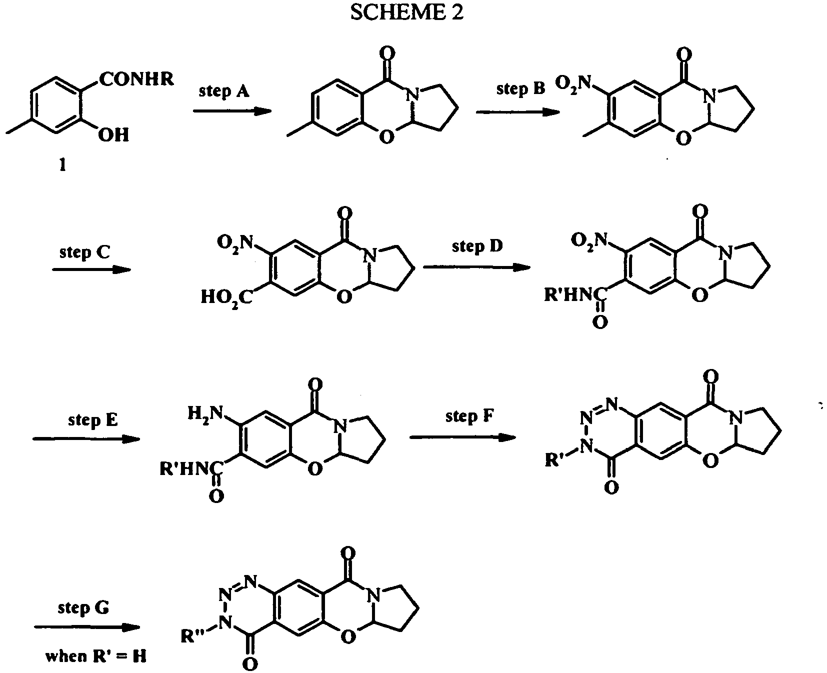

In like manner, other methods known in the chemical literature to close an anthranilamide as shown below are within the scope of the invention. As an example, Scheme 3 below is an alternative method employed for some of the examples of the present invention. Step A may be achieved via methods similar to those for step E of Schemes 1 and 2 and step B is a classical diazotization reaction used for many aromatic transformations. In Scheme 3, step C is carried out without isolation of intermediate 2 by addition of a primary amine followed by a suitable base, such as a tertiary amine to give the triazinone product structure 3.

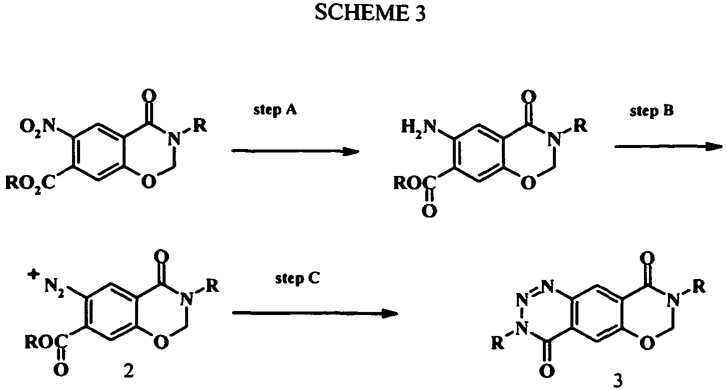

Another method used to prepare compounds of the present invention that are not derivatives of salicylates as starting materials is shown in Scheme 4 below:

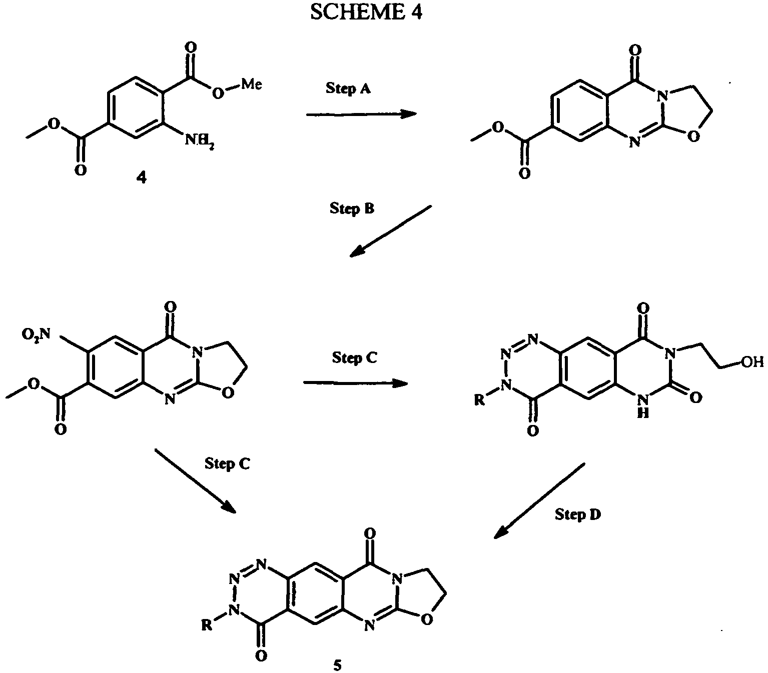

Step A in Scheme 4 is conducted by reacting structure 4 with 2-bromoethyl isocyanate at elevated temperature in the range of 50 to 150 °C and preferable at about 90 °C, in the presence of a base such as a tertiary amine. Step B of Scheme 4 can be carried out by a number of methods known to those skilled in the art as described in many texts including Reagents for Organic Syntheses (Fieser and Fieser). Preferably the reaction is conducted using a mixture of nitric and sulfuric acids near ambient temperature. The last reactions of Scheme 3 may be carried out in a single step C, wherein a) the nitro group is reduced using any number of reagents, including freshly prepared Zn/Cu catalyst plus formic acid, b) the resulting aniline is diazotized by the conditions of step B in Scheme 3, and c) the desired amine is as RNH₂ is inserted to form the triazinone structure (5) by the method of step C, Scheme 3 above. Occasionally, ring opening was observed during step C part c, which required the transformation of the alcohol into the bromide (Step D), followed by ring closure under the conditions mentioned for step A.

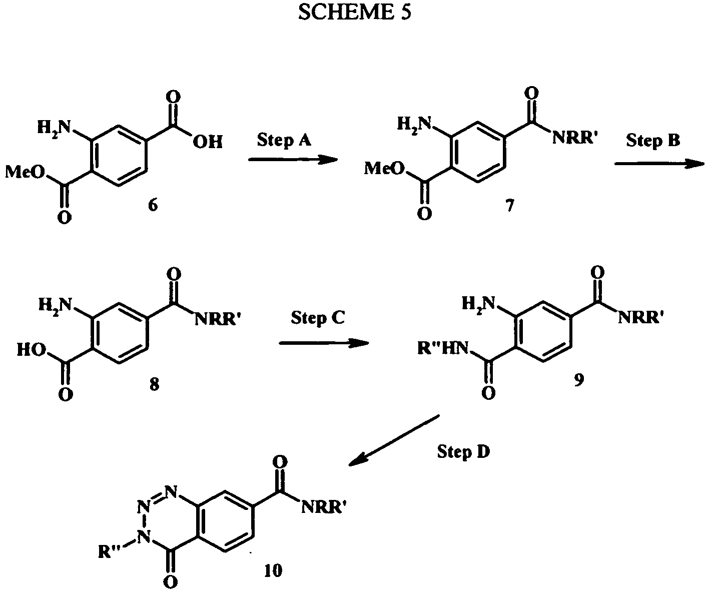

The synthesis of a further class of compounds of the invention is shown in Scheme 5. Step A in Scheme 5 can be carried out using a number of different reagents known to be useful in the coupling of amines to carboxylic acids. Such reagents are described in a number of texts, including Reagents for Organic Syntheses (Fieser and Fieser). A preferred method uses a carbodiimide reagent to activate the free carboxyl group of structure 6, most preferably the water-soluble carbodiimide, EDCI. Activation is achieved in DMF as solvent and under acyl transfer conditions provided by HOBT, triethyl amine and DMAP in order to couple structure 6 to a primary or secondary amine to give structure 7.

Step B in Scheme 5 to convert the ester of structure 7 to the free acid of structure 8 is carried out under mildly basic conditions in aqueous/organic solvents such that the newly-formed amide is not also hydrolyzed. Preferably, hydrolysis of the ester is performed in a solvent mixture of THF, methanol and water using sodium or potassium carbonate as the base. The free acid is isolated following acidification using concentrated HCl.

Step C is conducted using the conditions set forth for step A of Scheme 5, with the exception that coupling must be to a primary amine to provided structure 9. The final reaction depicted by step D to give the final product of structure 10 is completed using isoamyl nitrite.

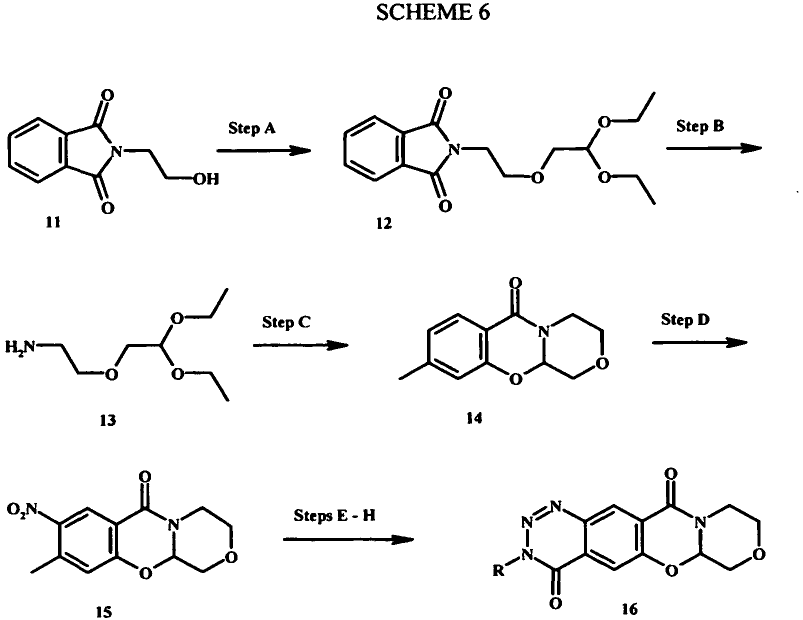

The synthesis of a further class of compounds of the invention is shown in Scheme 6 above. In Scheme 6, step A may involve standard condensation of phthalimide 11 with bromoacetaldehyde diethyl acetal under basic conditions. Deprotection of the amino group in derivative 12 by treatment with hydrazine releases amine 13 (step B). Condensation of amine 13 with 4-methylsalicylic acid using one of the common agents (e.g. CDI) followed by cyclization under acidic conditions provides compound 14 (step C) that is consecutively nitrated using conditions previously described to give derivative 15 (step D). The following steps (E - H) may be performed in analogy to the corresponding steps from Scheme 1 (C - F) to give compounds 16.

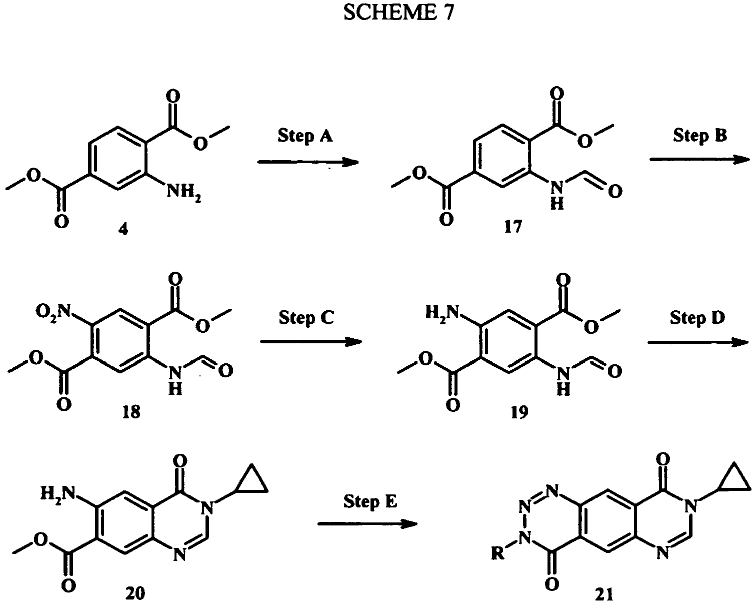

The synthesis of a yet further class of compound of the invention is depicted in Scheme 7 above. In Scheme 7, aminoterephthalate (4) can be conveniently formylated using a mixture of acetic anhydride and formic acid (step A). The obtained formamide 17 is preferentially nitrated at C-5 to give nitro-amide 18 in high yield (step B). Reduction of the nitro group (step C) gives amine 19. The following cyclocondensation with cyclopropylamine (step D) provides quinazolinone 20. Finally, the methoxycarbonyl group may be converted to an amide which is subsequently treated with a diazotizating reagent (e.g., isopentyl nitrite) to give product 21 (step E).

All compositions disclosed in the present application may be synthesized by the above-described methods using analogous synthetic steps to those specifically presented in the examples described herein as well as those known in the art. Isolation of stereo- and/or optical isomers may be performed by methods which are well-known in the art such as fractional crystallization and chromatography on a variety of stationary phases, including those with chiral auxiliaries.

### III. Method of Treatment

According to one aspect of the invention, compounds for use in treating a mammalian subject suffering from a hypoglutamatergic condition, or from deficiencies in the number or strength of excitatory synapses or in the number of AMPA receptors are provided. In such a subject, memory or other cognitive functions may be impaired, or cortical/striatal imbalance may occur, leading to loss of memory, dementia, depression, attention disorders, sexual dysfunction, movement disorders, schizophrenia or schizophreniform behavior. Memory disorders and learning disorders, which are treatable according to the present invention include those disorders that result from aging, trauma, stroke and neurodegenerative disorders. Examples of neurodegenerative disorders include, but are not limited to, those associated with drug-induced states, neurotoxic agents, Alzheimer's disease, and aging. These conditions are readily recognized and diagnosed by those of ordinary skill in the art and treated by administering to the patient an effective amount of one or more compounds according to the present invention.

According to a further aspect of the invention compounds for use in for reducing or inhibiting respiratory depression (RD) in a subject as a result of disease or pharmacological agents are provided.
The method of treatment comprises administering to the subject in need of treatment, in a pharmaceutically acceptable carrier, an effective amount of a compound having the Formula of I below: 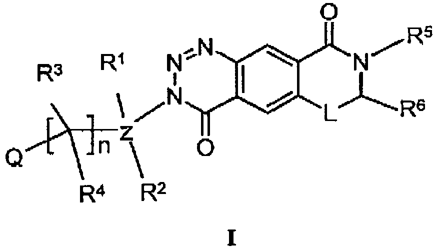 wherein:
R¹ and R² are independently hydrogen, alkyl, substituted alkyl, cycloalkyl, alkynyl, substituted alkynyl, cyano, alkoxy, carboxamido, substituted carboxamido, and if R¹ and R² are alkyl, R¹ and R² may be joined with a bond or -(CH₂)ₘ- to produce a cycloalkane ring,
R³ and R⁴ are independently hydrogen, alkyl, hydroxyl, alkoxy, cyano, fluoro, and if R³ and R⁴ are alkyl, R³ and R⁴ may be joined with a bond or -(CH₂)ₘ- to produce a cycloalkane ring,
Q may be hydrogen, alkyl, cycloalkyl, cycloalkenyl, alkoxy, substituted alkoxy, alkynyl, substituted alkynyl, substituted thio, cyano, thionitrile, sulfonamide, substituted sulfonamide, substituted sulfonyl, aromatic, substituted aromatic, heteroaromatic, substituted heteroaromatic, or bicycloheteroaromatic,
R⁵ is hydrogen, alkyl, cycloalkyl, or when R⁵ is alkyl, together with R⁶ may form a heterocycloalkane heterocycloalkyl ring, R⁶ may be hydrogen, alkyl, substituted alkyl, or together with R⁵, when R⁵ is alkyl, may form heterocycloalkane ring or -OR⁷,
R⁷ is alkyl or, when R⁵ is alkyl, together with R⁵ forms a 5-, 6-, or 7-membered ring,
L may be -O-, -S-, -N= or absent,
Z may be carbon or nitrogen,
m = 1, 2 or 3,
n = 0, 1 or 2, and when n = 0,
Q may be directly bonded to Z;
with the provisos that when the compounds of the formula wherein R⁵ and R⁶ together form a morpholino ring and L is absent, then neither R¹, nor R² may be -C≡C-H; and
when the compounds of the formula wherein R⁵ is cyclopropyl, R¹, R², R³, R⁴,
and R⁶ may not all be hydrogen, or Q may not be meta-fluorophenyl.
or a pharmaceutically acceptable addition salt of an acid or base thereof.

As noted above, treatment of a subject according to compound for use in the method is useful for enhancing AMPA receptor activity, and thus may be used to facilitate the learning of behaviors dependent upon AMPA receptors, and to treat conditions, such as memory impairment, in which AMPA receptors, or synapses utilizing these receptors, are reduced in numbers or efficiency. The compounds are also useful for enhancing excitatory synaptic activity in order to restore an imbalance between brain sub-regions, which may manifest itself in schizophrenia or schizophreniform behavior, or other behavior as described above. The compounds administered in accordance with the method have been found to be more effective than previously described compounds in enhancing AMPA receptor activity, as shown in the *in vitro* and *in vivo* tests described below.

### IV. Biological Activity: Enhancement of AMPA Receptor Function

### IVa. In Vitro Assays

Synaptic responses mediated by AMPA receptors are increased according to compounds for use according to the invention, using the compounds described herein. These compounds are demonstrated, in the Examples that follow, to be substantially more potent than previously-described compounds in increasing AMPA mediated whole cell currents in cultured neurons and AMPA receptor function in slices of rat hippocampus. The physiological effects of invention compounds were tested *in vitro* on primary cultures of rat cortical or hippocampal neurons or on slices of rat hippocampus according to the following procedures.

### Patch Clamp Electrophysiology Assay

Cortical cells were prepared from day 18-19 embryonic Sprague-Dawley rats and recorded after 3 days in culture. The extracellular solution (ECS) contained (in mM): NaCl (145), KCl (5.4), HEPES (10), MgCl2 (0.8), CaCl2 (1.8), glucose (10), sucrose (30); pH. 7.4. In order to block the voltage-gated sodium currents, 40 nM TTX was added to the recording solution. The intracellular solution contained (in mM): K-gluconate (140), HEPES (20), EGTA (1.1), phosphocreatine (5), MgATP (3), GTP (0.3), MgCl2 (5), and CaCl2 (0.1); pH: 7.2. All test compound and glutamate solutions were made-up in the extracellular solution.

The whole-cell current was measured with patch-clamp amplifier (Axopatch 200B), filtered at 2 kHz, digitized at 5 kHz and recorded on a PC with pClamp 8. The cells were voltage-clamped at - 80 mV. Solutions were applied by DAD-12 system. A baseline response for each cell was recorded using a 1 s pulse of 500 µM glutamate dissolved in ECS. Responses to test compound were then determined by application of a 10 s pulse of test compound followed by a 1 s pulse of the same concentration of test compound plus 500 µM glutamate and then 10 s of saline. This pulse sequence was repeated until a stable reading was obtained, or until sufficient data points were measured to allow extrapolation to a calculated maximum change.

The mean value of plateau current between 600 ms to 900 ms after application of glutamate or test compound plus glutamate was calculated and used as the parameter to measure the drug effect. The plateau responses in the presence of varying concentrations of test compound were divided by the baseline response in order to calculate the percentage increase. Compounds are deemed active in this test if, at a test concentration of 3 µM or less, they produce a greater than 100% increase in the value of the steady-state current measured due to application of glutamate alone. The concentration at which the glutamate induced current is increased by 100% is commonly referred to as the EC2x value. Compounds of the examples disclosed above displayed EC2x values in the range of 0.003 to 10 µM.

### Rat Hippocampal Slice Assay

In another test, excitatory responses (field EPSPs) were measured in hippocampal slices, which were maintained in a recording chamber continuously perfused with artificial cerebrospinal fluid (ACSF). During a 15 - 30 minute interval, the perfusion medium was switched to one containing various concentrations of the test compounds. Responses collected immediately before and at the end of drug perfusion were superimposed in order to calculate the percent increase in EPSP amplitude.

The field EPSP (excitatory post-synaptic potential) recorded in field CA1 after stimulation of CA3 axons is known to be mediated by AMPA receptors, which are present in the synapses (Kessler et al., Brain Res. 560: 337-341 (1991)). Drugs that selectively block the receptor selectively block the field EPSP (Muller *et al*., *Science*, supra). Aniracetam, which has been shown to increase the mean open time of the AMPA receptor channel, increases the amplitude of the synaptic current and prolongs its duration (Tang *et al*., *Science*, *supra*). These effects are mirrored in the field EPSP (see, for example, Staubli *et al*., *Psychobiology, supra*; Xiao *et al.*, *Hippocampus, supra*; Staubli et al., Hippocampus 2: 4958 (1992)). Similar results have been reported for the previously disclosed stable benzamide analogs of aniracetam (Lynch and Rogers, PCT Pubn. No. WO 94/02475).

To obtain data for the activity of invention compounds on synaptic responses, a bipolar nichrome stimulating electrode was positioned in the dendritic layer (stratum radiatum) of the hippocampal subfield CA1 close to the border of subfield CA3, as described in Example 30. Current pulses (0.1 msec) through the stimulating electrode activate a population of the Schaffer-commissural (SC) fibers, which arise from neurons in the subdivision CA3 and terminate in synapses on the dendrites of CA1 neurons. Activation of these synapses causes them to release the transmitter glutamate. Glutamate binds to post-synaptic AMPA receptors, which then transiently open an associated ion channel and permit a sodium current to enter the postsynaptic cell. This current results in a voltage in the extracellular space (the field EPSP), which is recorded by a high impedance recording electrode positioned in the middle of the stratum radiatum of CA1.

The intensity of the stimulation current was adjusted to produce half-maximal EPSPs (typically about 1.5 - 2.0 mV). Paired stimulation pulses were given every 40 sec with an interpulse interval of 200 msec, as described further in Example 30.

Hippocampal slices were maintained in a recording chamber continuously perfused with artificial cerebrospinal fluid (ACSF). During 15 - 30 minute intervals, the perfusion medium was switched to one containing various concentrations of the test compounds. Responses collected immediately before and at the end of drug perfusion were superimposed in order to calculate the percent increase in EPSP amplitude.

Studies that compared the effects of AMPA modulators on monosynaptic (as reported here) and polysynaptic responses demonstrated that a 10% increase in the amplitude of the monosynaptic field EPSP was amplified to an increase of 300% on a trisynaptic response (Servio et al., Neuroscience 74: 1025-1035 (1996)). Furthermore, the concentration of the modulator that evoked these responses was shown to exist in plasma from behaviorally relevant doses (Granger *et al*., *Synapse*, *supra*). Thus, the concentration of compound sufficient to produce a 10% increase in amplitude of the monosynaptic field EPSP is likely to represent a behaviorally relevant plasma concentration.

### IVb. In Vivo Physiological Testing

The physiological effects of invention compounds were tested *in vivo* in anesthetized animals according to the following procedures.

Animals are maintained under anesthesia by phenobarbital administered using a Hamilton syringe pump. Stimulating and recording electrodes are inserted into the perforant path and dentate gyrus of the hippocampus, respectively. Once electrodes are implanted, a stable baseline of evoked responses are elicited using single monophasic pulses (100 µs pulse duration) delivered at 3/min to the stimulating electrode. Field EPSPs are monitored until a stable baseline is achieved (about 20-30 min), after which a solution of test compound in HPCD is injected intraperitoneally and evoked field potentials are recorded. Evoked potentials are recorded for approximately 2 h following drug administration or until the amplitude of the field EPSP returns to baseline. In the latter instance, it is common that an iv administration is also carried out with an appropriate dose of the same test compound.

The activity of selected compounds of the invention in the patch clamp electrophysiology assay, the rat hippocampal slice assay and in the rat *in vivo* electrophysiology assay is summarized in Table 1.

**Table 1**

| Compound Example Number | *In vitro* Patch Clamp Electrophysiology ¹EC2x | ²Rat Hippocampal slice assay | ^{3,4}*In vivo* Electrophysiology |
|---|---|---|---|
| 1 | 3nM | 9% @ 3µM | 24%³ |
| 4 | 400nM | 5% @ 3µM | 19%⁴ |
| 9 | 140nM | 11% @ 3µM | 11%⁴ |
| 10 | 190nM | 10% @ 10µM | 12%³ |
| 16 | 3nM | 1% @ 3µM | 34%³ |
| 35 | 80nM | NT | 19%³ |
| 36 | 100nM | 12% @ 3µM | 18%⁴ |
| 37A | 40nM | NT | 39%³ |
| 64 | 170nM | NT | 30%³ |

| | | | |
|---|---|---|---|
| 1. Concentration at which glutamate induced current is increased by 100% in the patch clamp assay 2. % Increase in the amplitude of the field EPSP in the CA1 region of rat hippocampal slice 3. % increase in the amplitude of the field EPSP in rat dentate gyrus @ 5mpk i.p. 4. % increase in the amplitude of the field EPSP in rat dentate gyrus @ 5mpk i.v. NT = Not tested | | | |

While the invention has been described with reference to specific compounds for use in a treatment methods and embodiments, it will be appreciated that various modifications may be made without departing from the invention.

### V. Administration, Dosages and Formulation

As noted above, the compounds and compounds for use in the treatment of the invention increase AMPA receptor-mediated responses, and are useful for the treatment of hypoglutamatergic conditions. They are also useful for treatment of conditions such as impairment of memory or other cognitive functions, brought on by a deficiency in the number or strength of excitatory synapses, or in the number of AMPA receptors. They may also be used in the treatment of schizophrenia or schizophreniform behavior resulting from a cortical/striatal imbalance, and in facilitation of learning of behaviors dependent upon AMPA receptors.

In subjects treated with the present compounds, pharmaceutical compositions and methods memory or other cognitive functions may be impaired, or cortical/striatal imbalance may occur, leading to loss of memory, dementia, depression, attention disorders, sexual dysfunction, movement disorders, schizophrenia or schizophreniform behavior. Memory disorders and learning disorders, which are treatable according to the present invention, include those disorders that result from aging, trauma, stroke and neurodegenerative disorders. Examples of neurodegenerative disorders include, but are not limited to, those associated with drug-induced states, neurotoxic agents, Alzheimer's disease, and aging. These conditions are readily recognized and diagnosed by those of ordinary skill in the art and treated by administering to the patient an effective amount of one or more compounds according to the present invention.

Generally, dosages and routes of administration of the compound will be determined according to the size and condition of the subject, according to standard pharmaceutical practices. Dose levels employed can vary widely, and can readily be determined by those of skill in the art. Typically, amounts in the milligram up to gram quantities are employed. The composition may be administered to a subject by various routes, e.g. orally, transdermally, perineurally or parenterally, that is, by intravenous, subcutaneous, intraperitoneal, or intramuscular injection, among others, including buccal, rectal and transdermal administration. Subjects contemplated for treatment according to compounds for use in treatment of the invention include humans, companion animals, laboratory animals, and the like.

Formulations containing the compounds according to the present invention may take the form of solid, semi-solid, lyophilized powder, or liquid dosage forms, such as, for example, tablets, capsules, powders, sustained-release formulations, solutions, suspensions, emulsions, suppositories, creams, ointments, lotions, aerosols, patches or the like, preferably in unit dosage forms suitable for simple administration of precise dosages.

Pharmaceutical compositions according to the present invention typically include a conventional pharmaceutical carrier or excipient and may additionally include other medicinal agents, carriers, adjuvants, additives and the like. Preferably, the composition will be about 0.5 to 75% by weight of a compound or compounds of the invention, with the remainder consisting essentially of suitable pharmaceutical excipients. For oral administration, such excipients include pharmaceutical grades of mannitol, lactose, starch, magnesium stearate, sodium saccharine, talcum, cellulose, glucose, gelatin, sucrose, magnesium carbonate, and the like. If desired, the composition may also contain minor amounts of non-toxic auxiliary substances such as wetting agents, emulsifying agents, or buffers.

Liquid compositions can be prepared by dissolving or dispersing the compounds (about 0.5% to about 20% by weight or more), and optional pharmaceutical adjuvants, in a carrier, such as, for example, aqueous saline, aqueous dextrose, glycerol, or ethanol, to form a solution or suspension. For use in oral liquid preparation, the composition may be prepared as a solution, suspension, emulsion, or syrup, being supplied either in liquid form or a dried form suitable for hydration in water or normal saline.

When the composition is employed in the form of solid preparations for oral administration, the preparations may be tablets, granules, powders, capsules or the like. In a tablet formulation, the composition is typically formulated with additives, e.g. an excipient such as a saccharide or cellulose preparation, a binder such as starch paste or methyl cellulose, a filler, a disintegrator, and other additives typically used in the manufacture of medical preparations.

An injectable composition for parenteral administration will typically contain the compound in a suitable i.v. solution, such as sterile physiological salt solution. The composition may also be formulated as a suspension in a lipid or phospholipid, in a liposomal suspension, or in an aqueous emulsion.

Methods for preparing such dosage forms are known or will be apparent to those skilled in the art; for example, see Remington's Pharmaceutical Sciences (17th Ed., Mack Pub. Co., 1985). The composition to be administered will contain a quantity of the selected compound in a pharmaceutically effective amount for effecting increased AMPA receptor currents in a subject.

The following examples illustrate -the invention. Unless otherwise stated, all temperatures are given in degrees Celsius. Unless otherwise stated, ¹H NMR spectra were obtained in deuterochloroform or deuterated DMSO as solvent using tetramethylsilane as an internal standard. All names of Example compounds conform to IUPAC nomenclature as provided by the computer software ChemSketch by ACD Labs.

### EXAMPLE 1

### 3-[2-(3-Fluorophenyl)ethyl]-6a,7,8,9-tetrahydro-3H-pyrrolo[2',1':2,3][1,3]oxazino[6,5-g][1,2,2]benzotriazine-4,11-dione

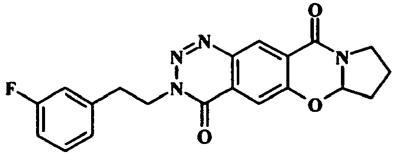

### 6-Methyl-1,2,3.3a-tetrahydro-9H-pyrrolo[2,1-b][1,3]benzoxazin-9-one

4-Methylsalicylic acid (10 g, 66 mmol) was dissolved in chloroform (80 mL) and *N*,*N-*carbonyl diimidazole (CDI; 12.2 g, 75 mmol) was added. After stirring at ambient temperature for 70 min, the CO₂ was removed under vacuum. 4-Aminobutyraldehyde dimethyl acetal (10.3 g, 77 mmol) was dissolved in chloroform (15 mL) and added over 30 min. The mixture was stirred for 2 hr at ambient temperature, after which 6M HCl (150 mL) was added under vigorous stirring. When the reaction was complete as monitored using TLC, the organic phase was separated and the aqueous phase was washed with chloroform (200 mL). The combined organic phases were washed with saturated sodium bicarbonate solution and dried over sodium sulfate. The chloroform was removed under vacuum and the residue was dissolved in ethyl acetate and crystallized using methyl tert-butyl ether/hexane to yield 11.2 g of the benzoxazinone.

### 6-Methyl-7-nitro-1,2,3,3a-tetrahydro-9H-pyrrolo[2,1-b][1,3]benzoxazin-9-one

The benzoxazinone (11.2 g, 55 mmol) was dissolved in chloroform (35 mL), acetic acid (25 mL) and acetic anhydride (25 mL) and cooled using an ice bath. Nitric acid (4 mL, 90%) was added dropwise, which produced an orange solution. The reaction was complete after 30 min, as confirmed by TLC. The reaction mixture was poured over crushed ice (650 g), extracted with chloroform (3 × 250 mL), washed with saturated sodium bicarbonate solution (500 mL), dried over sodium bicarbonate and filtered through a 1 cm layer of silica gel. The solvent was removed under vacuum and the residue was crystallized from ethyl acetate. The yield of the desired 5-nitro isomer was 6.3 g (25 mmol). The mother liquor contained 3.6 g (14 mmol) of both 3- and 5-nitro isomers (1:1).

The nitro derivative from multiple repetitions of the previous step (20.2 g, 81.4 mmol), anhydrous DMF (10 mL) and *N,N*-dimethylformamide dimethyl acetal (48 g, 0.40 mol) were heated under argon atmosphere at 125°C for 24 hr. The reaction mixture was cooled and the volatiles were removed under vacuum. The resulting residue was dissolved in THF (300 mL) and treated with an aqueous solution of sodium periodate (44 g, 206 mmol) at ambient temperature for 15 min. The resulting beige slurry was filtered and the solids were further stirred with water (200 mL) and chloroform (300 mL). The mixture was filtered and the two filtrates were combined. The organic phase was separated, dried over sodium sulfate, filtered through a 2 cm bed of silica gel, and concentrated under vacuum to about 200 mL. Ethyl acetate (200 mL) was added and the solution was further concentrated to about 100 mL until crystallization began. The crystals were collected by filtration, washed with a small amount of ethyl acetate and air dried to give 17.6 g (67.2 mmol) of nitro aldehyde derivative.

### 7-Nitro-9-oxo-1,2,3,3a-tetrahydro-9H-pyrrolo[2,1-b][1,3]benzoxazine-6-carboxylic acid

The nitro aldehyde derivative from the previous step (16 g, 61 mmol) was dissolved in DMF (300 mL) and treated with oxone (45 g, 73 mmol, 1.2 eq.) at ambient temperature for 18 hr. Chloroform (250 mL) was added and the stirring was continued for 10 min. The mixture was set aside for 30 min to let the solids settle. The mixture was filtered, the solids were washed with 150 mL DMF/chloroform (1/1) and the filtrate was concentrated under vacuum to about 25 mL. Chloroform (40 mL) was added and the solution was poured into water (400 mL) during vigorous stirring. The stirring was continued for 20 min after which the product was collected by filtration. The solid was dried under high vacuum for 18 hr to give 15.5 g (56 mmol) of a light yellow powder.

CDI (3.5 g, 22 mmol) was added to a solution of the nitro acid derivative (3.1 g, 11 mmol) in chloroform (60 mL) and the solution was stirred for 2 hr. Methanol (10 mL) was added and the solution was stirred overnight. Water (150 mL) was added, the pH was adjusted to 2 using 2M H₂SO₄ and the mixture was extracted with chloroform (2 × 200 mL). The organic phase was dried over sodium sulfate and the volatiles evaporated from 10 g of silica gel. The product was purified using flash chromatography with ethyl acetate/hexane (1:1) as the mobile phase, followed by ethyl acetate/methylene chloride/hexane (5:1:4). The solvent evaporated under vacuum, but not to dryness. White crystals formed (1.34 g, 4.6 mmol) from the concentrated solution.

The methyl ester from the previous step (1.1 g, 3.8 mmol) was dissolved in a mixture of chloroform (25 mL) and methanol (25 mL). Palladium (10% on carbon, 380 mg) was added and the mixture was hydrogenated at ambient temperature for 3.5 hr. The solids were removed by filtration and washed with methanol (20 mL). The volatiles were evaporated to give the amino ester as a yellow solid, which was dissolved in a mixture of chloroform (10 mL) and THF (10 mL). A solution of sodium nitrite (300 mg, 4.3 mmol) in water (10 mL) was added and the mixture was cooled to 10 °C. Concentrated HCl (10 drops) was added during vigorous stirring. After 15 min, 3-fluorophenethylamine (1 mL, 7 mmol) was added followed by triethyl amine (3 mL, 22 mmol) to yield a brown mixture, which was stirred at ambient temperature for 18 hr. Water (100 mL) was added and the pH was adjusted to 2 using concentrated HCl. The mixture was extracted with chloroform (3 × 100 mL), the organic phase was dried over sodium sulfate and concentrated under vacuum. The product was purified using flash chromatography with ethyl acetate/chloroform/hexane (2:1:1) as the mobile phase. The solvent was removed under vacuum and the residue was recrystallized from chloroform / methyl tert-butyl ether. The white solid (195 mg) had the following properties: MP: 162 - 164 °C; ¹H NMR (300 MHz, CDCl₃) δ 8.74 (1H, s), 7.84 (1H, s), 7.3 - 6.8 (4H, m), 5.62 (1H, t, J=6 Hz), 4.67 (2H, m), 3.92 (1H, m), 3.70 (1H, m), 3.22 (2H, t, J=8 Hz), 2.54 (1H, m), 2.36 (1H, m), 2.19 (1H, m) and 2.04 ppm (1H, m).

### EXAMPLE 2

### 3-(4,11-Dioxo-4,6a,7,8,9,11-hexahydro-3H-pyrrolo[2',1':2,3][1,3]oxazino[6,5-g][1,2,3] benzotriazin-3-yl)propanenitrile

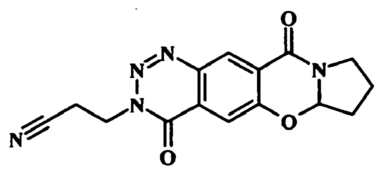

The procedure in Example 1 was followed to produce the amino ester intermediate using 0.96 g (3.3 mmol) of nitro ester derivative. It was dissolved in a mixture of chloroform (10 mL) and THF (10 mL), a solution of sodium nitrite (0.29 mg, 4.2 mmol) in water (10 mL) was added and the mixture cooled to 0 °C in an ice bath. HCl (2M, 3 mL) was added and the mixture was stirred for 30 min. 3-Aminopropionitrile (0.46 g, 6.6 mmol) was added followed by triethylamine (enough to reach pH 7-8) and the mixture was stirred for 30 min at ambient temperature to give a brown slurry. Water (100 mL) was added and the slurry was extracted with chloroform (3 × 100 mL). The organic phase was dried over sodium sulfate and concentrated under vacuum. The product was purified using flash chromatography with chloroform/ethyl acetate, 85/15, as the mobile phase to give three products. The fractions with the second product (intermediate) were combined and triethylamine (1 mL, 7.2 mmol) was added and the solution was left at ambient temperature for three days after which the intermediate had converted to product III. The solutions with product III were combined and the solvent removed to yield a yellow residue. It was further purified using flash chromatography (50 g silica gel) with chloroform/ethyl acetate/hexane, 6/3/1, as the mobile phase. A second chromatography purification was needed (chloroform/acetone, 9/1) to remove the color. The solvent was removed under vacuum (the product crystallized out when a small amount of solvent remained), and the product was recrystallized from chloroform/ethyl acetate/hexane to give a white powder (142 mg) with the following properties: MP: 184 - 186 °C; ¹H NMR (300 MHz, CDCl₃) δ 8.78 (1H, s), 7.86 (1H, s), 5.63 (1H, t, J=6 Hz), 4.73 (2H, m), 3.93 (1H, m), 3.70 (1H, m), 3.04 (2H, m), 2.54 (1H; m), 2.36 (1H, m), 2.19 (1H, m) and 2.04 ppm (1H, m).

### EXAMPLE 3

### 3-Cyclobutyl-6a,7,8,9-tetrahydro-3H-pyrrolo[2',1':2,3][1,3]oxazino[6,5-g][1,2,3] benzotriazine-4,11-dione

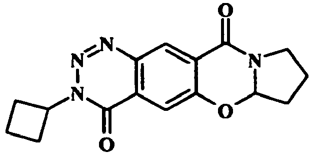

The nitro acid derivative (see example 1; 0.70 g, 2.52 mmol) was suspended in methylene chloride (40 mL). Five drops of DMF followed by thionyl chloride (1.82 mL, 21 mmol) was added and the mixture was stirred at ambient temperature overnight. The volatiles were removed under vacuum to yield a yellow solid which was dissolved in methylene chloride (20 mL). Cyclobutylamine hydrochloride (1.45 g, 12.5 mmol) and triethyl amine (3.5 mL, 25 mmol) were dissolved in methylene chloride (30 mL) and the solution of the yellow solid was slowly added. The reaction was complete after one hour of stirring at ambient temperature. The reaction mixture was washed with 1N HCl, followed by a sodium bicarbonate solution. It was dried over magnesium sulfate, and concentrated to give 0.60 g of a yellow solid.

The Zn/Cu reagent was prepared in the following manner: Conc. HCl (3 mL) was added to 10 g of Zinc in 100 mL water during vigorous stirring. The stirring continued for 2 min (clumps start to form), after which the water was decanted off. An additional 100 mL of water was added with vigorous stirring. Any remaining clumps were crushed with a spatula. Conc. HCl (3 mL) was added and the stirring was continued for 2 min. After removing the water by decantation, the solid was washed with an additional 100 mL of water. Water (50 mL) was added to the solid and the stirring was continued while a solution of CuSO₄ (300 mg in 50 mL water) was added slowly. After the zinc turned black, the water was removed by decantation. The residue was sequentially washed with methanol (50 mL) and THF (50 mL).

The yellow solid from the previous step (0.60 g, 1.5 mmol) was dissolved in a mixture of THF (50 mL) and methanol (70 mL) by warming it to 50 °C. The solution was added to freshly prepared Zn/Cu reagent (6 g, see above). Formic acid (40 drops) was added and the mixture was stirred at ambient temperature for 1 hour after which the reduction was complete. DMF (10) mL) was added and the mixture was filtered through 2 cm of silica gel. The filtrate was evaporated to dryness then dissolved in DMF (20 mL). An excess of isoamyl nitrite (8 mL) was added and the mixture was stirred over night. The DMF was removed under vacuum and the crude product was purified by column chromatography (60 g silica gel, ethyl acetate: hexane 3:1). The product fractions were combined and the solvent was removed under vacuum. The product was re crystallized from methylene chloride: methyl t-butylether to give 0.31 g of a white solid with the following properties: MP: 216 - 218 °C; ¹H NMR (300 MHz, CDCl₃) δ 8.75 (1H, s), 7.83 (1H, s), 5.61 (1H, m), 5.51 (1H, m), 4.0-3.6 (2H, m) and 2.9-1.9 (10H, m) ppm (4H, m).

### EXAMPLE 4

### 3-Cyclopropyl-6a,7,8,9-tetrahydro-3H-pyrrolo[2',1':2,3][1,3]oxazino[6,5-g][1,2,3] benzotriazine-4,11-dione

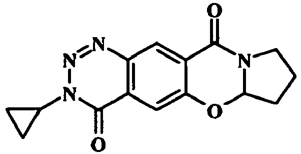

The nitroaldehyde derivative (see example 1; 0.73 g, 2.8 mmol) was dissolved in DMF (20 mL) and oxone (1.97 g, 3.2 mmol) was added. The mixture was stirred over night. Chloroform (50 mL) was added and the mixture was filtered. The filtrate was concentrated under high vacuum. The residue was dissolved in methylene chloride (30 mL). Thionyl chloride (2 mL) and DMF (5 drops) were added and the mixture was stirred over night. The mixture was concentrated under vacuum to remove the DMF and the residue was suspended in methylene chloride (30 mL) and allowed to settle. Cyclopropylamine (2 mL) and triethylamine (2 mL, 14.4 mmol) were dissolved in methylene chloride (30 mL). The acid chloride solution was slowly added leaving behind the solids. The solution was stirred for 1 hour. The methylene chloride solution was washed with 1N HCl (2 × 20 mL) followed by sodium bicarbonate solution (2 × 20 mL). It was dried over magnesium sulfate and concentrated to give 0.61 g of a beige solid.

The reduction of the nitro group to the amine and the following ring closure was carried out as in Example 3 giving 148 mg of light pink crystals with the following properties: MP: 215 - 217 °C; ¹H NMR (300 MHz, CDCl₃) δ 8.74 (1H, s), 7.86 (1H, s), 5.61 (1H, m), 4.0-3.6 (3H, m), 2.6-1.9 (4H, m) and 1.4-1:1 ppm (4H, m).

### EXAMPLE 5

### 3-Ethyl-6a,7,8,9-tetrahydro-3H-pyrrolo[2',1':2,3][1,3]oxazino|6,5-g][1,2,3]benzotriazine-4,11-dione

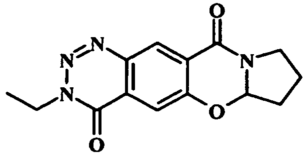

The synthesis was carried out as in Example 3 using 0.70 g (2.52 mmol) of the nitro acid derivative and substituting ethylamine for cyclobutylamine. The reaction gave 0.329 mg of a beige solid with the following properties: MP: 186 - 188 °C; ¹H NMR (300 MHz, CDCl₃) δ 8.75 (1H, s) 7.86 (1H, s) 5.61 (1H, m) 4.51 (2H, m) 4.0-3.6 (2H, m) 2.6-1.9 (4H, m) and 1.51 ppm (3H, m).

### EXAMPLE 6

### 3-(Cyclopropylmethyl)-6a,7,8,9-tetrahydro-3H-pyrrolo[2',1':2,3][1,3]oxazino[6,5-g][1,2,3]benzotriazine-4,11-dione

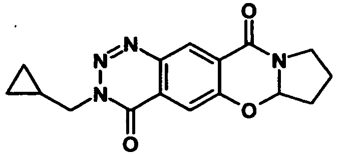

A suspension of 7-nitro-9-oxo-1,2,3,3a-tetrahydro-9*H*-pyrrolo[2,1-*b*]benzoxazine-6-carboxylic acid (from Example 1, 1.12 g, 3.82 mmol) in chloroform (15 mL) was treated with thionyl chloride (2.0 mL, 27.4 mmol) and DMF (0.1 mL). The mixture was heated at reflux for 30 min. The volatiles were removed under reduced pressure. A solution of the residue in chloroform (10 mL) was treated with cyclopropanemethylamine (1.0 mL, 11.5 mmol) and stirred at room temperature for 1 h. The reaction mixture was poured into 1 M sodium bicarbonate (50 mL), stirred for 10 min and extracted with chloroform (2 x 50 mL). The extract was dried over sodium sulfate, and the solvent was removed under reduced pressure to give 943 mg of amide.

A solution of the obtained amide (382 mg, 1.15 mmol) in ethanol/dichloromethane (50 + 50 mL) was hydrogenated (10% Pd/C, 100 mg) at 50 psi for 24 h. The solution was filtered through a pad of celite, and the solvent was removed under reduced pressure. The residue was dissolved in DMF (15 mL), and the solution was treated with isoamyl nitrite (3 mL) and acetic acid (0.5 mL). After 18 h at room temperature, the volatiles were removed under reduced pressure, and the residue was chromatographed (chloroform/ ethyl acetate, 80 : 20) to give the triazinone (125 mg) as a white solid with MP: 139 - 140°C. ¹H NMR (CDCl₃) δ 0.51 (2 H, m), 0.59 (2 H, m), 1.45 (1 H, m), 2.02 (1 H, m), 2.19 (1 H, m), 2.35 (1 H, m), 2.53 (1 H, m), 3.67 (1 H, ddd, J = 5.1, 8.1, and 12.9 Hz), 3.93 (1 H, dt, J = 11.7 and 7.2 Hz), 4.30 (2 H, m), 5.61 (1 H, t, J = 6.0 Hz), 7.86 (1 H, s), and 8.76 (1 H, s).

### EXAMPLE 7

### 3-tert-Butyl-6a,7,8,9-tetrahydro-3H-pyrrolo[2',1':2,3][1,3]oxazino[6,5-g][1,2,3] benzotriazine-4,11-dione

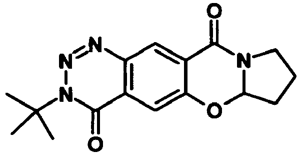

A suspension of the nitro acid (from Example 1, 1.15 g, 4.16 mmol) in chloroform (15 mL) was treated with thionyl chloride (2.0 mL, 27.4 mmol) and DMF (0.1 mL). The mixture was heated at reflux for 30 min. The volatiles were removed under reduced pressure. A solution of the residue in chloroform (20 mL) was cooled in an ice/methanol bath and treated with *tert-*butylamine (2.0 mL, 19.0 mmol) added dropwise. The mixture was stirred at room temperature for 24 h, then poured into 1 M sodium bicarbonate (50 mL), stirred for 20 min and extracted with chloroform (2 x 50 mL). The extract was dried over magnesium sulfate, and the solvent was removed under reduced pressure to give 1.25 g of amide.

A solution of the obtained amide (1.25 g, 3.75 mmol) in ethanol/dichloromethane (100 + 100 mL) was hydrogenated (10% Pd/C, 250 mg) at 50 psi for 24 h. The solution was filtered through a pad of celite, and the solvent was removed under reduced pressure. The residue was dissolved in DMF (20 mL), and the solution was treated with isoamyl nitrite (3 mL) and acetic acid (0.5 mL). After stirring for 3 days at room temperature, the volatiles were removed under reduced pressure, and the residue was chromatographed (chloroform/ ethyl acetate, 1 : 1) to give the triazinone (241 mg) as a white solid with MP: 224 - 225°C. ¹H NMR (CDCl₃) δ 1.80 (9 H, s), 2.02 (1 H, m), 2.18 (1 H, m), 2.34 (1 H, m), 2.52 (1 H, m), 3.69 (1 H, ddd, J = 4.8, 8.1, and 12.1 Hz), 3.92 (1 H, dt, J = 12.1 and 7.3 Hz), 5.59 (1 H, t, J = 6.3 Hz), 7.83 (1 H, s), and 8.71 (1 H, s).

### EXAMPLE 8

### 3-(Dimethylamino)-6a,7,8,9-tetrahydro-3H-pyrrolo[2',1':2,3][1,3]oxazino[6,5-g][1,2,3]benzotriazine-4,11-dione

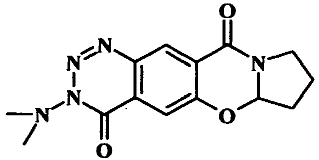

N,N-Dimethylhydrazine (10 mL) and triethylamine (10 mL, 72 mmol) were dissolved in methylene chloride (50 mL) and a solution of the acid chloride derivative (1.5 g, 5 mmol, see example 3) in methylene chloride (10 mL) was added. The mixture was stirred for 18 hr after which dilute sulfuric acid (100 mL, pH ~3) was added. The intermediate was extracted with chloroform (2 × 100 mL), dried over sodium sulfate, and dried under vacuum. The residue was dissolved in a mixture of THF (50 mL) and methanol (50 mL). The solution was added to freshly prepared Zn/Cu reagent (10 g, see example 3). Formic acid (40 drops) was added and the mixture was stirred at ambient temperature for 15 minutes after which the reduction was complete. The mixture was filtered, the filtrate was evaporated to dryness then dissolved in DMF (40 mL). An excess of isoamyl nitrite (4 mL) was added and the mixture was stirred over night. The DMF was removed under vacuum and the product was purified using flash chromatography (ethyl acetate: chloroform: methanol, 50:48:2). The combined product fractions were concentrated under vacuum then crystallized from chloroform:methyl-t-butyl ether to give a white crystalline material (101 mg) with the following properties: MP: 180 °C (turns brown); ¹H NMR (300 MHz, CDCl₃) δ 8.77 (1H, s) 7.91 (1H, s) 5.61 (1H, m) 4.0-3.6 (2H, m) 3.08 (6H, s) and 2.6-1.9 ppm (4H, m).

### EXAMPLE 9

### 3-Prop-2-yn-1-yl-6a,7,8,9-tetrahydro-3H-pyrrolo[2',1':2,3][1,3]oxazino[6,5-g][1,2,3]benzotriazine-4,11-dione

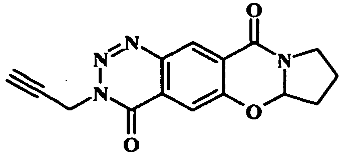

The synthesis was carried out as in Example 3 using 730 mg (2.6 mmol) of the nitro acid derivative and substituting (1g) propargylamine for cyclobutylamine. The product was purified using flash chromatography (ethyl acetate: chloroform: hexane, 70:10:20). The combined product fractions were concentrated under vacuum then crystallized from methylenechloride:methyl-t-butyl ether to give 308 mg of a white solid with the following properties: MP: 186 - 188 °C, but turns brown above 160 °C; ¹H NMR (300 MHz, CDCl₃) δ 8.78 (1H, s), 7.88 (1H, s), 5.62 (1H, m), 5.19 (2H, d, J=2.7 Hz), 4.0-3.6 (2H, m) and 2.6-1.9 ppm (5H, m).

### EXAMPLE 10

### (4,11-Dioxo-4,6a,7,8,9,11-hexahydro-3H-pyrrolo[2',1':2,3][1,3]oxazino[6,5-g][1,2,3]benzotriazin-3-yl)acetonitrile

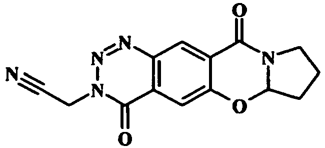

The synthesis was carried out as in Example 3 using 1.0 g (3.6 mmol) of the nitro acid derivative and substituting aminoacetonitrile bisulfate (1.94g) for cyclobutylamine. The product was purified using flash chromatography (ethyl acetate: hexane, 75:25) The combined product fractions were concentrated under vacuum then crystallized from methylenechloride:methyl-t-butyl ether to give 222 mg of a white solid with the following properties: MP: 210 - 212 °C; ¹H NMR (300 MHz, CDCl₃) δ 8.80 (1H, s), 7.88 (1H, s), 5.65 (1H, m), 5.28 (2H, s), 4.0-3.6 (2H, m) and 2.6-1.9 (4H, m).

### EXAMPLE 11

### 2-(4,11-Dioxo-4,6a,7,8,9,11-hexahydro-3H-pyrrolo[2',1':2,3][1,3]oxazino[6,5-g][1,2,3]benzotriazin-3-yl)propanenitrile

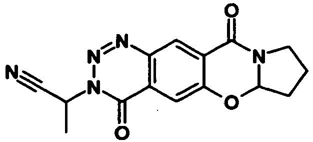

A suspension of the nitrile from Example 10 (570 mg, 1.92 mmol) in DMF (5 mL) was treated with methyl iodide (0.4 mL, 6.4 mmol) and 60% NaH in mineral oil (84 mg, 2.1 mmol). The obtained mixture was stirred under argon at 20°C for 3 h. The volatiles were removed under reduced pressure, and the residue was chromatographed (chloroform/ethyl acetate, 8 : 2) to separate the monomethylated product from the dimethyl derivative and the starting material. The obtained mixture of diastereomers was carefully rechromatographed using the same eluent. The material from fractions enriched in more polar diastereomer was recrystallized from ethanol (70 mL) to give the monomethylated triazinone (59 mg) as white powder, MP: 220 - 221°C. ¹H NMR (CDCl₃) δ 1.97 (3 H, d, J = 6.9 Hz), 2.02 (1 H, m), 2.18 (1 H, m), 2.36 (1 H, m), 2.54 (1 H, m), 3.69 (1 H, ddd, J = 5.1, 8.4, and 12.3 Hz), 3.92 (1 H, dt, J = 11.7 and 7.2 Hz), 5.63 (1 H, t, J = 5.7 Hz), 6.05 ( 1 H, q, J = 6.9 Hz), 7.87 (1 H, s), and 8.81 (1 H, s).

Concentration of the fractions enriched in the less polar diastereomer gave white crystals (12 mg), MP: 200 - 201°C. ¹H NMR (CDCl₃) δ 1.98 (3 H, d, J = 6.9 Hz), 2.02 (1 H, m), 2.18 (1 H, m), 2.36 (1 H, m), 2.54 (1 H, m), 3.69 (1 H, ddd, J = 5.1, 8.4, and 12.3 Hz), 3.92 (1 H, dt, J = 11.7 and 7.2 Hz), 5.64 (1 H, t, J = 5.7 Hz), 6.07 (1 1 H, q, J = 6.9 Hz), 7.87 (1 H, s), and 8.81 (1 H, s).

### EXAMPLE 12

### 2-(4,11-Dioxo-4,6a,7,8,9,11-hexahydro-3H-pyrrolo[2'.1':2,3][1,3]oxazino[6,5-g][1,2,3]benzotriazin-3-yl)-2-methylpropanenitrile

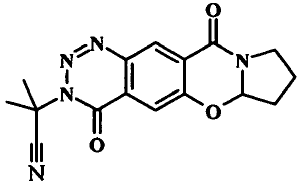

The nitro acid derivative (1.1 g, 4.0 mmol, see example 1) was suspended in 50 ml chloroform, 4 ml thionylchloride and 30 drops DMF were added, and the mixture was stirred for 18 hours at room temperature. After evaporation of the solvent, the residue was dissolved in 10 ml chloroform. This solution was slowly added to a mixture of 500 mg (5.94 mmol) 2-amino-2-methylpropanenitrile, 2 ml NEt₃ and 60 ml chloroform, which was stirred at 25 °C for 60 min. The solution was diluted with 100 ml chloroform, washed with dilute sulfuric acid (pH 2, 100 mL) followed by sodium bicarbonate solution (100 mL). The aqueous phases were extracted with chloroform (100 mL). The organic phases were combined, dried over sodium sulfate and concentrated under vacuum.

The residue was dissolved in methanol/THF/DMF (30 mL/30 mL/ 30 mL). The reduction of the nitro group (using 10g freshly prepared Zn/Cu reagent) and the following ring closure (using isoamyl nitrite) was carried out as in Example 3. The crude product was purified using flash chromatography (50 g silica gel, ethyl acetate/chloroform/hexanes 50/40/10 → chloroform/THF 60/40). The product fractions were combined and concentrated under vacuum, which caused the product to crystallize (614 mg). The resulting off white material had the following properties: MP: 240 - 242 °C; ¹H NMR (300 MHz, CDCl₃) δ 8.77 (1H, s), 7.89 (1H, s), 5.63 (1H, t, J = 6.0 Hz), 3.98-3.89 (1H, m), 3.74-3.66 (1H, m), 2.15 (6H, s) and 2.61-1.97 ppm (4H, m).

### EXAMPLE 13

### 3-[(2-Methyl-2H-tetrazol-5-yl)methyl]-6a,7,8,9-tetrahydro-3H pyrrolo[2',1':2,3] [1,3]oxazino[6,5-g][1,2,3]benzotriazine-4,11-dione and 3-[(1-methyl-1H-tetrazol-5-yl)methyl]-6a,7,8,9-tetrahydro-3H-pyrrolo[2',1':2,3][1,3]oxazino[6,5-g][1,2,3] benzotriazine-4,11-dione

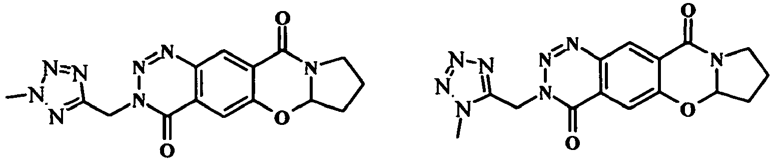

The nitrile (example 10) derivative (368 mg, 1.24 mmol) was suspended in 15 ml toluene and 15 mL DMF. 322 mg (4.95 mmol) NaN₃ and 500 mg Et₃N·HCl were added, and the mixture was stirred for 18 hours at 115°C. After evaporation of the solvent, water (50 mL) was added to the residue and the pH was adjusted to 2 using HCl. The aqueous phase was extracted with ethylacetate (2x100 mL) and chloroform/MeOH 95/5 (100 mL). The organic phases were combined, dried over magnesium sulfate and concentrated under vacuum. Trituration with MeOH yielded 160 mg beige solid.

The residue was dissolved in DMF (5 mL), 160 mg K₂CO₃ and 0.3 mL methyliodide were added and heated to 45°C for 18 hours. The solvent was evaporated and 30 mL water were added.. The aqueous phase was extracted with methylenechloride (2x50 mL), the organic phases were combined, dried over magnesium sulfate and concentrated under vacuum. The crude product was purified using flash chromatography (20 g silica gel, ethyl acetate/chloroform 3/1). The product fractions were combined and concentrated under vacuum.

The less polar product was crystallized from methylenechloride/ether/hexane to yield 87 mg of a white solid which had the following properties: MP: 166 - 167 °C; ¹H NMR (300 MHz, CDCl₃) δ 8.79 (1H, s), 7.86 (1H, s), 5.89 (2H, s), 5.62 1H, t), 4.32 (3H, s), 3.98-3.87 (1H, m), 3.75-3.64 (1H, m) and 2.60-1.93 ppm (4H, m).

The more polar product was triturated with ether to yield 56 mg of a white solid which had the following properties: MP: 254 - 255 °C; ¹H NMR (300 MHz, DMSO + CDCl₃) δ 8.74 (1H, s), 7.81 (1H, s), 5.89 (2H, s), 5.66 1H, t), 4.29 (3H, s), 3.95-3.85 (1H, m), 3.75-3.62 (1H, m) and 2.60-1.96 ppm (4H, m).

### EXAMPLE 14

### 3-(2-Cyclohex-1-en-1-ylethyl)-6a,7,8,9-tetrahydro-3H-pyrrolo[2',1':2,3][1,3]oxazino[6,5-g][1,2,3]benzotriazine-4,11-dione

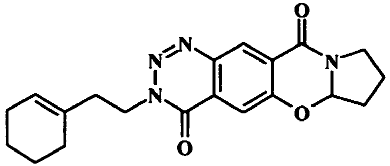

The nitro acid derivative (1.0 g, 3.60 mmol, see example 1) was dissolved in DMF (18 mL) followed by CDI (0.76 mg, 3.96 mmol) and then 2-(1-cyclohexenyl)ethylamine (0.55 mL, 3.96 mmol). Triethylamine (1.5 mL, 11 mmol) was added to the solution and it was stirred for 4 hr. Dichloromethane (100 mL) was added and the solution was washed with saturated sodium bicarbonate solution and dried over sodium sulfate. The solvent was removed under vacuum and the residue was purified on a silica gel column (dichloromethane:methanol, 98:2) to give 568 mg of intermediate.

The ring closure reaction, using the freshly made Zn/Cu reagent and isoamyl nitrite, was carried out as in Example 3. The crude product was purified on a silica gel column (chloroform:THF, 19:1). The product fractions were combined and concentrated under vacuum. The residue was triturated with diethyl ether to give a solid (105 mg) with the following properties: MP: 124 -124.5 °C; ¹H NMR (300 MHz, DMSO) δ 8.74 (1H, s), 7.84 (1H, s), 5.61 (1H, m), 5.37 (1H, broad), 4.52 (2H, m), 3.91 (1H, m), 3.69 (1H, m) and 2.6-1.4 ppm (14H, m).

### EXAMPLE 15

### 3-(2-Cyclohexylethyl)-6a,7,8,9-tetrahydro-3H-pyrrolo[2',1':2,3][1,3]oxazino[6,5-g][1,2,3]benzotriazine-4,11-dione

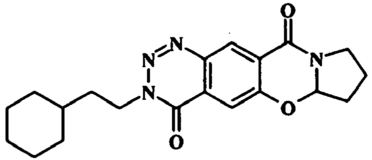

N-Ethylcyclohexylamine hydrochloride was synthesized from 2-(1-cyclohexenyl) ethylamine in the following manner: The amine (300 mg, 2.4 mmol) was dissolved in methanol (25 mL). Palladium (10% on carbon, 150 mg) and acetic acid (0.25 mL) were added. The mixture was hydrogenated at ambient temperature for 3 days. The reaction was about 75% complete according to NMR. An additional 150 mg of catalyst was added and hydrogenation was continued for 4 hr. The mixture was filtered to remove the catalyst and the filtrate was concentrated under vacuum. A solution of HCl in dichloromethane was added to exchange the acetate counter ion for HCl, and the mixture was concentrated under vacuum. This step was repeated several times until a white crystalline product formed.

The triazinone synthesis was carried out in the same manner as in Example 14, with the substitution of N-ethylcyclohexylamine hydrochloride for 2-(1-cyclohexenyl)ethylamine, starting with 667 mg (2.4 mmol) of the nitro acid derivative. The product was recrystallized from methylene chloride:diethyl ether to give 38 mg of a crystalline material with the following properties: MP: 169.5 - 170.5 °C; ¹H NMR (300 MHz, DMSO) δ 8.75 (1H, s), 7.85 (1H, s), 5.60 (1H, m), 4.47 (2H, m), 3.91 (1H, m), 3.69 (1H, m) and 2.6-0.9 ppm (17H, m).

### EXAMPLE 16

### (6aR)-3-[(2S)-1-(3,5-Difluorophenyl)but-3-yn-2-yl]-6a,7,8,9-tetrahydro-3H-pyrrolo [2',1':2,3][1,3]oxazino[6,5-g][1,2,3]benzotriazine-4,11-dione

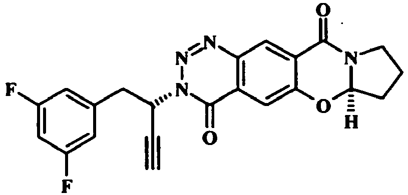

### (S)-[2-(3,5-Difluorophenyl)-1-(methoxymethylcarbamoyl)ethyl]carbamic acid tert-butyl ester

To a well stirred solution of Boc-3,5-difluoro-L-phenylalanine (5.0 g, 16.0 mmol) in dichloromethane (50 mL) at 0 °C under nitrogen was added *N*,*N*-carbonyldiimidazole (3.10 g, 19.1 mmol) as a solid. Carbon dioxide evolution was observed and this mixture was allowed to warm to ambient temperature and stirred for 3 hr. Solid N,O-dimethylhydroxylamine hydrochloride (2.06 g, 20.7 mmol) was added, the reaction mixture was again cooled to 0 °C and triethylamine (2.89 mL, 20.7 mmol) was slowly added via a syringe. The cloudy slurry was diluted with dichloromethane (20 mL) to give a clear solution that was allowed to warm to ambient temperature and stirred for 1 hour. The reaction mixture was poured into 10% citric acid (75 mL), the layers separated and the aqueous phase was extracted with dichloromethane (4x50mL). The organic phases were combined, dried over sodium sulfate and concentrated to give the crude product as a solid. This material was subjected to chromatography on silica gel (Merck Kieselgel 60, 230-400 mesh, 350 g, elution with 30% EtOAc/hexane) to give 5.65 g of (*S*)-[2-(3,5-difluorophenyl)-1-(methoxymethyl-carbamoyl)-ethyl]-carbamic acid tert-butyl ester as a white crystalline solid. ¹H NMR (400 MHz, CDCl₃) δ 1.41 (9 H, s), 2.85 (1 H, dd, J=13.5, 7.46 Hz), 3.05 (1 H, dd, J=13.6 and 5.49 Hz), 3.21 (3 H, s), 3.74 (3 H, s), 4.92 (1 H, m), 5.24 (1 H, d, J=8.29 Hz), 6.69 ppm (3 H, m)

### (S)-[1-(3,5-Difluorobenzyl)-2-oxoethyl]carbamic acid tert-butyl ester

To a cold (-45 °C; ACN/CO₂ bath) well stirred slurry of (S)-[2-(3,5-difluorophenyl)-1-(methoxymethyl-carbamoyl)-ethyl]-carbamic acid tert-butyl ester (5.65 g, 16.6 mmol) in dry diethyl ether (180 mL) was slowly added LiAlH₄ (1.0 M in diethyl ether, 21 mmol) via a syringe. This mixture was stirred at -45 °C for 1 hour and slowly and carefully quenched with a solution of potassium bisulfate (3.94 g, 29 mmol) in water (100 mL). This slurry was allowed to warm to ambient temperature, diluted with ethyl acetate (100 mL) and filtered through a Celite plug. The solids were washed with ethyl acetate and the filtrates combined. The organic phase was washed with 10% citric acid, saturated sodium bicarbonate, brine, dried over sodium sulfate and concentrated. This gave 4.65g of (*S*)-[1-(3,5-difluorobenzyl)-2-oxo-ethyl]-carbamic acid tert-butyl ester as a white solid.

### (S)-[3,3-Dibromo-1-(3,5-difluorobenzyl)allyl]carbamic acid tert-butyl ester

A flame dried 500 mL SN flask under nitrogen was charged with triphenylphosphine (17.4 g, 66.2 mmol), carbon tetrabromide (11.0 g, 33.1 mmol) and dichloromethane (100 mL). This well-stirred mixture was cooled to -32 °C and a solution of [2-(3,5-difluorophenyl)-1-formyl-ethyl]-carbamic acid tert-butyl ester (4.65 g, 16.5 mmol) in dichloromethane (100 mL) added dropwise over a period of 1 hour. The reaction mixture was allowed to stir an additional 2 hr at -32 °C and quenched with saturated sodium bicarbonate solution (50 mL). The mixture was allowed to warm to ambient temperature, the layers separated and the aqueous phase extracted with dichloromethane (3x50 mL). The organic phases were combined, dried over sodium sulfate and filtered through a silica gel plug (50g, elution with dichloromethane). The filtrate was concentrated and chromatographed on silica gel (Merck Kieselgel 60; 230-400mesh, 300g, elution with dichloromethane) to give 2.95g of a white solid. The product was recrystallized from MTBE/hexane to give 1.79 g of enantiomerically pure (S)-[3,3-dibromo-1-(3,5-difluorobenzyl)-allyl]-carbamic acid tert-butyl ester as a fine white crystalline product. ¹H NMR (400 MHz, CDCl₃) δ 1.43 (9 H, s), 2.83 (1 H, m), 2.96 (1 H, m), 4.45 (1 H, m), 4.64 (1 H, m), 6.43 (1 H, m) and 6.76 ppm (3 H, m). MS (ESI+) for C₁₅H₁₇Br₂F₂NO₂ m/z 463.7 (M+Na)⁺.

### (S)-[1-(3,5-Difluorobenzyl)prop-2-ynyl]carbamic acid tert-butyl ester

To a cold (at -45 °C) well-stirred solution of (S)-[3,3-dibromo-1-(3,5-difluorobenzyl)-allyl]-carbamic acid tert-butyl ester (1.72 g, 3.9 mmol) in dry tetrahydrofuran (40 mL) under nitrogen was added 1.43 M n-butyllithium in hexane (8.7 mL) dropwise over a period of 15 min. The reaction mixture was allowed to stir at -45 °C for 2.5 hr and subsequently quenched with saturated ammonium chloride (30 mL). This mixture was diluted with ethyl acetate (100 mL) and allowed to warm to ambient temperature. The layers were separated, the aqueous phase was extracted with additional ethyl acetate (3 × 30 mL) and the organic phases were combined, dried over sodium sulfate and concentrated. The residue was chromatographed on silica gel (Isco Redisep 40g, elution with 20% ethyl acetate/hexane) to give 650 mg of purified product. The mixed fractions were re-chromatographed on silica gel (Isco Redisep 40g, elution with 10% ethyl acetate/hexane) and gave an additional 355 mg. Combination of the products provided 1.05g of (S)-[1-(3,5-difluorobenzyl)-prop-2-ynyl]-carbamic acid tert-butyl ester as a white solid. ¹H NMR (400 MHz, CDCl₃) δ ppm 1.46 (9 H, s), 2.34 (1 H, s), 2.98 (2 H, m), 4.70 (2 H, m), 6.73 (1 H, m), 6.82 (2 H, m). (ESI+) for C₁₅H₁₇F₂NO₂ m/z 304.1 (M+Na)⁺.

(S)-[1-(3,5-Difluorobenzyl)prop-2-ynyl]carbamic acid tert-butyl ester (0.96 g, 3.4 mmol) was dissolved in chloroform (25 mL) and trifluoroacetic acid (4 mL) was added. After one hour the solvent was evaporated by vacuum and methylene chloride (30 mL, saturated with HCl gas) was added, then removed by vacuum. The last step was repeated once to give a white solid which was dissolved in DMF (10 mL) and set aside. The nitro acid derivative from Example 1 (1.0 g, 4.0 mmol) was dissolved in DMF (40 mL) followed by DMAP (0.42 g, 3.4

mmol), HOBT (0.45 g, 3.3 mmol), triethylamine (2 mL, 14.4 mmol), and EDCI (3.0 g, 16 mmol). The solution was heated to 46 °C for 15 min, after which the DMF solution of the white solid was added. The mixture was stirred at 46 °C for 4 days. The DMF was removed under vacuum, water (100 mL) was added and the pH was adjusted to 2 using 2M sulfuric acid. The product was extracted with ethyl acetate (2 × 150 mL), washed with sodium bicarbonate solution (100 mL), dried over sodium sulfate, and concentrated under vacuum to yield a 1:1 mixture of diastereomers (1.5 g, 3.4 mmol).

The diastereomer mixture from the previous step (0.75 g, 1.7 mmol) was dissolved in a mixture of THF (50 mL) and methanol (25 mL).

The fresh Zn/Cu reagent (10g, see example 3) was added to the diastereomer solution and stirred at ambient temperature. Glacial acetic acid (45 drops) was added and the mixture was stirred for 15 min. The solids were removed by filtration, washed with a mixture of THF (20 mL) and methanol (10 mL) and concentrated under vacuum. The yellow solid was dissolved in DMF (50 mL) and about 10 mL of the solvent was distilled off. An excess of isoamyl nitrite (10 mL) was added. After 4 hr at ambient temperature TLC (ethyl acetate/chloroform, 65/35) shows incomplete reaction. Another 5 mL of isoamyl nitrite was added and the mixture was left overnight. The DMF was removed under vacuum, and the product was purified using flash chromatography with ethyl acetate/chloroform/hexane, 4/3/3, as the mobile phase. The solvent was removed under vacuum and the product crystallized from methylene chloride/methyl tert-butyl ether to give a white solid (306 mg).

NMR shows trace impurities. The product was further purified using MPLC with THF/chloroform/hexane, 35/15/50, as the mobile phase. The solvent was removed under vacuum and the product crystallized from methylene chloride/methyl tert-butyl ether to give 54 mg of the more polar isomer with the following properties: MP: 120 - 143 °C; ¹H NMR (300 MHz, CDCl₃) δ 8.77 (1H, s), 7.81 (1H, s), 6.79 (2H, m), 6.66 (1H, m), 6.08 (1H, m), 5.61 (1H, m), 4.0-3.6 (2H, m), 3.48 (2H, m), 2.49 (1H, s) and 2.6-1.9 ppm (4H, m).

### EXAMPLE 17

### 3-[(2S)-1-(3-Fluoroohenyl)but-3-yn-2-yl]-6a,7,8,9-tetrahydro-3H-pyrrolo[2',1':2,3] [1,3]oxazino[6,5-g][1,2,3]benzotriazine-4,11-dione

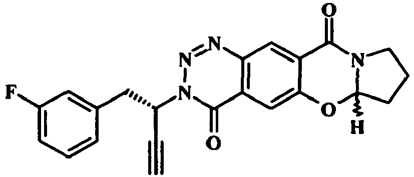

### (S)-[2-(3-Fluorophenyl)-1-(methoxymethylcarbamoyl)ethyl]carbamic acid tert-butyl ester

To a well stirred solution of Boc-3-fluoro-L-phenylalanine (2.00 g, 7.06 mmol in dichloromethane (10 mL), at ambient temperature under nitrogen, was added N,N-carbonyldiimidazole (1.32 g, 8.14 mmol) as a solid. Carbon dioxide evolution was observed and the mixture was stirred at ambient temperature for 3 hour. Solid N,O-dimethylhydroxylamine hydrochloride (0.878 g, 8.82 mmol) was added followed by the slow addition of triethylamine (1.23 mL, 8.82 mmol) via syringe. The cloudy slurry was diluted with dichloromethane (5mL) to give a clear solution that was stirred at ambient temperature for 1 hour. The reaction mixture was poured into 10% citric acid (20 mL) and extracted with dichloromethane (4x20mL). The organic phases were combined, dried over sodium sulfate and concentrated to give the crude product as a solid. This material was subjected to chromatography on silica gel (Merck Kieselgel 60, 230-400mesh, 90 g, elution with 25% EtOAc/hexane) to give 2.25 g of (*S*)-[2-(3-fluorophenyl)-1-(methoxymethyl-carbamoyl)-ethyl]-carbamic acid tert-butyl ester as a white crystalline solid. ¹H NMR (400 MHz, CDCl₃) δ 1.41 (9 H, s), 2.87 (1 H, dd, J=13.58, 7.36 Hz), 3.07 (1 H, dd, J=13.6, 5.70 Hz), 3.20 (3 H, s), 3.71 (3 H, s), 4.94 (1 H, m), 5.20 (1 H, d, J=8.71 Hz), 6.93 (3 H, m), 7.26 ppm (1 H, m). MS (ESI+) for C₁₆H₂₃FN₂O₄ m/z 349.1 (M+Na)⁺

### (S)-[1-(3-Fluorobenzyl)-2-oxoethyl]carbamic acid tert-butyl ester

To a cold (-45 °C; ACN - dry ice bath) well stirred slurry of (*S*)-[2-(3-fluorophenyl)-1-(methoxymethylcarbamoyl)ethyl]carbamic acid tert-butyl ester (11.46 g, 35.1 mmol) in dry diethyl ether (300 mL) was added LiAlH₄ (1.0 M in diethyl ether, 43.9 mmol) slowly via syringe. This mixture was stirred at -45 °C for 1 hour and slowly, carefully quenched with a solution of potassium bisulfate (8.37 g, 61.4 mmol) in water (80 mL). This slurry was allowed to warm to ambient temperature, diluted with ethyl acetate (200 mL) and filtered through a Celite plug. The solids were washed with ethyl acetate and the filtrates combined and separated. The organic phases were washed with 10% citric acid, saturated sodium bicarbonate, and brine, dried over sodium sulfate and concentrated. This provided 9.30g of (*S*)-[1-(3-fluorobenzyl)-2-oxo-ethyl]-carbamic acid tert-butyl ester as a white solid. ¹H NMR (400 MHz, CDCl₃) δ 1.45 (9 H, s), 3.14 (2 H, m), 4.43 (1 H, m), 5.08 (1 H, m), 6.96 (3 H, m), 7.28 (1 H, m), 9.65 ppm (1 H, s). MS (ESI-) for C₁₄H₁₈FNO₃ m/z 266.2 (M-H)⁻

### (S)-[3,3-Dibromo-1-(3-fluorobenzyl)allyl]carbamic acid tert-butyl ester

To a cold (-33 °C; FTS Flexicool) well-stirred mixture of carbon tetrabromide (23.3 g, 70.2 mmol), triphenylphosphine (36.8 g, 0.140 mol) and dichloromethane (500 mL) under nitrogen was dropwise added a solution of (*S*)-[1-(3-fluorobenzyl)-2-oxo-ethyl]-carbamic acid tert-butyl ester (9.38 g, 35.1mmol) in dichloromethane (100 mL). Stirring was continued for I hour, the reaction was quenched with saturated sodium bicarbonate (100 mL) and extracted with methylene chloride (3 × 100mL). The organic phases were combined, dried over sodium sulfate, and passed through a silica gel plug (∼100 g). This plug was washed with additional dichloromethane (200 mL), the filtrates were combined and concentrated. The residue was chromatographed on silica gel (Merck Kieselgel 60, 230-400 mesh, 350 g, elution with dichloromethane) and the mixed fractions re-chromatographed similarly. The pure fractions from both columns were combined to give 10.05 g of (*S*)-[3,3-dibromo-1-(3-fluorobenzyl)-allyl]-carbamic acid tert-butyl ester as a white solid. The product was re-crystallized from 1:1 hexane/ether to provide 4.4 g of enantiomerically pure product. ¹H NMR (400 MHz, CDCl₃) δ 1.43 (9 H, s), 2.91 (2 H, m), 4.49 (1 H, m), 4.57 (1 H, m), 6.41 (1 H, m), 6.95 (3 H, m), 7.31 ppm (1 H, m). MS (ESI+) for C₁₅H₁₈Br₂FNO₂ m/z 445.9 (M+Na)⁺

### (S)-[1-(3-Fluorobenzyl)prop-2-ynyl]carbamic acid tert-butyl ester

To a cold (-78 °C), well stirred solution of (S)-[3,3-dibromo-1-(3-fluorobenzyl)allyl]carbamic acid tert-butyl ester (4.25 g, 10.0 mmol) in dry tetrahydrofuran (50 mL) was added 1.35 M of n-butyllithium in hexane (23.8 mL) over a period of 15 min. After 2.5 hr at -78 °C, the reaction was quenched with saturated ammonium chloride (30 mL) and diluted with diethyl ether (100 mL). This mixture was allowed to warm to ambient temperature and extracted with diethyl ether (4×100 mL). The organic phases were combined, dried over sodium sulfate and concentrated. The residue was chromatographed on silica gel (Merck Kieselgel 60, 230-400 mesh, 150 g, elution with 20% ethyl acetate/hexane) to give 2.45 g of (S)-[1-(3-fluorobenzyl)-prop-2-ynyl]-carbamic acid tert-butyl ester which solidified under vacuum. ¹H NMR (400 MHz, CDCl₃) δ 1.45 (9 H, s), 2.32 (1 H, s), 2.97 (2 H, m), 4.70 (2 H, m), 7.01 (3 H, m), 7.30 ppm (1 H, m). ¹³C NMR (CDCl₃) δ 162.67 (d, J = 246 Hz), 154.52, 138.84 (d, J = 7.3 Hz), 129.68 (d, J = 8.1 Hz), 125.43 (d, J = 2.9 Hz), 116.68 (d, J = 21.2 Hz), 113.81 (d, J = 20.5 Hz), 82.38, 80.15, 72.50, 43.70, 41.42, 28.29 ppm (3C). MS (ESI+) for C₁₅H₁₈FNO₂ m/z 286.1 (M+Na)+

(S)-[1-(3-Fluorobenzyl)prop-2-ynyl]carbamic acid tert-butyl ester (1.1 g, 4.2 mmol) was dissolved in chloroform (25 mL) and trifluoroacetic acid (4 mL) was added. After one hour the solvent was evaporated by vacuum and methylene chloride (20 mL, saturated with HCl gas) was added, then removed by high vacuum. The last step was repeated once to give a solid which was dissolved in DMF (10 mL) and set aside. The nitro acid derivative from Example 1 (1.2 g, 4.8 mmol) was dissolved in DMF (50 mL) followed by DMAP (0.50 g, 4.1 mmol), HOBT (0.54 g, 4.0 mmol), triethylamine (2 mL, 14.4 mmol), and EDCI (3.0 g, 16 mmol). The solution was heated to 46 °C after which the DMF solution of the solid was added. The mixture was stirred at 46 °C for 18 hr. The DMF was removed under vacuum, water (200 mL) was added and the pH was adjusted to 2 using 2M sulfuric acid. The product was extracted with ethyl acetate (200 mL) and washed with sodium bicarbonate solution (100 mL). The acidic phase was extracted a second time with ethyl acetate (2 × 150 mL) and the product was washed with sodium bicarbonate solution (100 mL). The organic phases were combined, dried over sodium sulfate, and concentrated under vacuum to yield a yellow foam (1.9 g, <4.2 mmol) with some impurities.

The product from the previous step (1.91 g, <4.2 mmol) was dissolved in a mixture of THF (25 mL) and methanol (50 mL). The Zn/Cu reagent was prepared according to the procedure in Example 3. The fresh Zn/Cu reagent was added to the above solution and stirred at ambient temperature. Glacial acetic acid (90 drops) was added over 10 min and the mixture was stirred for an additional 5 min. The solids were filtered off, first washed with a mixture of THF (35 mL) and methanol (35 mL), then DMF (50 mL). The filtrate was concentrated to dryness under vacuum. The solid was dissolved in DMF (60 mL) and isoamyl nitrite (5 mL) was added. After 18 hr at ambient temperature, DMF was removed under vacuum, and the product was purified using flash chromatography with ethyl acetate/hexane, 35/65, as the mobile phase. The solvent was removed under vacuum to give a yellow solid (1.3 g) which was re crystallized from methylene chloride/methyl tert-butyl ether to give a white solid. The crystallization was repeated once using methylene chloride/methanol. The product was further purified using flash chromatography with chloroform/ethyl acetate, 90/10, as the mobile phase. The solvent was removed under vacuum and the product crystallized from methylene chloride/methanol to give 500 mg of a 1:1 mixture of the two isomers with the following properties: MP: 171 - 173 °C; ¹H NMR (300 MHz, CDCl₃) δ 8.76 (1H, s), 7.79 (1H, s), 7.4-6.8 (4H, m), 6.11 (1H, m), 5.60 (1H, m), 4.0-3.6 (2H, m), 3.48 (2H, m), 2.48 (1H, s) and 2.6-1.9 ppm (4H, m).

### EXAMPLE 18

### 3-[(1S)-2-(3-Fluorophenyl)-1-isoxazol-3-ylethyl]-6a,7,8,9-tetrahydro-3H-pyrrolo [2',1':2,3][1,3]oxazino[6,5-g][1,2,3]benzotriazine-4,11-dione

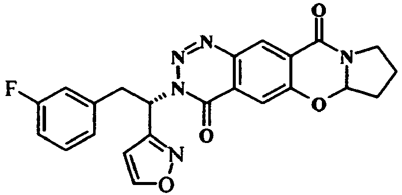

8.5 g (32 mmol) (*S*)-[1-(3-fluorobenzyl)-2-oxo-ethyl]-carbamic acid tert-butyl ester (see example 17) was dissolved in 300 ml MeOH and 21.0 g (140 mmol) of K₂CO₃ and 9.17 g (133 mmol) of NH₂OH.HCl (dissolved in 160 ml water) were added at 0 °C. The mixture was stirred overnight. The total volume was then reduced to one third by evaporation followed by extraction with 2 × 50 mL methylene chloride. The organic solution was washed with water, dried and evaporated. The residue was recrystallized from hexane : EtOAc (1 : 1) to yield 2.35 g oxime.

333 mg (2.5 mmol) of *N*-chlorosuccinimide (NCS) was added to a solution of 1.13 g (4 mmol) of oxime in 5 mL of abs. DMF. After 30 minutes when the color of the reaction mixture changed to light green a second portion of 333 mg of NCS was added and the reaction was kept with stirring for 3 h at room temp. To this mixture 20 mL of ice water was added then it was extracted with 3 × 20 mL ether. The extract was washed with water, dried and evaporated to afford 1.3 g oil which solidified on standing.

1.3 g (4 mmol) Hydroxamic acid chloride was dissolved in 20 mL toluene. 860 mg (10 mmol) ethyl vinyl ketone in 5 mL toluene was added dropwise during 10 min. Then 500 mg triethyl amine in 10 mL toluene was added at rate of 2 mL/hr. The mixture was then quenched by addition of 3 mL 1N HCl. After stirring for an additional hour, the organic phase was separated and washed with saturated NaHCO₃, with water and dried. After evaporation the product was crystallized from a mixture of EtOAc : Hexane yielding 190 mg isoxazoline. Treatment of this material with concentrated HCl yielded 45 mg isoxazole.

The nitro acid derivative (100 mg, 0.36 mmol, see example 1) was dissolved in DMF (15 mL) to which DMAP (25 mg, 0.20 mmol), HOBT (27 mg, 0.20 mmol), triethylamine (0.5 mL) and EDCI (700 mg, 3.6 mmol) and 45 mg (0.18 mmol) isoxazole were added. The mixture was stirred at 25 °C for 18 hours. The DMF was removed under vacuum and ethyl acetate (50 mL) was added. The solution was washed with dilute sulfuric acid (pH 2, 50 mL) followed by sodium bicarbonate solution (50 mL). The aqueous phases were extracted with ethyl acetate (50 mL). The organic phases were combined, dried over sodium sulfate and concentrated under vacuum to give 125 mg amide, which was used in the next step without further purification.

The product from the previous step (125 mg) was dissolved in a mixture of THF (25 mL) and methanol (25 mL). The Zn/Cu reagent (2g) was prepared according to the procedure in Example 3. The fresh Zn/Cu reagent was added to the above solution and stirred at ambient temperature. Formic acid (0.3 mL) was added and the mixture was stirred for 20 minutes at 25°C. The solids were filtered off, washed with a mixture of THF (5 mL) and methanol (5 mL) and the filtrate was concentrated to dryness under vacuum. The solid was dissolved in DMF (10 mL) and isoamyl nitrite (1 mL) was added. After 18 hr at ambient temperature, DMF was removed under vacuum, and the product was purified using flash chromatography with ethyl acetate/hexane, 60/40, as the mobile phase. The solvent was removed under vacuum, and the material was re crystallized from methylene chloride/methyl tert-butyl ether to yield an off white solid (45 mg) as a 1:1 mixture of the two isomers with the following properties: MP: 170 - 180 °C.; ¹H NMR (300 MHz, CDCl₃) δ 8.73 (1H, s), 8.39 (1H, d, J = 1.5 Hz), 7.76 (1H, s), 7.20-6.72 (4H, m), 6.50 (1H, d, J = 1.5 Hz), 5.58 (1H, t, J = 6.0 Hz), 3.95-3.64 (4H, m), 3.17-3.08 (1H, m), and 2.56-1.95 ppm (4H, m).

### EXAMPLE 19

### (2S)-2-[(6aS)-4,11-Dioxo-4,6a,7,8,9,11-hexahydro-3H-pyrrolo[2',1':2,3][1,3]oxazino[6,5-g][1,2,3]benzotriazin-3-yl]-3-(3-fluorophenyl)-N-methylpropanamide and (2S)-2-[(6aR)4,11-dioxo-4,6a,7,8,9,11-hexahydro-3H-pyrrolo[2',1':2,3][1,3]oxazino[6,5-g][1,2,3]benzotriazin-3-yl]-3-(3-fluorophenyl)-N-methylpropanamide

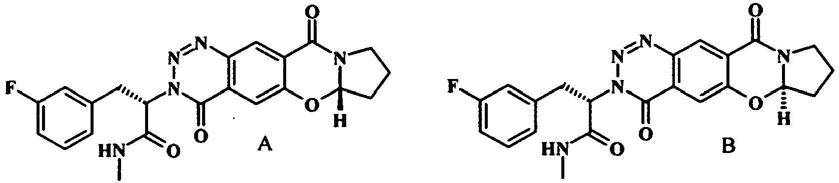

To a well stirred solution of Boc-3-fluoro-L-phenylalanine (1.41 g, 5.0 mmol, Peptech) in chloroform (30 mL), at ambient temperature, was added *N*,*N*-carbonyldiimidazole (1.3 g, 8.0 mmol) as a solid. Carbon dioxide evolution was observed and the mixture was stirred at ambient temperature for 1.5 hours. A solution of ∼5ml Methylamine in10 ml THF was added and the mixture was stirred for 10 minutes. The reaction mixture was poured into 100 ml water, acidified with sulfuric acid (→ pH 2) and extracted with chloroform (2x 75mL). The organic phases were combined, dried over sodium sulfate and concentrated to give the crude product. This material was subjected to chromatography on silica gel (Merck Kieselgel 60, 230-400mesh, 50 g, elution with EtOAc/Chloroform 1/1) to give 1.48 g (quant.) of (*S*)-[2-(3-fluorophenyl)]-1oxo-1-(methyamino)-carbamic acid tert-butyl ester as a white crystalline solid.

The amide from the previous step (1.0 g, 3.3 mmol) was dissolved in chloroform (40 mL) and trifluoroacetic acid (5 mL) was added. After 35 minutes the solvent was evaporated by vacuum and methylene chloride (80 mL, saturated with HCl gas) was added, then removed by high vacuum. The last step was repeated once to give a solid which was dissolved in DMF (60 mL), the nitro acid derivative from Example 1 (977 mg, 3.5 mmol) was added, followed by DMAP (416 mg, 3.4 mmol), HOBT (460 mg, 3.4 mmol), triethylamine (2 mL, 14.4 mmol), and EDCI (2.0 g, 10.6 mmol). The mixture was stirred at 45 °C for 18 hr. The DMF was removed under vacuum, water (100 mL) was added and the pH was adjusted to 2 using 2M sulfuric acid. The product was extracted with ethyl acetate (100 mL) and washed with sodium bicarbonate solution (100 mL). The acidic phase was extracted a second time with ethyl acetate (100 mL) and the product was washed with sodium bicarbonate solution (100 mL). The organic phases were combined, dried over sodium sulfate, and concentrated under vacuum to yield a yellow foam. The product was further purified using flash chromatography with ethyl acetate as the mobile phase. The solvent was removed under vacuum to give 1046 mg of a 1:1 mixture of the two isomers.

The product from the previous step (754 mg, 1.65 mmol) was dissolved in a mixture of THF (50 mL) and methanol (50 mL). The Zn/Cu reagent (10g) was prepared according to the procedure in Example 3. The fresh Zn/Cu reagent was added to the above solution and stirred at ambient temperature. Formic acid (3 ml) was added and the mixture was stirred for an additional 15 min. The solids were filtered off, first washed with a mixture of THF (35 mL) and methanol (35 mL). The filtrate was concentrated to dryness under vacuum. The solid was dissolved in DMF (30 mL) and isoamyl nitrite (4 mL) was added. After 18 hr at ambient temperature, DMF was removed under vacuum, and the product was purified using flash chromatography with ethyl acetate as the mobile phase.

The solvent was removed under vacuum to give the less polar Isomer A as a white solid (210 mg) with the following properties: MP: 147 -150 °C; ¹H NMR (300 MHz, CDCl₃) δ 8.49 (1H, s), 7.63 (1H, s), 7.15-6.75 (4H, m), 6.71 (1H, m), 5.88 (1H, dd, J = 5.4 and 10.2 Hz), 5.49 (1H, t, J = 6.3 Hz), 3.92-3.50 (4H, m), 2.84 (3H, d, J = 4.8 Hz), 2.55-1.90 ppm (4H, m).

The more polar isomer B was crystallized from methylenchloride/MTBE to give a white solid (125 mg) with the following properties: MP: 196 - 197 °C; ¹H NMR (300 MHz, CDCl₃) δ 8.72 (1H, s), 7.75 (1H, s), 7.19-6.81 (4H, m), 6.11 (1H, m), 5.77 (1H, dd, J = 6.5 and 9.0 Hz), 5.60 (1H, t, J = 5.7 Hz), 3.95-3.60 (4H, m), 2.81 (3H, d, J = 4.8 Hz), 2.60-1.96 ppm (4H, m).

### EXAMPLE 20

### (2S)-2-[(6aS)-4,11-Dioxo-4,6a,7,8.9,11-hexahydro-3H-pyrrolo[2',1':2,3][1,3]oxazino[6,5-g][1,2,3]benzotriazin-3-yl]-3-(3-fluorophenyl)propanamide and (2S)-2-[(6aR)-4,11-dioxo-4,6a,7,8.9,11-hexahydro-3H-pyrrolo[2',1':2,3][1,3]oxazino[6,5-g][1,2,3] benzotriazin-3-yl]-3-(3-fluorophenyl)propanamide

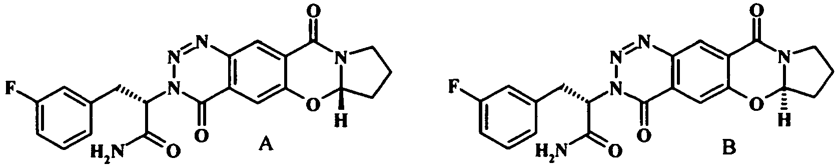

To a well stirred solution of Boc-3-fluoro-L-phenylalanine (1.5 g, 5.3 mmol) in chloroform (30 mL), at ambient temperature, was added N,N-carbonyldiimidazole (1.5 g, 9.3 mmol) as a solid. Carbon dioxide evolution was observed and the mixture was stirred at ambient temperature for 1.5 hours. A solution of∼10ml Ammonia in 30 ml THF was added and the mixture was stirred for 10 minutes. The reaction mixture was poured into 250 ml water, acidified with sulfuric acid (→ pH 2) and extracted with chloroform (100 mL). The organic phase was dried over sodium sulfate and concentrated to give 1.5g white (quant.) product.

Following all steps of the previous example (19), a 1:1 mixture of amides was obtained, which were purified using flash chromatography with ethyl acetate as the mobile phase.

The solvent was removed under vacuum to give the less polar isomer A as a white solid (570 mg) with the following properties: MP: 135-139 °C; ¹H NMR (300 MHz, CDCl₃) δ 8.44 (1H, s), 7.61 (1H, s), 7.15-6.76 (5H, m), 5.94 (1H, dd, J = 5.4 and 10.5 Hz), 5.69 (1H, NH), 5.47 (1H, t, J = 6.0 Hz), 3.92-3.51 (4H, m) and 2.55-1.92 ppm (4H, m).

The more polar isomer B was crystallized from AcOEt/MTBE to give a white solid (585 mg) with the following properties: MP: degradation > 191 °C; ¹H NMR (300 MHz, CDCl₃) δ 8.70 (1H, s), 7.75 (1H, s), 7.20-6.81 (4H, m), 6.12 (1H, NH), 5.82 (1H, dd, J = 8.1 and 9.6 Hz), 5.60 (1H, t, J = 5.7 Hz), 5.60 (1H, NH), 3.95-3.62 (4H, m) and 2.56-1.96 ppm (4H, m).

### EXAMPLE 21

### (2S)-2-[(6aR)-4,11-Dioxo-4,6a,7,8,9,11-hexahydro-3H-pyrrolo[2',1':2,3][1,3]oxazino[6,5-g][1,2,3]benzotriazin-3-yl]-3-(3-fluorophenyl)propanenitrile

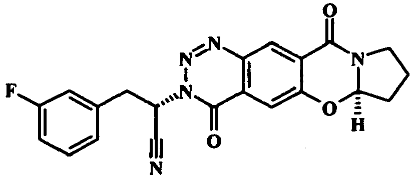

Dissolve 290 mg (0.68 mmol) of isomer B from the previous experiment (20) in chloroform (25 mL), add 600 mg (2.5 mmol) Burgess reagent as a solid and stir for 3 days at 25°C. Evaporate the mixture onto 5g silica gel, put the material on top of a column (45g) and elute with AcOEt/Hexane 65/35. Evaporation of the product fractions yields a colorless oil. Crystallization from methylenechloride/MTBE yields 210 mg white crystals with the following properties: MP: 182 -184 °C; ¹H NMR (300 MHz, CDCl₃) δ 8.77 (1H, s), 7.81 (1H, s), 7.30-6.94 (4H, m), 6.14 (1H, t, J = 8.1 Hz), 5.64 (1H, t, J = 6.0 Hz), 3.97-3.52 (4H, m) and 2.61-1.97 ppm (4H, m).

### EXAMPLE 22

### (6aS)-3-[(2S)-1-(3-Fluorophenyl)-3-hydroxypropan-2-yl]-6a,7,8,9-tetrahydro-3H-pyrrolo[2',1':2,3][1,3]oxazino[6,5-g][1,2,3]benzotriazine-4,11-dione and (6aR)-3-[(2S)-1-(3-fluorophenyl)-3-hydroxypropan-2-yl]-6a,7,8,9-tetrahydro-3H-pyrrolo[2',1':2,3] [1,3]oxazino[6,5-g][1,2,3]benzotriazine-4,11-dione

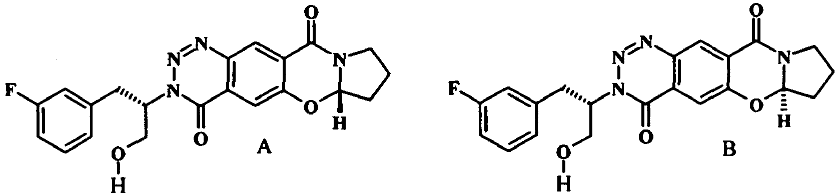

To a solution of 1g LiAlH₄ in 15 ml THF was added a solution of 2.7g (*S*)-[1-(3-fluorobenzyl)-2-oxo-ethyl]-carbamic acid tert-butyl ester in 5 ml THF. After the addition, the solution was refluxed for 1 hour. Then 1 mL water, 1 mL 15% NaOH and 3 ml water were added. The solids were filtered off and the THF solution was evaporated to yield 2.3g alcohol in form of a white solid.

The alcohol from the previous step (700 mg, 2.6 mmol) was dissolved in chloroform (100 mL) and trifluoroacetic acid (4 mL) was added. After 30 minutes the solvent was evaporated by vacuum and methylene chloride (150 mL, saturated with HCl gas) was added, then removed by high vacuum. The last step was repeated once to give a solid which was dissolved in DMF (80 mL), the nitro acid derivative from Example 1 (723 mg, 2.6 mmol) was added, followed by DMAP (317 mg, 2.6 mmol), HOBT (351 mg, 2.6 mmol), triethylamine (1 mL, 7.2 mmol), and EDCI (1.34 g, 7.0 mmol). The mixture was stirred at 45 °C for 18 hr. The DMF was removed under vacuum, water (100 mL) was added and the pH was adjusted to 2 using 2M sulfuric acid. The product was extracted with ethyl acetate (100 mL) and washed with sodium bicarbonate solution (100 mL). The acidic phase was extracted a second time with ethyl acetate (100 mL) and the product was washed with sodium bicarbonate solution (100 mL). The organic phases were combined, dried over sodium sulfate, and concentrated under vacuum to yield an orange oil. The product was further purified using flash chromatography with ethyl acetate as the mobile phase. The solvent was removed under vacuum to give 700 mg of a ∼1:1 mixture of the two isomers.

The product from the previous step (700 mg, 1.6 mmol) was dissolved in a mixture of THF (30 mL) and methanol (30 mL). The Zn/Cu reagent (7g) was prepared according to the procedure in Example 3. The fresh Zn/Cu reagent was added to the above solution and stirred at ambient temperature. Formic acid (1 ml) was added and the mixture was stirred for an additional 15 min. The solids were filtered off, washed with a mixture of THF (50 mL) and methanol (50 mL) and the filtrate was concentrated to dryness under vacuum. The solid was dissolved in DMF (70 mL) and isoamyl nitrite (4 mL) was added. After 18 hr at ambient temperature, DMF was removed under vacuum, and the product was purified using flash chromatography with THF/Hexane/CHCl₃ (50/30/20) as the mobile phase.

The solvent was removed under vacuum (∼ 1mL, let crystallize) to give the less polar isomer A as a white solid (120 mg) with the following properties: MP 172 -174 °C; ¹H NMR (300 MHz, CDCl₃) δ 8.62 (1H, s), 7.73 (1H, s), 7.22-6.82 (4H, m), 5.56-5.49 (2H, m), 4.25-4.07 (2H, m), 3.96-3.87 (1H, m), 3.67-3.60 (1H, m), 3.30 (2H, d, J = 7.5 Hz), 2.85-1.94 ppm (5H, m).

The more polar isomer B was crystallized accordingly, to give a white solid (110 mg) with the following properties: MP: 181 -183 °C; ¹H NMR (300 MHz, CDCl₃) δ 8.73 (1H, s), 7.79 (1H, s), 7.22-6.83 (4H, m), 5.60 (1H, t, J = 6.0 Hz), 5.50-5.41 (1H, m), 4.18-4.06 (2H, m), 3.96-3.87 (1H, m), 3.73-3.65 (1H, m), 3.31 (2H, d, J = 8.1 Hz), 2.59-1.94 ppm (5H, m).

### EXAMPLE 23

### 3-[1-(3-Nitrophenyl)but-3-yn-2-yl]-6a,7,8,9-tetrahydro-3H-pyrrolo[2',1':2,3][1,3] ozazino[6,5-g][1,2,3]benzotriazine-4,11-dione

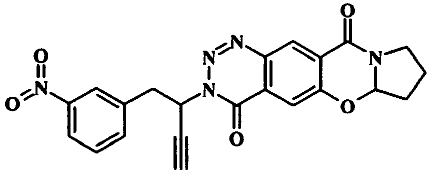

The nitro methyl ester derivative (0.7 g, 2.4 mmol, see example 1) was dissolved in a mixture of warm methanol:THF (100 mL, 3:1) and fresh Zn/Cu reagent (10 g, prepared according to the procedure in Example 3) was added followed by formic acid (50 drops) during vigorous stirring. After 10 min, the Zn/Cu reagent was removed by filtration and washed with methanol (50 mL). The filtrate was evaporated onto silica gel (∼5 g), which was transferred to a column (50 g silica gel). The intermediate product was eluted with chloroform:ethyl acetate (70:30). The combined product fractions were concentrated under vacuum to give 520 mg of yellow powder.

The powder from the previous step was dissolved in methanol (50 mL) and a solution of potassium hydroxide (1.0 g) in water (10 mL) was added. The mixture was stirred for 3 hr, after which an aqueous solution of hydrochloric acid (pH∼2) was added. The methanol and water were removed under high vacuum. DMF (200 mL) was added and then evaporated under vacuum to give the anthranilic acid intermediate, which was used in the coupling with [1-(3-nitrobenzyl)prop-2-ynyl]carbamic acid tert-butyl ester.

### (S)-[1-Hydroxymethyl-2-3-nitrophenyl)ethyl]carbamic acid tert-butyl ester

To a well stirred slurry of (S)-2-tert-butoxycarbonylamino-3-(3-nitrophenyl)propionic acid (3.0 g, 9.7 mmol) and Py-BOP (6.29 g, 12.1 mmol) in tetrahydrofuran (40 mL) under dry nitrogen at ambient temperature was added N,N-diisopropylethylamine (2.10 mL, 12.1 mmol). After 2h, sodium borohydride (0.475 g, 12.6 mmol) was added and stirring continued for 2h. The reaction mixture was concentrated, the residue was re-dissolved in ethyl acetate (100 mL) and washed with 10% citric acid, saturated sodium bicarbonate and brine. The organic phases were dried over anhydrous sodium sulfate and concentrated. Chromatography on silica gel (Silicycle, 230-400 mesh, 150 g, elution with 30% EtOAc/hexane) gave 2.45 g as a white solid.

### (S)-[1-Formyl-2-(3-nitrophenyl)-ethyl]carbamic acid tert-bulyl ester

A slurry of (S)-[1-hydroxymethyl-2-(3-nitrophenyl)-ethyl]-carbamic acid tert-butyl ester (2.25 g, 7.59 mmol) and Dess-Martin periodinane (6.76 g, 15.9 mmol) in wet methylene chloride (90 mL) was stirred at ambient temperature for 1h. TLC (1:1 EtOAc/hexane) suggested complete consumption of the starting material. The reaction was diluted with diethyl ether (100 mL) and quenched with a solution containing 13 g of sodium thiosulfate in 80% saturated sodium bicarbonate solution (100 mL). This mixture was stirred rapidly until both phases were clear. The layers were separated and the aqueous phase was extracted with ethyl acetate (3x50 mL). The organics were combined, washed with saturated sodium bicarbonate, water, and brine. The solution was dried over anhydrous sodium sulfate and concentrated to give the crude aldehyde, 2.23 g, as an oil that was utilized immediately without further purification.

### [1-(3-Nitrobenzyl)prop-2-ynyl]carbamic acid tert-butyl ester

To a well stirred slurry of potassium carbonate (3.14 g, 22.7 mmol) and 4-acetamidobenzenesulfonyl azide (2.73 g, 11.4 mmol) in anhydrous acetonitrile (80 mL) at ambient temperature under nitrogen was slowly added dimethyl 2-oxopropylphosphonate (1.57 mL, 11.4 mmol) via syringe. The mixture was stirred at ambient temperature for 2h. A solution of (S)-[1-formyl-2-(3-nitrophenyl)ethyl]carbamic acid tert-butyl ester (2.23 g, 7.6 mmol) in anhydrous methanol/acetonitrile (1:2, 60 mL) was then dropwise added over a period of 10 min. The mixture was stirred at ambient temperature overnight. The next day the mixture was concentrated, partitioned between ethyl acetate/water (100 mL, 1:1), and the aqueous phase was extracted with ethyl acetate (3×40 mL). The organic phases were combined, washed with 10% citric acid, saturated bicarbonate and brine. The solution was dried over anhydrous sodium sulfate and concentrated. The residue was dissolved in DCM (15 mL) and loaded onto a 150 g medium pressure silica gel column. The product was eluted with 20% ethyl acetate/hexane to give 930 mg (3.2 mmol) of clear, colorless oil that solidified on standing.

The solidified oil (581 mg, 2 mmol) was dissolved in methylene chloride (10 mL) and TFA (3 mL) was added. The mixture was left at ambient temperature for 1 h. The solvent was removed under vacuum and methylene chloride (30 mL) saturated with HCl (gas) was added. The last step was repeated once and the solvent was removed under vacuum. The residue was dissolved in DMF (80 mL) and the amino acid intermediate (see above) was added together with DMAP (245 mg, 2 mmol), HOBT (270 mg, 2 mmol), triethyl amine (2 mL) and EDCI (2 g). The mixture was stirred at ambient temperature for three days and then concentrated under high vacuum. Water (200 mL, pH∼2 with sulfuric acid) was added and the mixture was extracted with ethyl acetate (4 × 150 mL). The organic fractions were combined, washed with saturated sodium bicarbonate solution (100 mL), dried over sodium sulfate and concentrated under vacuum to give 950 mg of yellow solid. The solid was dissolved in DMF (50 mL) and isoamyl nitrite (5 mL) was added. The mixture was stirred at ambient temperature for 5 h. The solvent was removed under vacuum and the residue was transferred to a column (100 g silica gel). The product was eluted with ethyl acetate:hexanes, 65:35 (500 mL) followed by chloroform:THF, 80:20. The fractions containing the desired product were combined and concentrated under vacuum. The residue was recrystallized from chloroform:ethyl acetate to give 250 mg of an off white solid with the following properties: MP: 212 - 214 °C (decomposes); ¹H NMR (300 MHz, CDCl₃) δ 8.77 (1H, s), 8.20-8.05 (2H, m) 7.803 (0.5H, s), 7.795 (0.5H, s), 7.60 (1H, m), 7.44 (1H, m), 6.14 (1H, m), 5.61 (1H, m), 4.00-3.86 (1H, m), 3.76-3.50 (3H, m) and 2.6-1.9 ppm (5H, m).

### EXAMPLE 24

### 3-[(1S,2R)-1-Hydroxy-1-phenylpropan-2-yl]-6a,7,8,9-tetrahydro-3H-pyrrolo[2',1':2,3] [1,3]oxazino[6,5-g][1,2,3]benzotriazine-4,11-dione

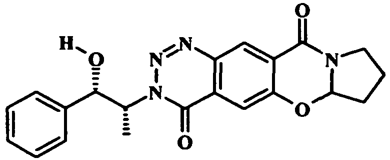

The nitro acid (405 mg, 1.46 mmol, see example 1) was suspended in dichloromethane, triethylamine (406 µL, 2.92 mmol) was added, resulting in a homogenous solution. (1S,2R)-2-amino-1-phenyl-1-propanol (200 mg, 1.32 mmol) and HBTU (608 mg, 1.61 mmol) were added and the mixture was stirred for 45 min. The mixture was washed with HCl (1 M) and dried over sodium sulfate.

The ring closure reaction, using freshly made Zn/Cu reagent and isoamyl nitrite, was carried out as in Example 3. The crude product was purified on a silica gel column (dichloromethane:methanol, 98:2). The product fractions were combined and concentrated under vacuum. The residue was triturated with hexanes to give 65 mg of a pale yellow solid with the following properties: MP: 235 -238 °C; ¹H NMR (300 MHz, CDCl₃) δ 8.73 (1H, s), 7.78 (1H, m), 7.5 - 7.2 (5H, m), 5.60 (1H, m), 5.42 (1H, m), 5.27 (1H, m), 3.92 (1H, m), 3.69 (1H, m), 3.22 (1H, m), 2.52 (1H, m), 2.34 (1H, m), 2.19 (1H, m), 2.05 (1H, m) and 1.59 ppm (3H, m).

### EXAMPLE 25

### Erythro-3-[1-(3-fluorophenyl)-1-hydroxypropan-2-yl]-6a,7,8,9-tetrahydro-3H-pyrrolo [2',1':2,3][1,3]oxazino[6,5-g][1,2,3]benzotriazine-4,11-dione

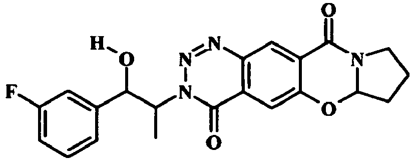

The nitro acid (223 mg, 0.82 mmol, see example 1) was suspended in dichloromethane, then DIPEA (428 µL, 2.46 mmol) was added, resulting in a homogenous solution. Racemic (erythro)-2-amino-1-(3-fluorophenyl)-1-propanol (300 mg, 0.98 mmol, synthesized according to: EP960876, Annalen 1929, 470, 168 and J.org.chem. 1982, 47, 2643-7 and crystallized from acetonitrile) and HBTU (371 mg, 0.98 mmol) were added and the mixture was stirred for 45 min. The mixture was washed with HCl (1 M) and dried over sodium sulfate.

The ring closure reaction, using freshly made Zn/Cu reagent and isoamyl nitrite, was carried out as in Example 3. The crude product was purified on a silica gel column (dichloromethane:methanol, 99:1 → 96:4). The product fractions were combined and concentrated under vacuum. The residue was triturated with hexanes to give 76 mg of a pale yellow solid with the following properties: MP: 197-204 °C; ¹H NMR (300 MHz, CDCl₃) δ 8.75 (1H, s), 7.82 (1H, s), 7.30-6.90 (4H, m), 5.63-5.59 (1H, m), 5.42-5.34 (1H, m), 5.30-5.25 (1H, m), 3.97-3.88 (1H, m), 3.74-3.66 (1H, m), 3.44 (1H, OH), 2.60-1.92 (4H, m) and 1.59-1.55 ppm (3H, m).

### EXAMPLE 26

### 3-{[1-(3-Fluorophenyl)cyclopropyl]methyl}-6a,7,8,9-tetrahydro-3H-pyrrolo[2',1':2,3] [1,3]oxazino[6,5-g][1,2,3]benzotriazine-4,11-dione

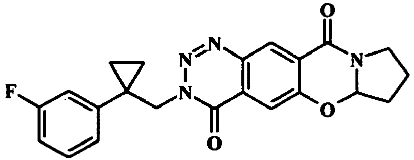

The synthesis of the nitro acid derivative was carried out in the same manner as in Example 1.

The synthesis of 1-(3-fluorophenyl)cyclopropyl methylamine was carried out as follows:
To a well stirred slurry of sodium hydride (5.90 g, 0.246 mol, oil-free, hexane washed) in dimethyl sulfoxide (150 mL, 2.1 mol) at ambient temperature under nitrogen was added 3-fluoro-benzeneacetonitrile (11.4 mL, 0.0983 mol) dropwise via a syringe. After 30 min, 1,2-dibromoethane (12.7 mL, 0.148 mol) was added over a period of 1h and stirring was continued for an additional 4h. The reaction was poured into ice-water (300 mL) and extracted with diethyl ether (4 × 100 mL). The extracts were combined, dried over anhydrous sodium sulfate and concentrated. This gave 14.4 g (85 mmol) of crude 1-(3-fluorophenyl)cyclopropane carbonitrile as a reddish solid, which was used without further purification.

To a cold (0 °C) well-stirred solution of 1-(3-fluorophenyl)cyclopropane carbonitrile (14.4 g, 85 mmol) in dry tetrahydrofuran (200 mL) under dry nitrogen was added lithium aluminum hydride in diethyl ether (89.1 mL) slowly over a period of 1h. The reaction mixture was stirred at 0 °C for 2h, diluted with diethyl ether (200 mL) and then carefully quenched (gas evolution!) sequentially with water (3.2 mL), 10% NaOH (3.2 mL) and water (9.6 mL). The reaction mixture was filtered, washed with diethyl ether, and the filtrate was extracted with 0.4 M HCl (4 × 100 mL). These extracts were combined and washed with diethyl ether (3 × 50 mL). The acidic aqueous layers were made basic with solid NaOH (6.4 g) and then extracted with diethyl ether (4 × 100 mL). The ether extracts were combined, washed with water (50 mL), brine (50 mL) and dried over anhydrous magnesium sulfate. The slurry was filtered and concentrated to provide 9.80 g (59 mmol) of the desired 1-(3-fluorophenyl)cyclopropylmethylamine as a reddish oil, which was used without further purification.

The nitro acid derivative (695 mg, 2.5 mmol) was suspended in methylene chloride (15 mL). DMF (10 drops) was added followed by thionyl chloride (1.8 mL, 25 mmol). The mixture was stirred over night. The resulting solution was concentrated under vacuum to remove the thionyl chloride. The residue was dissolved in methylene chloride (10 mL) then added to a solution of 1-(3-fluorophenyl)cyclopropylmethylamine (826 mg, 5.0 mmol) and triethylamine (1.4 mL, 10 mmol) in methylene chloride (10 mL). The mixture was stirred at ambient temperature for 2 hr. The reaction mixture was washed with 1M HCl and sat. sodium bicarbonate solution. The solution was dried over magnesium sulfate and concentrated under vacuum. The dry residue was suspended in a mixture of ethyl acetate:methylene chloride (1:1) and stirred briskly. A yellow solid (517 mg, 1.22 mmol) was obtained by filtration.

The reduction of the nitro group and the following ring closure reaction (using freshly made Zn/Cu reagent and isoamyl nitrite) was carried out as in Example 3. The crude product was purified on a silica gel column (50 g silica gel, first chloroform, then chloroform:ethyl acetate, 8:1). The product fractions were combined and concentrated under vacuum to give a yellow foam, which was recrystallized from methylene chloride/diethyl ether to give 380 mg of product with the following properties: MP: 162 - 163 °C; ¹H NMR (300 MHz, CDCl₃) δ 8.70 (1H, s), 7.76 (1H, s), 7.2 - 6.8 (4H, m), 5.60 (1H, m), 4.62 (2H, m), 3.95 - 3.64 (2H, m), 2.6 - 1.9 (4H, m), 1.21 (2H, m) and 0.98 ppm (2H, m).

### EXAMPLE 27

### 3-[(2R)-1-(3-Fluorophenyl)butan-2-yl]-6a,7,8,9-tetrahydro-3H-pyrrolo[2',1':2,3][1,3] oxazino[6,5-g][1,2,3]benzotriazine4,11-dione

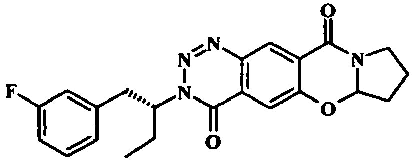

(S)-[1-(3-Fluorobenzyl)prop-2-ynyl]carbamic acid tert-butyl ester (728 mg, 2.76 mmol, see example 17) was dissolved in chloroform (30 mL). Palladium on carbon (10%, 400 mg) was added and the mixture was hydrogenated at ambient temperature for 18 hr. The catalyst was removed by filtration. Trifluoroacetic acid (4 mL) was added to the solution and it was left at ambient temperature for 1 hr. The solvent was removed under vacuum and methylene chloride (30 mL saturated with HCl gas) was added. The solvent was removed under vacuum and the last step was repeated once to give an oil that was used in the next step without further purification.

The nitro acid derivative (800 mg, 3.22 mmol, see example 1) was dissolved in DMF (30 mL) to which DMAP (330 mg, 2.7 mmol), HOBT (360 mg, 2.7 mmol), triethylamine (1 mL, 7.2 mmol) and EDCI (2.2 g, 11 mmol) were added. The mixture was stirred at 46 °C for 20 min. A solution of the oil (from the previous paragraph) in DMF (5 mL) was added and the mixture was stirred for 18 hr at 46 °C. The DMF was removed under vacuum and ethyl acetate (200 mL) was added. The solution was washed with dilute sulfuric acid (pH 2, 150 mL) followed by sodium bicarbonate solution (100 mL). The aqueous phases were extracted with ethyl acetate (150 mL). The organic phases were combined, dried over sodium sulfate and concentrated under vacuum to give 1.25 g of yellow oil, which was used in the next step without further purification.

The yellow oil (1.25 g, ≤ 1.76 mmol) was dissolved in methanol/THF (50 mL:30 mL). The reduction of the nitro group (using freshly prepared Zn/Cu reagent) and the following ring closure (using isoamyl nitrite) was carried out as in Example 3. The crude product was purified using flash chromatography (100 g silica gel, ethyl acetate:hexanes 50:50, 500 mL; 60:40, 250 mL; 70:30, 250 mL). The product fractions were combined and concentrated under vacuum to give a yellow oil, which crystallized overnight from a mixture of methylene chloride, diethyl ether and hexanes to give 510 mg of crystals with the following properties: MP: 117 - 119°C; ¹H NMR (300 MHz, CDCl₃) δ 8.72 (1H, s), 7.75 (1H, s), 7.2-6.2 (4H, m), 5.59 (1H, m), 5.36 (1H, m), 4.0-3.6 (2H, m), 3.4-3.1 (2H, m), 2.6-1.9 (6H, m) and 0.89 ppm (3H, m).

### EXAMPLE 28

### 3-[(1R)-1-Phenylethyl]-6a,7,8,9-tetrahydro-3H-pyrrolo[2',1':2,3][1,3]oxazino[6,5-g][1,2,3]benzotriazine-4,11-dione

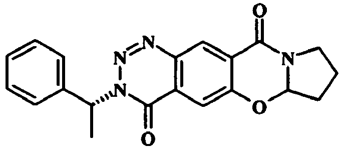

The nitro acid derivative from Example 1 (1.0 g, 3.6 mmol) was dissolved in 70 ml DMF, followed by DMAP (440 mg, 3.6 mmol), HOBT (486 mg, 3.6 mmol), triethylamine (2 mL), (R)-1-Phenyl ethylamine (1 mL) and EDCI (1.9 g, 10 mmol). The mixture was stirred at 25 °C for 18 hr. The DMF was removed under vacuum, water (100 mL) was added and the pH was adjusted to 2 using 2M sulfuric acid. The product was extracted with ethyl acetate (100 mL) and washed with sodium bicarbonate solution (100 mL). The water phases were reextracted with 100 mL ethyl acetate. The organic phases were combined, dried over sodium sulfate, and concentrated under vacuum.

The product from the previous step was dissolved in a mixture of THF (30 mL) and methanol (40 mL). The Zn/Cu reagent (10g) was prepared according to the procedure in Example 3. The fresh Zn/Cu reagent was added to the above solution and stirred at ambient temperature. Formic acid (2 ml) was added and the mixture was stirred for 10 min. The solids were filtered off and washed with methanol (50 mL). The filtrate was concentrated to dryness under vacuum. A mixture of DMF (30 mL) and isoamyl nitrite (5 mL) was added. After 18 hours at ambient temperature, DMF was removed under vacuum, and the product was purified using flash chromatography with AcOEt/hexane/CHCl₃ (40/40/20) as the mobile phase. The solvent was removed under vacuum to yield 206 mg (as a 1:1 mixture of isomers) white crystals with the following properties: MP: 112 - 119 °C; ¹H NMR (300 MHz, CDCl₃) δ 8.73 (1H, s), 7.82 (1H, s), 7.54-7.26 (5H, m), 6.43-6.34 (1H, m), 5.61-5.55 (1H, m), 3.96-3.87 (1H, m), 3.72-3.65 (1H, m) and 2.56-1.95 ppm (7H, m).

### EXAMPLE 29

### (6aS)-3-[(1R)-1-(3-Fluorophenyl)ethyl]-6a,7,8,9-tetrahydro-3H-pyrrolo[2',1':2,3] [1,3]oxazino[6,5-g][1,2,3]benzotriazine-4,11-dione and (6aR)-3-[(1R)-1-(3-fluorophenyl) ethyl]-6a,7,8,9-tetrahydro-3H-pyrrolo[2',1':2.3][1,3]oxazino[6,5-g][1,2,3]benzotriazine-4,11-dione

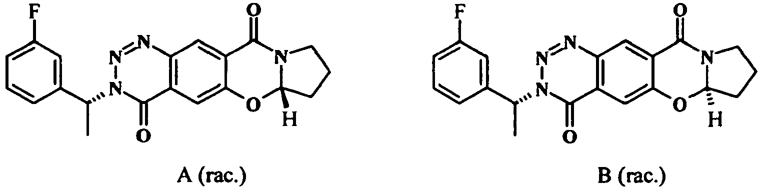

Dissolve 2.5g of 3-fluoro acetophenone in 50 ml MeOH, add a solution of 5.0 g hydroxylamine hydrochloride in 50 ml NaHCO₃ solution and stir for 18 hours. The reaction mixture was extracted with ethyl acetate (2x 150 mL), the organic layers were combined, dried over magnesium sulfate and concentrated under vacuum. The resulting oxime was dissolved in 50 mL THF, slowly added to a suspension of 4g LiAlH₄ in 50 mL ether and stirred for 2 hours. 30 ml Hexane were added and the reaction was quenched with 4 ml NaOH solution. A small amount of celite was added and the solids were filtered off and washed with a little THF. 3 mL NEt₃ were added and the solution was set aside.

In the meantime the nitro acid derivative (937 mg, 3.37 mmol, see example 1) was suspended in 50 ml chloroform, 4 ml thionylchloride and 30 drops DMF were added, and the mixture was stirred for 18 hours at room temperature. After evaporation of the solvent, the residue was dissolved in 10 ml chloroform. This solution was slowly added to the mixture of the amines, and then stirred at 25 °C for 18 hours. The solution was diluted with 150 ml chloroform, washed with dilute sulfuric acid (pH 2, 100 mL) followed by sodium bicarbonate solution (100 mL). The aqueous phases were extracted with chloroform (150 mL). The organic phases were combined, dried over sodium sulfate and concentrated under vacuum. The crude product was purified using flash chromatography (100 g silica gel, ethyl acetate/hexane 70/30 → 100/0). The product fractions were combined and concentrated under vacuum to yield 1.4g foam.

The residue was dissolved in methanol/THF (50 mL/50 mL). The reduction of the nitro group (using 10g freshly prepared Zn/Cu reagent, 1 mL HCOOH) and the following ring closure (using 4 mL isoamyl nitrite) was carried out as in Example 8. The crude product was purified using flash chromatography (125 g silica gel, ethyl acetate/chloroform/hexanes 45/20/35).

The product fractions containing the less polar isomer A (racemic) were combined and concentrated under vacuum, some MTBE was added, and the mixture was set aside, which caused product A to crystallize (254 mg). The resulting white material had the following properties: MP: 166-168 °C; ¹H NMR (300 MHz, CDCl₃) δ 8.74 (1H, s), 7.82 (1H, s), 7.32-6.92 (4H, m), 6.36 (1H, q, J = 6.9 Hz), 5.58 (1H, t, J = 6.0 Hz), 3.97-3.88 (1H, m), 3.73-3.65 (1H, m), 2.58-1.95 (4H, m) and 1.99 ppm (3H, d, J = 6.9 Hz).

The product fractions containing the more polar isomer B (racemic) were combined and concentrated under vacuum, some MTBE was added, and the mixture was set aside, which caused product B to crystallize (200 mg). The resulting white material had the following properties: MP: 201-203 °C; ¹H NMR (300 MHz, CDCl₃) δ 8.74 (1H, s), 7.82 (1H, s), 7.31-6.93 (4H, m), 6.35 (1H, q, J = 7.5 Hz), 5.60 (1H, t, J = 6.0 Hz), 3.96-3.87 (1H, m), 3.73-3.65 (1H, m), 2.58-1.95 (4H, m) and 1.99 ppm (3H, d, J = 7.5 Hz).

### EXAMPLE 30

### (6aS)-3-[(1R)-1-(3,5-Difluorophenyl)ethyl]-6a,7,8,9-tetrahydro-3H-pyrrolo[2',1':2,3] [1,3]oxazino[6,5-g][1,2,3]benzotriazine-4,11-dione and (6aR)-3-[(1R)-1-(3,5-difluorophenyl)ethyl]-6a,7,8,9-tetrahydro-3H-pyrrolo[2',1':2,3][1,3]oxazino[6,5-g][1,2,3]benzotriazine-4,11-dione

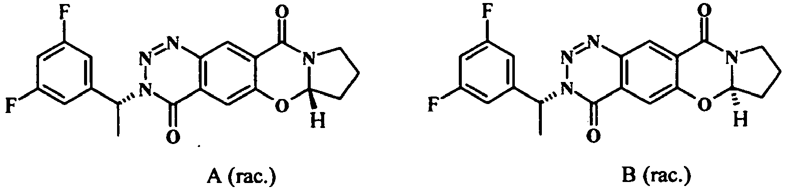

Both Isomers were synthesized, using the procedure from the previous example 29, starting with 3,5-difluoro acetophenone. The crude product was purified using flash chromatography (75 g silica gel, ethyl acetate/chloroform/hexanes 45/10/45). The product fractions containing the less polar isomer A (racemic) were combined and concentrated under vacuum, some MTBE was added, and the mixture was set aside, which caused product A to crystallize (77 mg). The resulting white material had the following properties: MP: 199-201 °C; ¹H NMR (300 MHz, CDCl₃) δ 8.75 (1H, s), 7.83 (1H, s), 7.03-6.69 (3H, m), 6.32 (1H, q, J = 7.2 Hz), 5.58 (1H, t, J = 6.0 Hz), 3.97-3.88 (1H, m), 3.73-3.65 (1H, m), 2.59-1.95 (4H, m) and 1.98 ppm (3H, d, J = 7.2 Hz).

The product fractions containing the more polar isomer B (racemic) were combined and concentrated under vacuum, some MTBE was added, and the mixture was set aside, which caused product B to crystallize (94 mg). The resulting white material had the following properties: MP: 199-201 °C; ¹H NMR (300 MHz, CDCl₃) δ 8.75 (1H, s), 7.82 (1H, s), 7.04-6.69 (3H, m), 6.31 (1H, q, J = 7.2 Hz), 5.60 (1H, t, J = 6.3 Hz), 3.97-3.88 (1H, m), 3.73-3.65 (1H, m), 2.59-1.95 (4H, m) and 1.97 ppm (3H, d, J = 7.2 Hz).

### EXAMPLE 31

### 3-(2,5-Difluorobenzyl)-6a,7,8,9-tetrahydro-3H-pyrrolo[2',1':2,3][1,3]oxazino[6,5-g][1,2,3]benzotriazine-4,11-dione

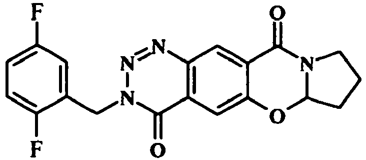

The nitro acid derivative from Example 1 (500 mg, 1.8 mmol) was dissolved in 70 ml DMF, followed by DMAP (220 mg, 1.8 mmol), HOBT (245 mg, 1.8 mmol), triethylamine (1mL), 2,5-Difluorobenzylamine (500 mg, 3.5 mmol) and EDCI (1.2 g, 6.3 mmol). The mixture was stirred at 25 °C for 19 hr. The DMF was removed under vacuum, water (100 mL) was added and the pH was adjusted to 2 using 2M sulfuric acid. The product was extracted with ethyl acetate (100 mL) and washed with sodium bicarbonate solution (100 mL). The water phases were re extracted with 100 mL ethyl acetate. The organic phases were combined, dried over sodium sulfate, and concentrated under vacuum.

The product from the previous step was dissolved in a mixture of THF (30 mL) and methanol (30 mL). The Zn/Cu reagent (10g) was prepared according to the procedure in Example 3. The fresh Zn/Cu reagent was added to the above solution and stirred at ambient temperature. Formic acid (2 ml) was added and the mixture was stirred for 15 min. The solids were filtered off and washed with a mixture of THF (50 mL) and methanol (50 mL). The filtrate was concentrated to dryness under vacuum. A mixture of DMF (25 mL) and isoamyl nitrite (5 mL) was added. After 18 hours at ambient temperature, DMF was removed under vacuum, water (100 mL) was added and the pH was adjusted to 2 using 2M sulfuric acid. The product was extracted with chloroform/THF 3/2 (250 + 175 mL), dried over sodium sulfate, and concentrated under vacuum. Crystallization from chloroform/ethyl acetate yielded 463 mg off white crystals with the following properties: MP: 235-237 °C; ¹H NMR (300 MHz, CDCl₃) δ 8.76 (1H, s), 7.84 (1H, s), 7.10-6.93 (3H, m), 5.67-5.59 (3H, m), 3.97-3.88 (1H, m), 3.74-3.65 (1H, m) and 2.59-1.94 ppm (4H, m).

### EXAMPLE 32

### 3-(1-Pyridin-3-ylethyl)-6a,7,8,9-tetrahydro-3H-pyrrolo[2',1':2,3][1,3]oxazino[6,5-g][1,2,3]benzotriazine-4,11-dione

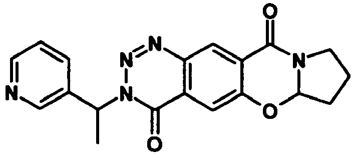

A mixture of 3-acetylpyridine (3.3 mL, 30 mmol) and ammonium formate (6.30 g, 100 mmol) was heated at 175-185°C for 2 h, then poured into 1 M sodium carbonate (50 mL) and extracted with ethyl acetate (100 mL). The extract was washed with brine (50 mL), dried over sodium sulfate, and the solvent was removed under reduced pressure. A solution of the residue in conc. HCl (6 mL) was heated at reflux for 1 h. The mixture was cooled in an ice-water bath, treated with 10 N NaOH (20 mL) and extracted with chloroform (2 x 50 mL). The extract was dried over sodium carbonate and chromatographed (chloroform/methanol/conc. ammonium hydroxide, 90 : 9 : 1) to give 3-(1-aminoethyl)pyridine (399 mg).

A suspension of the nitro acid (from Example 1, 1.02 g, 3.68 mmol) in chloroform (10 mL) was treated with thionyl chloride (1.0 mL, 13.7 mmol) and DMF (1 drop). The mixture was heated at reflux for 2 h. The volatiles were removed under reduced pressure. A solution of the residue in chloroform (20 mL) was treated with 3-(1-aminoethyl)pyridine (330 mg, 2.70 mmol) and triethylamine (0.5 mL), and stirred at room temperature for 2 h. The reaction mixture was poured into 1 M sodium bicarbonate (50 mL), stirred for 10 min and extracted with chloroform (2 x 50 mL). The extract was dried over sodium sulfate, concentrated and chromatographed (chloroform/ ethyl acetate/ ethanol/ triethylamine, 50 : 35 : 10 : 5) to give 347 mg amide.

A solution of the amide in ethanol/dichloromethane (20 + 20 mL) was hydrogenated (10% Pd/C, 100 mg) at 50 psi for 2 h. The solution was filtered through a pad of celite, and the solvent was removed under reduced pressure. The residue was dissolved in DMF (5 mL), and the solution was treated with isoamyl nitrite (1 mL) and acetic acid (0.5 mL). After 16 h at room temperature, the volatiles were removed under reduced pressure, and the residue was chromatographed (chloroform/ ethyl acetate/ ethanol/ triethylamine, 50 : 45 : 3 : 2) to give the desired product (162 mg) as a white solid (4 isomers) with MP: 195-196°C. ¹H NMR (CDCl₃) δ 1.96 (1 H, m), 2.03 (3 H, 2 doublets), 2.18 (1 H, m), 2.34 (1 H, m), 2.52 (1 H, m), 3.69 (1 H, m), 3.91 (1 H, m), 5.59 (1 H, m), 6.41 (1 H, m), 7.30 (1 H, m), 7.82 (1 H, s), 7.88 (1 H, d, J = 7.7 Hz), 8.54 (1 H, m), 8.74 (1 H, s), and 8.77 (1 H, m).

### EXAMPLE 33

### 3-(1-Pyridin-4-ylethyl)-6a,7,8,9-tetrahydro-3H-pyrrolo[2',1':2,3][1,3]oxazino[6,5-g][1,2,3]benzotriazine-4,11-dione

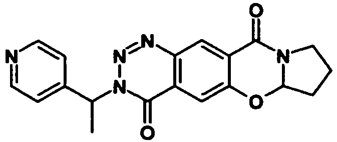

A mixture of 4-acetylpyridine (2.21 mL, 20 mmol), hydroxylamine hydrochloride (1.73 g, 25 mmol) and pyridine (25 mL) was heated at reflux for 16 h. The volatiles were removed under reduced pressure. The residue was poured into water (50 mL) and extracted with ethyl acetate (3 x 50 mL). The extract was dried over sodium sulfate, and the solvent was evaporated to give 2.37 g pure oxime.

Zinc dust (3.10 g, 47 mmol) was added in small portions to a stirred solution of the oxime (2.30 g, 18.8 mmol) in a mixture of ethanol (50 mL) and acetic acid (20 mL). After overnight stirring at room temperature, the solids were filtered off, and the solvents were removed under reduced pressure. The residue was dissolved in 10 N NaOH and extracted with THF/benzene (50 + 50 mL). The extract was dried over sodium carbonate, and the solvent was evaporated under reduced pressure to give pure 4-(1-aminoethyl)pyridine (2.00 g).

A suspension of the nitro acid (from Example 1, 1.25 g, 4.49 mmol) in chloroform (30 mL) was treated with thionyl chloride (2.0 mL, 27.4 mmol) and DMF (3 drops). The mixture was heated at reflux for 30 min. The volatiles were removed under reduced pressure. A solution of the residue in chloroform (50 mL) was treated with 4-(1-aminoethyl)pyridine (1.90 g, 17.5 mmol), and the obtained mixture was heated at 60°C for 30 min. The reaction mixture was poured into 1 M sodium bicarbonate (50 mL), stirred for 30 min and extracted with chloroform (2 x 50 mL). The extract was dried over sodium sulfate, concentrated, and chromatographed (chloroform/ ethyl acetate/ ethanol/ triethylamine, 50 : 35 : 10 : 5) to give 1.46 g amide.

A solution of the obtained amide (730 mg, 1.91 mmol) in methanol (100 mL) was hydrogenated at 50 psi for 16 h. The solution was filtered through a pad of celite, and the solvent was removed under reduced pressure. The residue was dissolved in DMF (5 mL), and the solution was treated with isoamyl nitrite (5 mL) and acetic acid (0.5 mL). After 6 h at room temperature, the volatiles were removed under reduced pressure, and the residue was chromatographed (chloroform/ ethyl acetate/ ethanol/ triethylamine, 50 : 45 : 3 : 2) to give the desired product (638 mg, 92%) as a mixture of two diastereomers. A sample of this product (312 mg) was rechromatographed using chloroform/THF (1 : 1) as an eluent. The less polar diastereomer (racemate) was recrystallized from ethanol (5 mL) to give 56 mg white crystals, MP: 197-198°C. ¹H NMR (CDCl₃) δ 1.99 (3 H, d, J = 6.9 Hz), 2.02 (1 H, m), 2.18 (1 H, m), 2.34 (1 H, m), 2.52 (1 H, m), 3.69 (1 H, ddd, J = 4.8, 7.7, and 12.5 Hz), 3.92 (1 H, dt, J = 12.1 and 7.3 Hz), 5.61 (1 H, t, J = 5.9 Hz), 6.31 (1 H, q, J = 7.3 Hz), 7.36 (2 H, m), 7.82 (1 H, s), 8.57 (2 H, m), and 8.76 (1 H, s).

The more polar diastereomer was recrystallized from ethanol (3 mL) to give 45 mg white crystals, MP: 212-213°C. ¹H NMR (CDCl₃) δ 1.99 (3 H, d, J = 6.9 Hz), 2.01 (1 H, m), 2.18 (1 H, m), 2.34 (1 H, m), 2.5 2 (1 H, m), 3.69 (1 H, ddd, J = 5.1, 8.1, and 12.1 Hz), 3.92 (1 H, dt, J = 11.7 and 7.3 Hz), 5.59 (1 H, t, J = 6.2 Hz), 6.32 (1 H, q, J = 7.3 Hz), 7.36 (2 H, d, J = 6.3 Hz), 7.83 (1 H, s), 8.57 (2 H, d, J = 6.3 Hz), and 8.76 (1 H, s).

### EXAMPLE 34

### 3-[1-(1,3-Thiazol-2-yl)ethyl]-6a,7,8,9-tetrahydro-3H-pyrrolo[2',1':2,3][1,3]oxazino[6,5-g][1,2,3]benzotriazine-4,11-dione

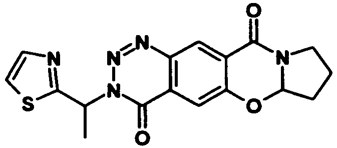

2-(1-Aminoethyl)thiazole was prepared in 90% yield by conversion of 2-acetylthiazole to its oxime and consecutive reduction with zinc dust according to the two-step procedure in Example 33.

A suspension of nitro acid (from Example 1, 1.25 g, 4.50 mmol) in chloroform (30 mL) was treated with thionyl chloride (2.0 mL, 27.4 mmol) and DMF (3 drops). The mixture was heated at reflux for 30 min. The volatiles were removed under reduced pressure. A solution of the residue in chloroform (50 mL) was treated with 2-(1-aminoethyl)thiazole (1.00 g, 7.8 mmol) and triethylamine (2.0 mL), and heated at reflux for 30 min. The reaction mixture was poured into 1 M sodium bicarbonate (50 mL), stirred for 10 min and extracted with chloroform (2 x 50 mL). The extract was dried over sodium sulfate, and the solvent was removed under reduced pressure to give 1.27 g of the amide.

A solution of the amide in conc. HCl (20 mL) was treated with tin powder (1.18 g, 10 mmol), and the mixture was heated at reflux for 15 min. The reaction mixture was poured onto crushed ice (200 g), neutralized with 10 N NaOH, treated with 1 M sodium bicarbonate (100 mL) and extracted with chloroform (3 x 100 mL). The extract was dried over sodium sulfate and concentrated under reduced pressure. The residue was dissolved in DMF (30 mL), and the solution was treated with isoamyl nitrite (6 mL, 44 mmol) and acetic acid (0.5 mL). After 16 h at room temperature, the volatiles were removed under reduced pressure, and the residue was chromatographed (chloroform/ ethyl acetate, 1 : 1). The material from fractions enriched in more polar diastereomer was additionally purified by recrystallization from ethanol (20 mL) to give the triazinone (130 mg, as a mixture of 4 isomers) as a white solid with MP: 166-168°C. ¹H NMR (CDCl₃) δ 2.03 (1 H, m), 2.13 (3 H, d, J = 7.5 Hz), 2.18 (1 H, m), 2.34 (1 H, m), 2.53 (1 H, m), 3.70 (1 H, ddd, J = 5.1, 7.8, and 12.6 Hz), 3.92 (1 H, dt, J = 12.0 and 7.5 Hz), 5.61 (1 H, t, J = 5.7 Hz), 6.72 (1 H, m), 7.33 (1 H, d, J = 3.3 Hz), 7.75 (1 H, d, J = 3.3 Hz), 7.87 (1 H, s), and 8.76 (1 H, s).

### EXAMPLE 35

### (2R)-2-(4,11-Dioxo-4,6a,7,8,9,11-hexahydro-3H-pyrrolo[2',1':2,3][1,3]oxazino[6,5-g][1,2,3]benzotriazin-3-yl)propyl thiocyanate

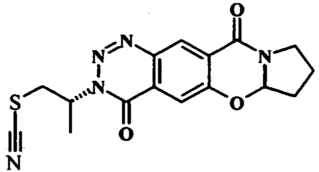

The nitro acid derivative from Example 1 (6.0 g, 21.6 mmol) was dissolved in 150 ml DMF, followed by DMAP (2.64 g, 21.6 mmol), HOBT (1.35 g, 10 mmol), triethylamine (4 mL), (R)-2-aminopropanol (2.25 g, 30 mmol) and EDCI (10 g, 52 mmol). The mixture was stirred at 45 °C for 18 hr. The DMF was removed under vacuum, water (200 mL) was added and the pH was adjusted to 2 using 2M sulfuric acid. The product was extracted with ethyl acetate/THF/EtOH 70/20/10 (200 mL) and washed with sodium bicarbonate solution (160 mL). This procedure was repeated 5 times. The organic phases were combined, dried over sodium sulfate, and concentrated under vacuum to yield an off white solid.

The product from the previous step was dissolved in a mixture of THF (100 mL) and methanol (100 mL) and DMF (50 mL). The Zn/Cu reagent (25g) was prepared according to the procedure in Example 3. The fresh Zn/Cu reagent was added to the above solution and stirred at ambient temperature. Formic acid (10 ml) was added and the mixture was stirred for 15 min. The solids were filtered off, first washed with a mixture of THF (50 mL) and methanol (50 mL). The filtrate was concentrated to ~ 50 mL under vacuum and a mixture of DMF (50 mL) and isoamyl nitrite (25 mL) was added. After 18 hr at ambient temperature, DMF was removed under vacuum, and the product was purified using flash chromatography with THF/EtOH/CHCl₃ (18/2/80 → 22/3/75) as the mobile phase. The solvent was removed under vacuum to yield 6.4g alcohol as a 1:1 mixture of isomers.

The alcohol from the previous step (1.15 g, 3.63 mmol) was dissolved in chloroform (100 mL) and THF (30 mL). triphenyl phosphine (2.6 g, 10 mmol) and NBS (1.8 g, 10 mmol) were added in portions (~20 minutes). The mixture was stirred for 60 min at ambient temperature. Then water (100 mL) was added and the the mixture was extracted with chloroform (3x 70 mL). The organic phases were combined, dried over sodium sulfate and concentrated. The product was purified using flash chromatography (100 g silica gel) and eluted with toluene/acetone (80/20). The product fractions were combined and concentrated to give 600 mg bromide.

The bromide from the previous step (600 mg, 1.58 mmol) was dissolved in DMF (60 mL) and 2.0 g NaSCN were added. The mixture was stirred for 3 days at 50°C. The solvent was evaporated, water (100 mL) was added and the the mixture was extracted with AcOEt (2x 100 mL). The organic phases were combined, dried over sodium sulfate and concentrated.

The product was purified using flash chromatography (100 g silica gel) using chloroform/THF (95/5 → 93/7). The product fractions were combined and concentrated to give 522 mg product with the following properties: MP: 183-189 °C; ¹H NMR (300 MHz, CDCl₃) δ 8.78 (1H, s), 7.87 (1H, s), 5.64-5.53 (2H, m), 3.97-3.89 (1H, m), 3.74-3.64 (2H, m), 3.47 (1H, dd, J = 4.5 and 13.8 Hz), 2.61-1.95 (4H, m) and 1.73 ppm (3H, d, J = 6.9 Hz).

### EXAMPLE 36

### 3-[2-(1H-Pyrazol-1-yl)ethyl]-6a,7,8,9-tetrahydro-3H-pyrrolo[2',1':2,3][1,3]oxazino[6,5-g][1,2,3]benzotriazine-4,11-dione

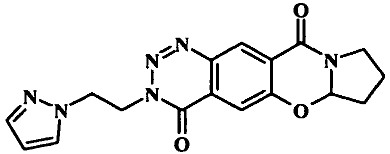

*N*-(2-Bromoethyl)phthalimide (10.64 g, 41.9 mmol) and pyrazole (8.56 g, 126 mmol) were dissolved in DMF (100 mL) and cooled to 0 °C. Sodium hydride (60 %, 3.36 g, 84 mmol) was slowly added in portions which resulted in evolution of hydrogen gas. The mixture was stirred for 15 min then allowed to reach ambient temperature over 2 hr. Hydrochloric acid (1M, 200 mL) was added and the product was extracted with chloroform (3 x 300 mL), dried over magnesium sulfate, and concentrated under vacuum. The residue was crystallized using chloroform:methyl tert-butylether to give a yellow solid (4.41 g, not the desired product). The mother liquor was concentrated under vacuum to give 4.7 g of the pyrazole.

The product from the previous step was dissolved in ethanol (100 mL). Hydrazine (3.0 mL, 96 mmol) was added and the mixture was heated to 90 °C for 3 hours. The formed precipitate was filtered, washed with methylene chloride and concentrated under vacuum. The residue was partially re dissolved in chloroform and the solids were removed by filtration. The solids were washed with chloroform and the filtrate was concentrated under vacuum to give 4.8 g of yellow oil which solidified upon standing. Three weeks later, the crystals were collected and purified further using silica gel chromatography (ethyl acetate:methanol:ammonia, 90:9:1) to obtain 0.50 g of a yellow oil.

The subsequent reactions were carried out as in Example 3 using 1.23 g (4.4 mmol) of the nitro acid and substituting the yellow oil (from the previous paragraph) for cyclobutylamine. The crude product was purified using silica gel chromatography (ethyl acetate:hexane, 3:1, followed by pure ethyl acetate). The product fractions were combined and concentrated under vacuum to give 230 mg of yellow foam. The foam was re-dissolved in methanol:chloroform and concentrated under vacuum. Ethyl acetate was added to the concentrate and the solvent was removed under vacuum. The solid residue was mixed with ethyl ether and filtered to give 94 mg of a solid material with the following properties: Too hygroscopic for determining a melting point; MS (M+H) m/z=353.2 (M+ACN) m/z=394.2; ¹H NMR (300 MHz, CDCl₃) δ 8.72 (1H, s), 7.83 (1H, s), 7.45 (1H, d, m), 7.29 (1H, m), 6.18 (1H, t), 5.61 (1H, t), 4.87 (2H, m), 4.68 (2H, t, J=6.0 Hz), 3.91 (1H, m), 3.69 (1H, m) and 2.6-1.9 (4H, m) ppm.

### EXAMPLE 37

### 3-[(2R)-1-(2H-Tetrazol-2-yl)propan-2-yl]-6a,7,8,9-tetrahydro-3H-pyrrolo[2',1':2,3] [1,3]oxazino[6,5-g][1,2,3]benzotriazine-4,11-dione and 3-[(2R)-1-(1H-tetrazol-1-yl)propan-2-yl]-6a,7,8,9-tetrahydro-3H-pyrrolo[2',1':2,3][1,3]oxazino[6,5-g][1,2,3] benzotriazine-4,11-dione

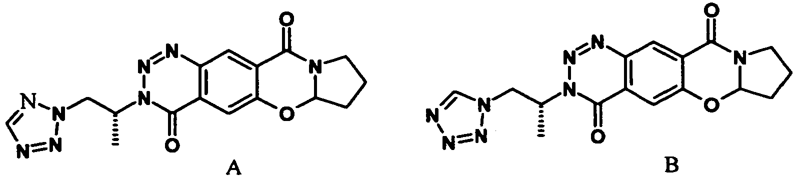

To a suspension of the alcohol from example 35 (500 mg, 1.58 mmol) in chloroform (20 mL) was added pyridine (0.808 mL, 10 mmol) and methanesulfonic anhydride (550 mg, 3.16 mmol), and the mixture was stirred under argon for 2 h. The reaction mixture was poured onto crushed ice (50 g), acidified with 1 N HCl to pH 1, and extracted with chloroform (2 x 50 mL). The extract was dried over magnesium sulfate, and the solvent was removed under reduced pressure to give the mesylate (506 mg).

A solution of the obtained mesylate (500 mg, 1.27 mmol) in DMF (10 mL) was treated with tetrazole (700 mg, 10 mmol) and 60% NaH (320 mg, 10 mmol). The obtained mixture was stirred under argon and heated at 60°C for 24 h. The volatiles were removed under reduced pressure, and the residue was chromatographed (chloroform/THF, 4 : 1) to give two regioisomeric products.

The chromatographic fractions containing the less polar regioisomer A were combined, concentrated, and the residue was rechromatographed (chloroform/THF, 9:1). The obtained product was recrystallized from ethanol (4 mL) to give the 2-tetrazole derivative A (122 mg), MP: 139-140°C, as a mixture of two diastereomers. ¹H NMR (CDCl₃) δ 1.74 (1.5 H, d, J = 6.9 Hz), 1.75 (1.5 H, d, J = 6.9 Hz), 2.03 (1 H, m), 2.18 (1 H, m), 2.35 (1 H, m), 2.52 (1 H, m), 3.70 (1H, m), 3.93 (1H, m), 5.10 (1H, m), 5.33 (1H, m), 5.60 (0.5 H, t, J = 5.7 Hz), 5.61 (0.5 H, t, J = 6.0 Hz), 5.78 (1H, m), 7.76 (0.5 H, s), 7.78 (0.5 H, s), 8.40 (0.5 H, s), 8.41 (0.5 H, s), and 8.75 (1H, s).

The more polar material recovered from chromatography was recrystallized from ethanol (2.5 mL) to give an equimolar mixture of two diastereomers of the tetrazol-1-yl isomer B (78 mg), MP: 170-175°C. ¹H NMR (CDCl₃) δ 1.70 (1.5 H, d, J = 6.6 Hz), 1.72 (1.5 H, d, J = 7.2 Hz), 2.03 (1H, m), 2.18 (1H, m), 2.35 (1H, m), 2.53 (1H, m), 3.70 (1H, m), 3.93 (1H, m), 4.88 (1H, m), 5.19 (1H, m), 5.61 (1H, t, J = 5.4 Hz), 5.77 **(**1 H, m), 7.75 (0.5 H, s), 7.77 (0.5 H, s), 8.54 (0.5 H, s), 8.56 (0.5 H, s), 8.74 (0.5 H, s), and 8.75 (0.5 H, s).

### EXAMPLE 38

### 6a,7,8,9-Tetrahydro-3H-pyrrolo[2',1':2,3][1,3]oxazino[6,5-g][1,2,3]benzotriazine-4,11-dione

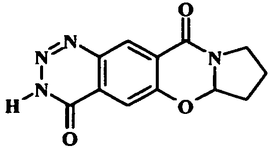

The nitro acid (3.0 g, 10.8 mmol, see example 1) was suspended in methylene chloride in the presence of thionyl chloride (6 mL) and 30 drops of DMF were added. The mixture was stirred at ambient temperature over night. The solvent was removed under vacuum (followed by high vacuum). The residue was dissolved in chloroform (50 mL). Ammonia gas (10 mL) was condensed into THF (100 mL) using a dry ice trap. The chloroform solution was slowly added and the mixture was stirred for 10 minutes. The solvent was removed under vacuum. Water (250 mL) was added and the mixture was stirred for 20 min. The nitro amide intermediate was collected on a filter, then dissolved in DMF. The DMF solution was concentrated under vacuum to remove traces of water. The residue was dissolved in DMF (200 mL) and chloroform (100 mL) was added together with palladium on carbon (10%, 1.0 g). The mixture was hydrogenated at ambient temperature for 24 hr. The solids were removed by filtration and washed with DMF. The chloroform in the filtrate was removed under vacuum. Isoamyl nitrite (6 mL) was added and the solution was left over night to complete the ring closure. The DMF was removed under vacuum and the residue was dissolved in chloroform : methanol and evaporated onto silica gel (10 g). The silica gel was transferred to a column (100g silica gel) and the product was eluted using chloroform:methanol 93:7. The product fractions were combined and concentrated to give 1.15 g of a yellow solid with the following properties: MP: 253 °C (degradation); ¹H NMR (300 MHz, CDCl₃) δ 14.5 (1H, sb), 8.69 (1H, s), 7.80 (1H, s), 5.64 (1H, t, J = 6.0 Hz), 3.95-3.86 (1H, m), 3.73-3.64 (1H, m), 2.65-1.98 ppm (4H, m).

### EXAMPLE 39

### 3-(1-Phenylpent-3-yn-2-yl)-6a,7,8,9-tetrahydro-3H-pyrrolo[2',1':2,3][1,3]oxazino[6,5-g][1,2,3]benzotriazine-4,11-dione

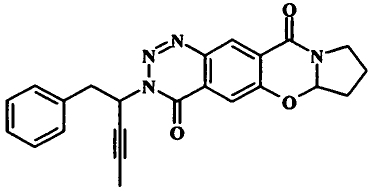

A three-neck round bottom flask was fitted with a cold trap, nitrogen inlet and an addition funnel. The flask was cooled to -78 °C using a dry ice/acetone bath, and THF (100 mL, anhydrous) was added. Propyne (11.2 g, 280 mmol) was condensed into the cooled THF. Ethylmagnesium bromide (50 mL, 150 mmol, 3.0 M in ether) was slowly added to control the internal temperature to below -60 °C. The mixture was stirred for 30 min, the cold bath was removed, and the stirring was continued for an additional hour. The reaction mixture became a light grey paste. THF (50 mL, anhydrous) was added followed by a slow addition of phenylacetaldehyde (12 mL in 20 mL THF, 100 mmol) below -30 °C. The mixture was then stirred at ambient temperature for 60 min. The reaction was quenched using cold, 2 M HCL (200 mL) followed by acidification with 10% acetic acid until pH 2 was reached. The mixture was extracted with methylene chloride (3 x 200 mL), dried over magnesium sulfate and concentrated under vacuum to obtain 16.7 g of a light yellow oil. The oil was used without further purification.

The oil from the previous step (6.2 g, ~39 mmol) was dissolved in methylene chloride (100 mL, anhydrous). Methane sulfonyl chloride (3.5 mL, 45 mmol) and DMAP (4.5 g, 37 mmol) were added followed by pyridine (4.5 mL, 37 mmol). The reaction was mildly exothermic. The mixture was stirred at ambient temperature over night, then quenched using 1 M HCl (100 mL). The product was extracted with methylene chloride (100 mL), dried over magnesium sulfate and concentrated under vacuum. The residue was further purified using silica gel chromatography (ethyl acetate:hexanes, 1:4). The product fractions were combined and concentrated under vacuum to give 5.38 g of the mesylate in the form of a yellow liquid.

The unsubstituted triazinone derivative (387 mg, 1.5 mmol, see example 38) was dissolved in DBU (457 mg, 3.0 mmol) and dimethylacetamide (10 mL). The mesylate (536 mg, 2.25 mmol) was added and the mixture was stirred over night. HCl (1M) was added and the product was extracted with ethyl acetate, dried over magnesium sulfate and concentrated under vacuum. The crude product was purified using silica gel chromatography. Of the two major fractions, the first one was the starting material and the second one was the product. The second fraction was concentrated and repurified using silica gel chromatography (ethyl acetate:hexane, 1:2). The product fractions were concentrated under vacuum and recrystallized from methylene chloride:ethyl acetate to give a light pink solid (90 mg) with the following properties: MP: 160 - 162 °C; ¹H NMR (300 MHz, CDCl₃) δ 8.77 (1H,s), 7.76 (1H, s), 7.4-7.1 (5H, m), 6.09 (1H, m), 5.59 (1H, m), 4.0-3.6 (2H, m), 3.46 (2H, m), 2.6-1.9 (4H, m) and 1.84 ppm (3H, s).

### EXAMPLE 40

### 3-Methyl-6a,7,8,9-tetrahydro-3H-pyrrolo[2',1':2,3][1,3]oxazino[6,5-g][1,2,3] benzotriazine-4,11-dione

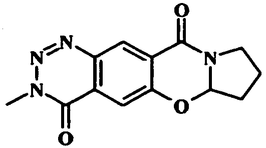

The triazinone from of example 38 (0.50 g, 1.94 mmol) was dissolved in DMF (20 mL). Silver (I) oxide (2 g, 8.6 mmol, freshly prepared) and methyl iodide (2 mL, 32 mmol) were added and the mixture was stirred for 3 days. The solids were removed by filtration, washed with DMF (20 mL) and the filtrate was concentrated under vacuum. The product was purified using flash chromatography (50 g silica gel, chloroform:THF 85:15). The product fractions were combined and slowly concentrated under vacuum (no heat) to a volume of 3 mL to start crystallization. The mother liquor was removed from the crystals and the crystals were dried under high vacuum to give 160 mg of an off white solid with the following properties: MP: 249 - 251 °C; ¹H NMR (300 MHz, CDCl₃) δ 8.74 (1H, s), 7.84 (1H, s), 5.62 (1H, m), 4.03 (3H, s), 4.0-3.6 (2H, m) and 2.6-1.9 ppm (4H, m).

### EXAMPLE 41

### 3-(3-Fluorobenzyl)-6a,7,8,9-tetrahydro-3H-pyrrolo[2',1':2,3][1,3]oxazino[6,5-g][1,2,3] benzotriazine-4,11-dione

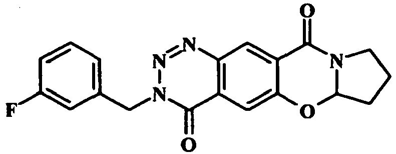

The unsubstituted triazinone (0.45 g, 1.74 mmol, see example 38) was dissolve in dimethylacetamide (25 mL). DBU (0.56 g, 3.67 mmol) and 3-fluorobenzylbromide (0.47 g, 2.5 mmol) were added and the solution was left over night. The solvent was removed under vacuum. Water (100 mL) and 2M sulfuric acid were added until pH reached 2. The product was extracted with chloroform:THF 80:20 (2 × 100 mL) and further purified using flash chromatography (100 g silica gel, ethyl acetate:hexane 500 mL 50:50 followed by ethyl acetate:chloroform:hexane 30:40:30). The product fractions were combined and the solvent removed under vacuum. The product was crystallized from chloroform:ethyl acetate to give 0.31 g of a white fluffy solid with the following properties: MP: 183-185 °C; ¹H NMR (300 MHz, CDCl₃) δ 8.75 (1H, s), 7.83 (1H, s), 7.4 - 6.9 (4H, m), 5.57 (3H, m), 4.0-3.8 (2H, m) and 2.6-1.9 ppm (4H, m).

### EXAMPLE 42

### 3-[3-(3-Fluorophenyl)propyl]-6a,7,8,9-tetrahydro-3H-pyrrolo[2',1':2,3][1,3]oxazino[6,5-g][1,2,3]benzotriazine-4,11-dione

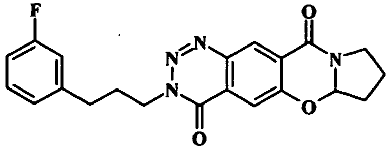

3-Fluorocinnamic acid (11.4 g, 68.6 mmol) was dissolved in THF (80 mL) and methylene chloride (80 mL). The mixture was reduced by hydrogenation at ambient temperature over night using palladium on carbon (10%, 1.1 g) as the catalyst. The catalyst was removed by filtration. The filtrate was concentrated under vacuum and re dissolved in THF (80 mL). 4-Methylmorpholine (7.43 g, 73.5 mmol) was added and the mixture was cooled to -10 °C. Ethylchloroformate (7.97 g, 73.4 mmol) was slowly added causing a white precipitate to form. The mixture was stirred for 25 min at -10 °C, then filtered slowly through a glass frit into a water/ice solution (150 mL) of sodium borohydride (5.64 g, 149 mmol). As the reaction mixture warmed up, gas bubbles formed. After 10 min, the filter cake was transferred to the solution with the help of THF:water, (100 mL, 1:1). The mixture was stirred for 10 min, methanol (100 mL) was added and the pH was slowly adjusted to 2 using hydrochloric acid. THF and methanol were removed under vacuum and the remaining aqueous phase was extracted with ethyl acetate (2 × 200 mL). The organic phase was concentrated under vacuum to give a colorless oil (11.4 g, 74 mmol). The colorless oil was dissolved in methylene chloride (200 mL). Triphenylphosphine (37 g, 151 mmol) and NBS (26 g, 146 mmol) were added portion wise. The mixture was then allowed to evaporate onto silica gel but it did not dry to a powder. Methyl tert-butylether (150 mL) was added and the mixture was stirred for 15 min then filtered through a 2 cm plug of silica gel. The plug was washed with methyl tert-butylether (200 mL) and allowed to dry. The product was purified using flash chromatography (hexane:methyl tert-butylether, 90:10). The product fractions were combined and concentrated under vacuum to give a colorless oil (9.3 g, 43 mmol). Distillation using a Kugelrohr apparatus gave a colorless liquid (8.4 g, 39 mmol).

In the final reaction the procedure in Example 41 was followed using 0.4 g (1.55 mmol) of triazinone and substituting the colorless oil described above for 3-fluorobenzylbromide. The product was crystallized from chloroform:methyl tert-butylether to give 305 mg of a white powder with the following properties: MP: 145-148 °C; ¹H NMR (300 MHz, CDCl₃) δ 8.74 (1H, s), 7.84 (1H, s), 7.4 - 6.8 (4H, m), 5.61 (1H, t, J=6 Hz), 4.48 (2H, t, J=7 Hz), 4.1-3.6 (2H, m), 2.76 and (2H, m) 2.6-1.9 ppm (6H, m).

### EXAMPLE 43

### 3-(1,3-Benzothiazol-2-ylmethyl)-6a,7,8,9-tetrahydro-3H-pyrrolo[2',1':2,3][1,3]oxazino [6,5-g][1,2,3]benzotriazine-4,11-dione

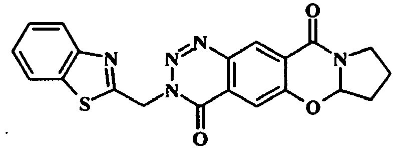

The procedure in Example 41 was followed using 0.44 g (1.70 mmol) of triazinone and substituting 570 mg (2.5 mmol) 2-(bromomethyl)-1,3-benzothiazole for 3-fluorobenzylbromide. The product was crystallized from methanol. The mother liquor was removed from the crystals which were subsequently dried under high vacuum to give 0.58 g of a beige solid with the following properties: MP: 224-227 °C; ¹H NMR (300 MHz, DMSO) δ 8.55 (1H, d, J = 1.5 Hz), 8.08 (1H, d, J = 7.8 Hz), 7.95 (1H, d, J = 7.5 Hz), 7.77 (1H, d, J= 1.5 Hz), 7.53-7.41 (2H, m), 6.03 (2H, s), 5.80 (1H, t, J = 5.4 Hz), 3.81-3.52 (2H, m) and 2.56-1.89 (4H, m) ppm.

### EXAMPLE 44

### 3-(2,1,3-Benzoxadiazol-5-ylmethyl)-6a,7,8,9-tetrahydro-3H-pyrrolo[2',1':2,3][1,3] oxazino[6,5-g][1,2,3]benzotriazine-4,11-dione

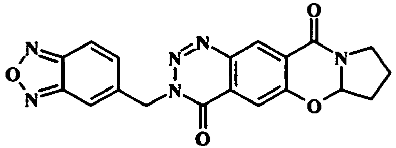

The procedure in Example 41 was followed using 0.44 g (1.70 mmol) of triazinone and substituting 533 mg (2.5 mmol) 5-(bromomethyl)-2,1,3-benzoxadiazole for 3-fluorobenzylbromide. The product was purified by crystallization from methanol. The mother liquor was removed from the crystals, which were subsequently dried under high vacuum to give 618 mg of a beige powder with the following properties: MP: 250-262 °C (decomposition); ¹H NMR (300 MHz, DMSO) δ 8.51 (1H, d, J = 1.5 Hz), 8.06 (1H, d, J = 9 Hz), 7.98 (1H, s), 7.75 (1H, d, J = 1.5 Hz), 7.64 (1H, d, J = 9 Hz), 5.79 (1H, t, J = 5.8 Hz), 5.70 (2H, s), 3.80-3.52 (2H, m) and 2.57-1.89 (4H, m) ppm.

### EXAMPLE 45

### 3-[(4,11-Dioxo-4,6a,7,8,9,11-hexahydro-3H-pyrrolo[2',1':2,3][1,3]oxazino[6,5-g][1,2,3] benzotriazin-3-yl)methyl]benzonitrile

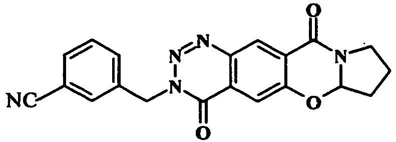

The procedure in Example 41 was followed using 150 mg (0.58 mmol) of triazinone and substituting 330 mg 3-cyano-benzylbromide for 3-fluorobenzylbromide. The product was purified using flash chromatography (50 g silica gel, ethyl acetate:chloroform 40:60). The product fractions were combined and the solvent removed under vacuum. The product was crystallized from chloroform:ethyl acetate to give 100 mg of a white solid with the following properties: MP: 214-217 °C; ¹H NMR (300 MHz, CDCl₃) δ 8.77 (1H, s), 7.83 (1H, s), 7.80-7.44 (4H, m), 5.67-5.55 (3H, m), 3.97-3.88 (1H, m), 3.74-3.66 (1H, m) and 2.60-1.95 ppm (4H, m).

### EXAMPLE 46

### 2-[(4,11-Dioxo-4,6a,7,8,9,11-hexahydro-3H-pyrrolo[2',1':2,3][1,3]oxazino[6.5-g][1,2,3] benzotriazin-3-yl)methyl]benzonitrile

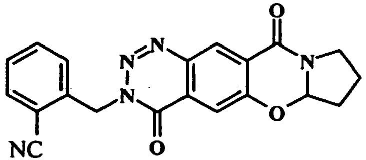

The procedure in Example 41 was followed using 150 mg (0.58 mmol) of triazinone and substituting 330 mg 2-cyano-benzylbromide for 3-fluorobenzylbromide. The product was purified using flash chromatography (50 g silica gel, ethyl acetate:chloroform 35:65). The product fractions were combined and the solvent removed under vacuum. The product was crystallized from chloroform:ethyl acetate to give 86 mg of a off-white solid with the following properties: MP: 257-260 °C (degradation); ¹H NMR (300 MHz, CDCl₃) δ 8.78 (1H, s), 7.85 (1H, s), 7.73 -7.39 (4H, m), 5.83 (2H, s), 5.61 (1H, t, J = 6.0Hz), 3.97-3.88 (1H, m), 3.74-3.66 (1H, m) and 2.60-1.95 ppm (4H, m).

### EXAMPLE 47

### 3-(Pyridin-3-ylmethyl)-6a,7,8,9-tetrahydro-3H-pyrrolo[2',1':2,3][1,3]oxazino[6,5-g][1,2,3]benzotriazine-4,11-dione

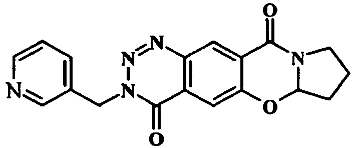

The procedure in Example 41 was followed using 150 mg (0.58 mmol) of triazinone and substituting 360 mg 3-bromomethyl-pyridine * HBr for 3-fluorobenzylbromide. The crude product was purified using flash chromatography (50 g silica gel, ethyl acetate:methanol 95/5 → 90/10). The product fractions were combined and the solvent removed under vacuum. The product was crystallized from methanol:ethyl acetate to give 96 mg of a white solid with the following properties: MP: 203-205 °C; ¹H NMR (300 MHz, CDCl₃) δ 8.80 (1H, s) 8.75 (1H, s), 8.56 (1H, d, J = 4.2 Hz), 7.85 (1H, d, J = 8.1 Hz), 7.83 (1H, s), 7.28 (1H, dd, J = 4.2 and 8.1 Hz), 5.65-5.55 (3H, m), 3.96-3.87 (1H, m), 3.73-3.65 (1H, m) and 2.60-1.95 ppm (4H, m).

### EXAMPLE 48

### 3-(Pyridin-2-ylmethyl)-6a,7,8,9-tetrahydro-3H-pyrrolo[2',1':2,3][1,3]oxazino[6,5-g][1,2,3]benzotriazine-4,11-dione

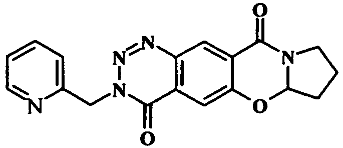

The procedure in Example 41 was followed using 160 mg (0.62 mmol) of triazinone and substituting 380 mg 2-bromomethyl-pyridine * HBr for 3-fluorobenzylbromide. The crude product was purified using flash chromatography (50 g silica gel, ethyl acetate:methanol 95/5). The product fractions were combined and the solvent removed under vacuum. The product was crystallized from methanol:ethyl acetate to give 77 mg of a white solid with the following properties: MP: 184-186 °C; ¹H NMR (300 MHz, CDCl₃) δ 8.79 (1H, s) 8.54 (1H, d, J =3.9 Hz), 7.85 (1H, s), 7.69-7.64 (1H, m), 7.32 (1H, d, J = 7.8 Hz), 7.22-7.18 (1H, m), 5.75 (2H, s), 5.61 (1H, t, J = 6.0 Hz), 3.97-3.88 (1H, m), 3.74-3.66 (1H, m) and 2.60-1.95 ppm (4H, m).

### EXAMPLE 49

### 3-(Pyrazin-2-ylmethyl)-6a,7,8,9-tetrahydro-3H-pyrrolo[2',1':2,3][1,3]oxazino[6,5-g][1,2,3]benzotriazine-4,11-dione

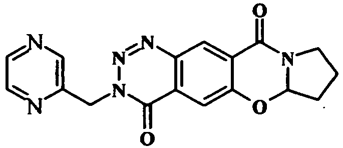

A solution of 2.0 g methylpyrazine and 1.8g trichloro isocyanuric acid in 30 ml chloroform was refluxed for 18 hours. The resulting solids were filtered off and the solvent evaporated. The residue was dissolved in 7 ml DMF, 160 mg (0.62 mmol) of triazinone (see example 38) and 1 ml DBU were added and stirred for 18 hours. The crude product was purified after evaporation of the solvent, using flash chromatography (50 g silica gel, ethyl acetate :chloroform 85/15). The product fractions were combined and the solvent removed under vacuum. The product was crystallized from chloroform:ethyl acetate to give 130 mg of a white solid with the following properties: MP: 229-231°C; ¹H NMR (300 MHz, CDCl₃) δ 8.79 (1H, s) 8.71 (1H, s), 8.51 (2H, s), 7.85 (1H, s), 5.78 (2H, s), 5.61 (1H, t, J = 6.0 Hz), 3.97-3.88 (1H, m), 3.74-3.66 (1H, m) and 2.60-1.95 ppm (4H, m).

### EXAMPLE 50

### 3-[(4-Bromo-1H-pyrazol-1-yl)methyl]-6a,7,8,9-tetrahydro-3H-pyrrolo[2',1':2,3][1,3] oxazino[6,5-g][1,2,3]benzotriazine-4,11-dione

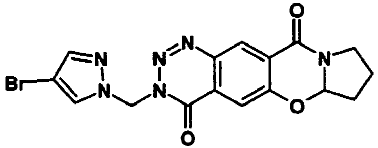

A solution of 3-bromopyrazole (1.00 g, 6.8 mmol) in ethyl ether (20 mL) was treated with 37% formaldehyde (0.3 mL, 4.0 mmol). The obtained solution was set aside at room temperature overnight. The volatiles were evaporated under reduced pressure. Toluene (5 mL) was added, and the evaporation was repeated. The obtained oil was triturated with ethyl ether (3 mL) to crystallize. The crystals were separated by decantation and dried to give 3-bromo-1-(hydroxymethyl)pyrazole (371 mg, 37%). A suspension of the obtained product in chloroform (5 mL) was treated with thionyl chloride (0.5 mL) and heated at reflux for 1 h. The volatiles were removed under reduced pressure to provide crude 3-bromo-1-(chloromethyl)pyrazole.

A suspension of the unsubstituted triazinone from Example 38 (100 mg, 0.41 mmol) in DMF (3 mL) was treated with 60% NaH (18 mg, 0.45 mmol) followed by 3-bromo-1-(chloromethyl)pyrazole (82 mg, 0.49 mmol). After stirring for 1 h at room temperature, the solvent was evaporated under reduced pressure, and the residue was chromatographed (chloroformlethyl acetate, 1:1). The obtained material was recrystallized from ethanol (5 mL) to give the desired product, 70 mg, MP: 180-181°C. ¹H NMR (CDCl₃) δ 2.03 (1 H, m), 2.19 (1 H, m), 2.35 (1 H, m), 2.53 (1 H, m), 3.70 (1 H, m), 3.91 (1 H, dt, J = 12.3 and 7.2 Hz), 5.61 (1 H, t, J = 6.0 Hz), 6.55 ( 1 H, d, J = 13.8 Hz), 6.60 (1 H, d, J = 13.5 Hz), 7.51 (1 H, s), 7.85 (1 H, s), 7.87 (1 H, s), and 8.77 (1 H, s).

### EXAMPLE 51

### 3-[2-(3-Fluorophenyl)ethyl]-8,9-dihydro-3H-[1,3]oxazolo[2',3':2,3][1,3]oxazino[6,5-g][1,2,3]benzotriazine-4,11-dione

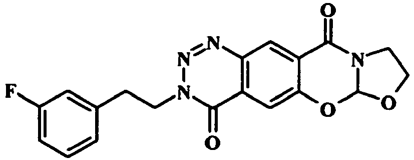

4-Methylsalicylic acid (17 g, 112 mmol) was suspended in methylene chloride (120 mL) and acetic anhydride (35 mL) was added together with acetic acid (7 mL).The mixture was cooled using an ice bath. Nitric acid (7mL, 90%) was added over 1 minute, the mixture was stirred for 10 min and nitric acid (4 mL) was added slowly until the reaction mixture was clear. The stirring was continued for 60 min at ambient temperature during which a precipitate formed. Hexane (120 mL) was added and the stirring was continued for 10 min. The solids were collected by filtration and washed with a small amount of hexane. The remaining solvent was removed under high vacuum to give 7.5 g of a white solid. This reaction was repeated to give more starting material for further synthesis.

The 5-nitro-4-methylsalicylic acid (9.7 g, 49 mmol) was dissolved in ethanol (150 mL) and thionyl chloride (9 mL) was added. The mixture was heated to reflux for 24 hr. The solvent was removed under vacuum and the residue was dissolved in a solution of sodium bicarbonate (200 mL) in ethyl acetate (200 mL). The phases were separated and the aqueous phase was extracted with ethyl acetate (200 mL). The organic phases were combined, dried over sodium sulfate and concentrated under vacuum to give 10.7 g (48 mmol) of solid ethyl ester intermediate. The solid was dissolved in a mixture of dioxane (100 mL) and toluene (200 mL) and the volume was reduced under vacuum to about 80 mL. DMF (20 mL) and toluene (150 mL) were added together with potassium carbonate (20 g) and 4-methoxybenzyl chloride (9.0 g, 57 mmol). Sodium iodide (1.0 g) was added and the mixture was stirred for three days. The solids were removed by filtration and washed with ethyl acetate (2 × 100 mL). The filtrate was concentrated under vacuum, dissolved in a small amount of chloroform, then purified using flash chromatography (170 g silica gel, hexane:chloroform:ethyl acetate, 60:35:5). Clean product fractions and mixed fractions eluted. The product fractions were combined and concentrated under vacuum. Hexane was added to dissolve residue and crystals formed as the solution was slowly concentrated. The crystals were collected by filtration to give 12.4 g of the 4-methoxybenzyl ether derivative. Another 0.94 g was recovered from the mother liquor and the mixed fractions by performing further purification using flash chromatography. The product from the previous step (13.3 g, 38.6 mmol) and N,N-dimethylformamide dimethyl acetal (25 g) were added to DMF (10 mL) and kept at 125 °C for 18 hr. The solvent was removed under vacuum and the residue was dissolved in THF (120 mL). A solution of sodium periodate (30 g, 140 mmol) in water (250 mL) was added and the mixture was stirred for 30 min. Ethyl acetate (100 mL) was added, the solids were removed by filtration. The phases were separated and the aqueous phase was extracted with ethyl acetate (200 mL). The organic phases were combined, dried over sodium sulfate and concentrated to give a brown, sticky solid. Methanol (100 mL) was added and the insoluble material was collected by filtration (2.45 g). The filtrate was concentrated to give a sticky, brown oil (12.9 g). It was dissolved in a small amount of chloroform and evaporated onto silica gel (15 g). The silica gel was transferred to a column (100 g silica gel) and purified using 500 mL hexane:chloroform:ethyl acetate, 60:35:5, then 40:55:5. The product fractions were concentrated under vacuum and crystals started to form when a small volume remained. Methanol (100 mL) was added and the solids were collected by filtration. The solids were dried under high vacuum to give 3.4 g of an off white solid. An additional 4.45g were recovered from the motherliquor. A total of 7.95 g of nitro aldehyde intermediate was thus collected.

The nitro aldehyde derivative (3.4 g, 9.5 mmol) from the previous step was dissolved in dry DMF (50 mL) and oxone (6.76 g, 11 mmol) was added. The mixture was stirred for 18 hours and chloroform (150 mL) was added. The solids were removed by filtration and the filtrate was concentrated by vacuum to a remaining volume of about 30 mL. To this DMF solution was added DMAP (1.16 g, 9.5 mmol), HOBT (1.28 g, 9.5 mmol), triethylamine (3 mL, 22 mmol), 3-fluorophenethylamine (1.7 g, 12.2 mmol) and EDCI (5 g, 26 mmol). The mixture was stirred at ambient temperature over night. The solvent was removed under vacuum and water (300 mL) was added together with 2M sulfuric acid (enough to bring pH to 2). The solution was extracted with ethyl acetate (2 × 250 mL) and the organic phase was washed with sodium bicarbonate solution (100 mL). The organic phase was dried over sodium sulfate, and concentrated to about 12 mL. A little chloroform was added (to keep the product in solution) and the product was purified using flash chromatography (100 g silica gel, ethyl acetate:hexane, 500 mL 40:60, 250 mL 50:50, 250 mL 60:40). The product fractions were combined and concentrated under vacuum to start crystallization of the amide derivative as a white solid (3.45 g).

The reduction of the nitro group to the amine, using 10g Zn/Cu and the following ring closure using isoamylnitrite was carried out as in Example 3 using 1.13 g (2.27 mmol) of the product from the previous step. The reaction gave 0.93 g of a yellowish solid.

The triazinone from the previous step (0.93 g, 1.95 mmol) was dissolved in methylene chloride (50 mL) and trifluoroacetic acid (1.5 mL, 19 mmol) was added. The mixture was stirred for 60 min at ambient temperature. The solvent was removed under vacuum, chloroform (20 mL) was added and the solvent was again evaporated. The residue was dissolved in THF (10 mL) and methanol (15 mL) and a solution of potassium hydroxide (3.0 g, 53 mmol, 15 mL) was added. The mixture was left at ambient temperature for 90 min. The organic solvents were removed under vacuum and the aqueous residue was washed with methyl t-butylether (30 mL). Hydrochloric acid was added until pH reached 3 and the product started precipitating. The product was extracted with chloroform:THF, 3:1 (2 x 40 mL) and concentrated under vacuum (followed by high vacuum) to a constant weight to give 624 mg of a yellow solid. This step in the synthesis was repeated to give more starting material for the next step.

The salicylic acid from the previous step (0.781 g, 2.37 mmol) was dissolved in chloroform (30 mL) and ethyl acetate (20 mL). CDI (2.0 g, 12.3 mmol) was added followed by DMF (about 10 mL) until a clear solution was obtained. The solution was stirred for 2 hr at ambient temperature after which ethanolamine (5 mL in 20 mL THF) was added. The mixture was stirred for three days. The solvent was removed under vacuum and 2N hydrochloric acid (40 mL) was added. The product was extracted with chloroform:methanol, 5:1 (2 × 75 mL) and the solvent was removed under vacuum (followed by high vacuum) to give 1.09 g (<2.9 mmol) of crude amide derivative.

The crude product from the previous step (1.09 g) was dissolved in a mixture of toluene (100 mL) and dioxane (30 mL). The solution was placed in a distillation apparatus. Toluene sulfonic acid (50 mg) was added as a catalyst and the solution was heated until distillation started. Trimethylorthoformate (4 mL in 6 mL toluene) was added over 10 min. A total of about 90 mL had distilled off at the completion of the reaction. The solution was cooled to ambient temperature and ethyl acetate (100 mL) was added. The reaction mixture was washed with sodium bicarbonate solution (50 mL), dried over sodium sulfate and concentrated to the point when crystals started forming. After crystallization was complete, the crystals were collected and dissolved in chloroform (12 mL) and filtered through 2 mm of silica gel. The silica was washed with a small amount of chloroform, then ethyl acetate was added to the filtrate and the solution was slowly concentrated until crystals started to form. The supernatant was removed and the crystals were dried under high vacuum to give 170 mg of product. It was combined with product from repeated syntheses, purified further using flash chromatography (50 g silica gel, chloroform:THF 96:4) and crystallized from chloroform:methyl t-butylether to give a white solid with the following properties: MP: 191-193 °C; ¹H NMR (300 MHz, CDCl₃) δ 8.74 (1H, s), 7.95 (1H, s), 7.29 - 6.89 (4H, m), 6.35 (1H, s), 4.71-4.66 (2H, m), 4.42-4.27 (3H, m), 3.73-3.63 (1H, m) and 3.25-3.20 ppm (2H, m).

### EXAMPLE 52

### 8-Ethyl-3-[2-(3-fluoroubenyl)ethyl]-7,8-dihydro-3H-[1,3]ozazino[6,5-g][1,2,3] benzotriazine-4,9-dione

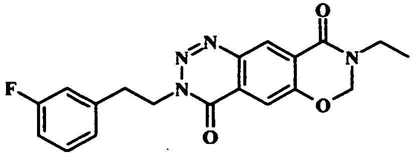

4-Methyl salicylic acid (25 g, 164 mmol) was dissolved in chloroform (250 mL) and CDI (25 g, 154 mmol) was added in portions. The mixture was stirred for 90 min at ambient temperature. Ethylamine hydrochloride (50 g, 613 mmol) and sodium hydroxide beads (75 g, 1.87 mol) were mixed and heated with a heat gun and a dry ice trap was used to condense the resulting ethylamine into the stirred chloroform solution. The mixture was left stirring over night. The next day, water (200 mL) was added, the pH was adjusted to 1 using 2M sulfuric acid and the phases were separated. The aqueous phase was extracted with chloroform (200 mL) and the organic phases were combined and washed with sodium bicarbonate solution (150 mL), dried over sodium sulfate and concentrated to give 24.6 g of crude intermediate. The intermediate was dissolved in chloroform (250 mL), then trioxane (50 g, 0.5 mol), sodium sulfate (50 g) and concentrated sulfuric acid (50 drops) were added. The mixture was heated to reflux for 1 hour. Another 50 drops of sulfuric acid was added and the heating was continued for 1 hour. This step as repeated three times after which trioxane (25 g) was added and the heating was continued for 18 hr. The reaction mixture was filtered through silica gel (2 cm layer), the silica was washed with ethyl acetate (250 mL) and the filtrates were combined and allowed to evaporate onto silica gel (30 g). The product was purified using flash chromatography (155 g silica gel) and eluting with ethyl acetate/hexane, 2:8, 1 L, followed by ethyl acetate/hexane, 3:7, 500 mL, and finally ethyl acetate/hexane, 1:1. The product fractions were combined and concentrated to give 12.6 g of a colorless oil.

The oil from the previous step (12.6 g, 66 mmol) was dissolved in chloroform (25 mL) containing acetic acid (10 mL) and acetic anhydride (25 mL). Concentrated sulfuric acid (10 mL) was added slowly, which caused warming of the mixture. An ice bath was therefore installed to keep the mixture cool. Nitric acid (7 mL, 90%) was added slowly and the mixture was allowed to warm to ambient temperature within 2 hr. TLC shows some dinitration occurred. The mixture was poured into water (250 mL), the product was extracted with chloroform (2 x 200 mL), concentrated at high vacuum to remove acetic acid, and purified using flash chromatography (170 g silica gel, toluene:acetone, 95:5) to give 3.56 g of mono nitrated product, which crystallized from toluene. Another 1.6 g crystallized from the mother liquor (total yield of clean 5-nitro derivative: 22%).

The 5-nitroderivative from the previous step (2.7 g, 11.4 mmol) was dissolved in N,N-dimethylformamide dimethyl acetal (15 mL) and heated to 120 °C for 24 hr. The solvent was removed under vacuum and THF (25 mL) was added. Periodate (6.5 g, 30.4 mmol) was dissolved in water (50 mL) and added to the solution which was stirred for 45 min at ambient temperature. Potassium permanganate (8 g, 50.6 mmol) was dissolved in water (200 mL) and added slowly to the reaction mixture which was stirred for another 60 min to yield a dark brown slurry. Chloroform (200 mL) and concentrated hydrochloric acid (12 mL) was added followed by sodium metabisulfite (15 g) in portions until the solution faded to yellow and became clear. The phases were separated and the aqueous phase was extracted with chloroform (200 mL). The organic phases were combined, dried over sodium sulfate and concentrated to give 3.8 g of the crude nitro acid derivative as a dark oil.

The nitro acid derivative (1.9 g, 7 mmol) was dissolved in 20 mL THF and 20 mL dichloromethane, DMAP (1.1 g, 9 mmol), HOBT (1.22 g, 9 mmol), triethylamine (1.5 mL), and EDCI (3.8 g, 19.8 mmol) were added. The mixture was stirred at 45 °C for 30 minutes, 2 ml (14 mmol) 3-fluorophenethylamine was added and the mixture was kept at 45 °C for 18 hours. The DMF was removed under vacuum, water (200 mL) was added and the pH was adjusted to 2 using 2M sulfuric acid. The product was extracted with chloroform (2x200 mL) and washed with sodium bicarbonate solution (150 mL). The organic phases were combined, dried over sodium sulfate, and evaporatet onto 10 g silica gel. The product was further purified using flash chromatography with ethyl acetate/hexane (60/40 → 100/0) as the mobile phase. The solvent was removed under vacuum to give 1.26 g of a beige solid.

The phenethylamide derivative (1.26 g, 3.25 mmol) was dissolved in chloroform (100 mL) and 10% palladium on carbon (900 mg) was added. The mixture was hydrogenated at ambient temperature for 2 hr. The solids were filtered off, washed with methanol (10 mL) and the filtrate was concentrated to give a yellow solid (714 mg).

The solid from the previous step (714 mg, 2 mmol) was dissolved in DMF (20 mL). Isoamyl nitrite (270 mg, 2.3 mmol, dissolved in 1 mL THF) was added followed by acetic acid (3 drops). The mixture was stirred overnight at ambient temperature. After evaporation of the DMF (vacuum), the residue was dissolved in ethyl acetate/THF and evaporated onto silica gel (10 g). The gel was transferred to the top of a silica gel column (90 g silica gel) and eluted with 500 mL ethyl acetate:hexane, 1:1, followed by 250 mL 7:3, then pure ethyl acetate. The product fractions were concentrated to yield 340 mg of crystals with the following properties: MP: 161-163 °C; ¹H NMR (300 MHz, CDCl₃) δ 8.78 (1H, s), 7.84 (1H, s), 7.30-6.85 (4H, m), 5.34 (2H, s), 4.67 (2H, m), 3.67 (2H, q, J=7.2 Hz), 3.22 (2H, m) and 1.30 ppm (3H, t, J=7.2 Hz).

### EXAMPLE 53

### 8-tert-Butyl-3-[(2R)-1-(2H-tetrazol-2-yl)propan-2-yl]-7,8-dihydro-3H-[1,3]oxazino[6,5-g][1,2,3]benzotriazine-4,9-dione

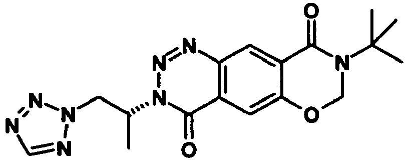

To a solution of R-2-Aminopropanol (10.05 g, 134 mmol) in 200 ml of toluene was added phthalic anhydride (19.83 g, 134 mmol) and triethylamine (1.5 ml). The reaction mixture was heated under reflux in a flask fitted with a Dean-Stark apparatus for 3 h. During this period, the temperature of the oil bath was maintained at about 130°C and water was separated. All volatiles were then evaporated under vaccum and the solid residue was taken up in EtOAc (150 ml) and washed with 2N HCl (2x 100 ml), saturated NaHCO₃ (2x100 ml) and with water (2x100 ml). The organic phase was dried (Na₂SO₄) and concentrated under vaccum to afford 22.0 g of a white solid, ¹H NMR (300 MHz, CDCl₃) δ 7.85 (2H, m), 7.72 (2H, m), 4.54 (1H, m), 4.02 (1H, m), 3.92 (1H, m), 2.64 (1H, brs) and 1.45 (3H, d).

To a solution of the alcohol (3.5 g, 17.0 mmol), tetrazole (1.8 g, 1.5 eq), Diphenyl-2-pyridylphosphine (6.7 g, 1.5 eq) in dry THF was added DIAD (5 ml) dropwise under N₂. The mixture was stirred at room temperature overnight (18 hours). The solvent was evaporated and the residue dissolved in chloroform (200 ml), washed with 6N HCl (300 ml), dried (Na₂SO₄) and concentrated which yielded a yellow oil. The oil was purified using flash chromatography (100 g silica gel, chloroform/ THF 100/0 → 80/20). Evaporation of the solvent yielded 2.7 g of the less polar 2-tetrazolyl isomer in semi-solid form. ¹H NMR (300 MHz, CDCl₃) δ 8.40 (1H, s), 7.80 (2H, m), 7.70 (2H, m), 5.40 (1H, m), 4.95 (2H, m) and 1.65 (3H, d, J = 6.9 Hz).

Evaporation of the solvent yielded 1.9 g of the more polar 1-tetrazolyl isomer in semisolid form. ¹H NMR (300 MHz, CDCl₃) δ 8.60 (1H, s), 7.80 (2H, m), 7.70 (2H, m), 5.25 (1H, dd, J = 10.2 and 14.1 Hz), 4.92 (1H, m), 4.73 (1H, dd, J = 4.8 and 14.1 Hz) and 1.62 (3H, d, J = 6.9 Hz).

The less polar 2-tetrazolyl isomer (2.7 g, 10.5 mmol) was dissolved in 50 ml ethanol. Hydrazine hydrate (2 ml) was added, and the mixture was refluxed at 90°C for 4 h. A white gelatinous precipitate was formed. The solids were filtered, washed with CH₂Cl₂ (150 ml) evaporated. Redissolve the residue in CH₂Cl₂ (150 ml) and filter to obtain semisolid (2R)-1-(2H-tetrazol-2-yl)-2-aminopropane (1.3 g), ¹H NMR (300 MHz, CDCl₃) δ 8.53 (1H, s), 6.40 (1H, brs), 4.60 (1 H, dd, J = 4.8 and 13.2 Hz), 4.52 (1H, dd, J = 7.5 and 13.2 Hz), 3.59 (1H, m) and 1.26 (3H, d, J = 6.9 Hz).

The more polar 1-tetrazolyl isomer (1.9 g, 7.39 mmol) was dissolved in 50 ml ethanol. Hydrazine hydrate (2 ml) was added, and the mixture was refluxed at 90°C for 4 h. A white gelatinous precipitate was formed. The solids were filtered, washed with CH₂Cl₂ (150 ml) and the solvent evaporated. The residue was redissolved in CH₂Cl₂ (150 ml), filtered and the solvent evaporated to obtain semisolid (2R)-1-(1H-tetrazol-1-yl)-2-aminopropane (1.0 g), ¹H NMR (300 MHz, CDCl₃) δ 8.78 (1H, s), 6.50 (1H, brs), 4.44 (1H, dd, J = 9.9 and 3.9 Hz), 4.22 (1H, dd, J = 7.5 and 4.0 Hz), 3.45 (1H, m) and 1.27 (3H, d, J = 6.9 Hz).

4-Methylsalicylic acid (42.6 g, 280 mmol) was dissolved in methylene chloride (250 mL) and CDI (45.4 g, 280 mmol) was added portion wise causing release of CO₂. The mixture was stirred at ambient temperature for 24 hr. A solution of t-butylamine (20.7 g, 280 mmol) in triethylamine (10 mL, 72 mmol) was added to the mixture, which was stirred for 24 h. The reaction mixture was acidified with 6N hydrochloric acid (150 ml). The phases were separated and the aqueous phase was extracted with chloroform (200 mL). The combined organic phases were washed with sodium bicarbonate solution (200 mL) and dried over sodium sulfate. Concentration and silica gel chromatography gave 15.2 g of amide as an off white solid.

The amide (15.2 g, 73.4 mmol) and trioxane (22.3 g, 0.24 mol) were dissolved in chloroform (150 mL), which was stirred at ambient temperature. Sodium sulfate (20 g) and concentrated sulfuric acid (50 drops) were added and the mixture was refluxed for 30 min, after which conc. sulfuric acid (25-30 drops) and 10 g of trioxane was added and refluxed overnight, the solids were removed by filtration and washed with ethyl acetate. The combined solvents were removed under vacuum to give 19 g of oil. The oil was purified using flash chromatography (150 g silica gel, ethyl acetate:hexane 30:70, then 40:60) to give 6.0 g of benzoxazinone as a white solid.

The benzoxazinone 4 g (18.3 mmol) was dissolved in CH₂Cl₂ (80 ml), acetic anhydride (8 ml) and cooled to 0°C in an ice bath. Nitric acid (6 ml, 90 %) was added slowly, which produced an orange solution. The reaction was complete in 90 min as confirmed by TLC. The reaction mixture was poured over crushed ice (300 g), extracted with chloroform (200 ml), washed with saturated sodium bicarbonate (200 ml), dried over Na₂SO₄ and concentration under vaccum afforded the mono nitro compound 3.5 g (single isomer) as an off white solid.

3.5 g (13.25 mmol) of the solid from the previous step was dissolved in dimethylformamide dimethyl acetal (25 ml) and heated to 125°C. for overnight. The orange solution was removed under vaccum and THF (40 ml) was added. Periodate (7.83 g, 36.8 mmol) was dissolved in 80 ml of water and added to the solution which was stirred for 30 minutes at room temperature. It was extracted with CHCl₃ (300 ml), dried over Na₂SO₄ and concentrated under vaccum to obtain a semi solid. Crystallization from MTBE afforded 2.5 g of pure 5-nitro aldehyde. The aldehyde (2.5 g, 8.86 mmol) was treated with oxone (6.52 g, 1.2 eq.) in 50 ml of DMF overnight. 50 ml of CHCl₃ was added and the solids were filtered off. The solvent was evaporated and the residue was poured into water. The formed solids were filtered and washed with water and dried (vaccum) to obtain 2.68 g of the off white nitro acid.

588 mg (2 mmol) of the nitro acid from the previous reaction were dissolved in Chloroform/DMF (20 mL and 1 mL), HOBT (270 mg, 2.0 mmol), DMAP (244 mg, 2.0 mmol), 1 ml of TEA, (2R)-1-(2H-tetrazol-2-yl)-2-aminopropane (380 mg 3.0 mmol) and EDCI (1.14 g, 3.0 eq.) were added and the mixture was stirred at room temperature overnight. The mixture was diluted with CHCl₃ (50 ml), washed with 2N HCl (100 ml) and dried over Na₂SO₄. Concentration under vaccum gave 1.0 g crude amide as yellow oil.

The nitro amide was dissolved in THF/MeOH (50 ml, 1:1) and reduced using 15 g of freshly prepared Zn/Cu and 1 ml of formic acid (see example 3) which yielded a bright yellow solid. The solid was dissolved in 30 ml of DMF and excess of isoamyl nitrite (8 ml) was added and stirred overnight to get the ring closure product at ambient temperature. The DMF was removed under vaccum, and the product was purified using flash chromatography (hexane: EtOAc 1:1). The solvent was removed under vaccum and the product was crystallized from ether to obtain off white solid of 140 mg with the following properties: MP: 153-155°C; ¹H NMR (300 MHz, CDCl₃) δ 8.79 (1H, s), 8.40 (1H, s), 7.75 (1H, s), 5.84-5.72 (1H, m), 5.39 (1H, d, J = 9 Hz), 5.36 (1H, d, J = 9 Hz), 5.33 (1H, dd, J = 9.0 and 14.0 Hz), 5.10 (1H, dd, J = 4.8 and 14.0 Hz), 1.74 (3H, d, J = 6.9 Hz), 1.60 (9H, s).

### EXAMPLE 54

### 8-tert-Butyl-3-[(2R)-1-(1H-tetrazol-1-yl)propan-2-yl]-7,8-dihydro-3H-[1,3]oxazino[6,5-g][1,2,3]benzotriazine-4,9-dione

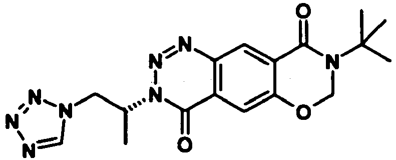

The synthesis of the 1-tetrazolyl derivative was executed by following the procedure in example 53, using (2R)-1-(1H-tetrazol-1-yl)-2-aminopropane (380 mg, 3.0 mmol). After chromatography and crystallization from ether, 130 mg of off white solid were obtained with the following properties: MP: 244-246°C; ¹H NMR (300 MHz, CDCl₃) δ 8.79 (1H, s), 8.53 (1H, s), 7.74 (1H, s), 5.82-5.71 (1H, m), 5.40 (1H, d, J = 9.3 Hz), 5.35 (1H, d, J = 9.3 Hz), 5.18 (1H, dd, J = 9.6 and 14.4 Hz), 4.88 (1H, dd, J = 4.8 Hz and 14.4 Hz), 1.71 (3H, d, J = 6.9 Hz), 1.59 (9H, s).

### EXAMPLE 55

### 8-Cyclopropyl-3-(2-methoxyethyl)-7,8-dihydro-3H-[1,3]oxazino[6,5-g][1,2,3]benzo triazine-4,9-dione

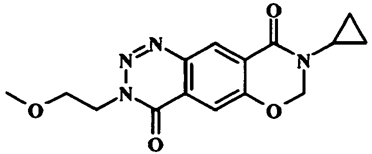

4-Methylsalicylic acid (21.3 g, 140 mmol) was dissolved in methylene chloride (120 mL) and CDI (22.7 g, 140 mmol) was added portion wise causing release of CO₂. The mixture was stirred at ambient temperature for 24 hr, then briefly heated to boiling. A solution of cyclopropylamine (8.0 g, 140 mmol) in triethylamine (5 mL, 36 mmol) was added to the mixture, which was stirred for 3 days. Water (200 mL) was added and the pH was adjusted to 2 using 12 M hydrochloric acid. The phases were separated and the aqueous phase was extracted with chloroform (200 mL). The combined organic phases were washed with sodium bicarbonate solution (100 mL) and dried over sodium sulfate. Concentration gave 22.7 g of amide as an off white solid. NMR showed some contaminants.

The crude amide (22.7 g, 119 mmol) and trioxane (36 g, 0.4 mol) were dissolved in chloroform (250 mL), which was stirred at ambient temperature. Sodium sulfate (32 g) and concentrated sulfuric acid (80 drops) were added and the mixture was refluxed for 30 min, after which conc. sulfuric acid (40 drops) was added. After 90 min, the solids were removed by filtration and washed with ethyl acetate. The solvent was removed under vacuum to give 30 g of oil. The oil was purified using flash chromatography (250 g silica gel, ethyl acetate:hexane 30:70, then 40:60) to give 20.1 g of benzoxazinone as a colorless oil.

The nitration of the benzoxazinone was carried out as in Example 1 using 16 g (79 mmol) of the oil from the previous step, 30 mL Ac₂O and 14 mL nitric acid (90%). 9.4 g of the clean 5-nitro derivative were obtained as an off white solid after crystallization from chloroform/ethyl acetate.

The formation of the nitro acid was carried out following the procedure in Example 52 using 9.4 g (37.8 mmol) of the solid from the previous step, 60 mL Dimethylformamide dimethylacetal, 22.5g (105 mmol) NaIO₄ and 37.7g KMnO₄. The reaction resulted in 7.5 g of a beige/orange acid.

To reduce the nitro group, the nitro acid (1.4 g, 5.0 mmol) from the previous step was dissolved in chloroform:THF (50 mL and 50 mL) and palladium on carbon (10%, 590 mg) was added. The mixture was hydrogenated at ambient temperature for 18 hr. The catalyst was filtered off and the filtrate was concentrated under vacuum to give 2.0 g of a yellow solid, which was used without further purification.

The formation of the amide was carried out as in Example 27 using 2.0 g of the product from the previous reaction, 611 mg (5 mmol) DMAP, 676 mg (5 mmol) HOBT, 1 mL NEt₃, 2.0g (10.4 mmol) EDCI and 2-methoxyethylamine (3 mL, 35 mmol). The reaction yielded 575 mg of the amide as a yellow oil.

The amide from the previous step (0.575 g, 1.88 mmol) was dissolved in DMF (6 mL) and isoamyl nitrite (2 mL) and allowed to stand at ambient temperature for 18 hr. The solvent was removed under vacuum and the residuepurified using flash chromatography (ethyl acetate:hexane, 8:2). The product fractions were combined and concentrated to about 1 mL. Methyl t-butyl ether (8 mL) was added and the solvent was slowly evaporated to yield 206 mg of white crystals with the following properties: MP: 103-105 °C; ¹H NMR (300 MHz, CDCl₃) δ 8.80 (1H, s), 7.84 (1H, s), 5.32 (2H, s), 4.64 (2H, m), 3.87 (2H, m), 3.37 (3H, s), 2.76 (1H, m) and 1.1-0.8 ppm (4H, m).

### EXAMPLE 56

### 8-Cyclopropyl-3-[(2S)-1-(3-fluorophenyl)but-3-yn-2-yl]-7,8-dihydro-3H-[1,3]oxazino [6,5-g][1,2,3]benzotriazine-4,9-dione

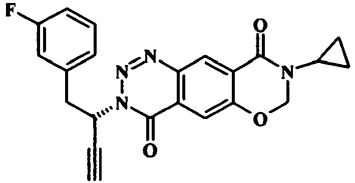

The synthesis of the amide was carried out as in Example 27 using 950 mg (3.83 mmol) of the aminoacid from the previous reaction, 450 mg (3.7 mmol) DMAP, 500 mg (3.7 mmol) HOBT, 2 mL NEt₃, 2.0g (10.4 mmol) EDCI and (S)-2-Amino-1-(3-Fluorophenyl)-3-butyne (650 mg, 3.25 mmol, see example 17). The reaction yielded 1.03 g of the amide as a yellow oil. This material was dissolved in 20 mL DMF and 2 mL isopentyl nitrite were added. After 18 hours at ambient temperature, DMF was removed under vacuum, and the product was purified using flash chromatography with chloroform/ethyl acetate (80/20 → 70/30) as the mobile phase. The solvent was removed under vacuum, and the material was crystallized from methylene chloride/methyl tert-butyl ether to yield pinkish crystals with the following properties: MP: 168-180 °C; ¹H NMR (300 MHz, CDCl₃) δ 8.81 (1H, s), 7.77 (1H, s), 7.3-6.8 (4H, m), 6.11 (1H, m), 5.32 (2H, s), 3.50 (2H, d, J = 7.8 Hz), 2.76 (1H, m), 2.48 (1H, d, J = 2.4 Hz), and 1.1-0.8 ppm (4H, m).

### EXAMPLE 57

### 8-Cyclopropyl-3-[(2R)-1-(2H-tetrazol-2-yl)propan-2-yl]-7,8-dihydro-3H-[1,3]oxazino [6,5-g][1,2,3]benzotriazine-4,9-dione and 8-Cyclopropyl-3-[(2R)-1-(1H-tetrazol-1-yl]propan-2-yl]-7,8-dihydro-3H-[1,3]oxazino[6,5-g][1,2,3]benzotriazine-4,9-dione

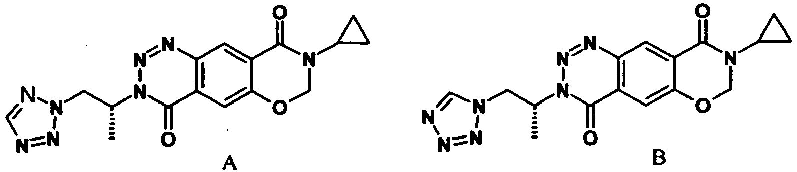

A solution of the aminoacid from Example 55 (2.87 g, 11.5 mmol) in DMF (20 mL) was treated with DMAP (1.40 g, 11.5 mmol), HOBT (0.54 g, 4 mmol), triethylamine (2.8 mL, 20 mmol), (R)-2-amino-1-propanol (3.06 mL, 20 mmol), and EDCI (5.75 g, 30 mmol). After stirring under argon and heating at 40°C for 16 h, the reaction mixture was poured into 10% NaCl (200 mL), neutralized with 6 N HCl, and extracted with chloroform-acetone-methanol, 5 : 1 : 1, 20 x 100 mL). The extract was dried over sodium sulfate, the solvent was removed under reduced pressure, and the residue was chromatographed to give 1.70 g of the desired amidoalcohol.

A solution of the amidoalcohol (1.70 g, 5.56 mmol) in DMF (50 mL) was treated with isoamyl nitrite (10 mL) and acetic acid (0.5 mL). The mixture was stirred at room temperature for 18 h. The volatiles were removed under reduced pressure to give the desired triazinone-alcohol (1.58 g).

Pyridine (2.4 mL, 30 mmol) was added to a stirred solution of the triazinone-alcohol (1.58 g, 5.0 mmol) and methanesulfonic anhydride (1.36 g, 10 mmol) in chloroform (30 mL). The mixture became warm (40°C). Stirring was continued then at room temperature for 4 h. The reaction mixture was poured onto crushed ice (200 g), acidified with 6 N HCl to pH 2, and extracted with chloroform (2 x 100 mL). The extract was dried over magnesium sulfate, concentrated, and the residue was chromatographed (chloroform/ethyl acetate, 5 : 1) to give the desired mesylate (767 mg).

A solution of the mesylate (532 mg, 1.35 mmol) and tetrazole (700 mg, 10 mmol) in DMF (10 mL) was treated with 60% NaH (320 mg, 8 mmol), and the obtained mixture was stirred under argon at 60°C for 20 h. The volatiles were removed under reduced pressure, and the residue was chromatographed (chloroform/THF, 4:1).

The fractions containing the less polar regioisomer A were combined and rechromatographed using ethyl ether as an eluent. A solution of the collected material in ethyl ether (10 mL) was stored in a freezer overnight to give 105 mg white crystals of the 2-tetrazolyl derivative. MP 113-115°C. ¹H NMR (CDCl₃) δ 0.86 (2 H, m), 1.02 (2 H, m), 1.74 (3 H, d, J = 6.9 Hz), 2.77 (1 H, m), 5.11 (1 H, dd, J = 13.8 and 4.8 Hz), 5.32 (1 H, dd, J = 13.5 and 9.0 Hz), 5.33 (2 H, s), 5.77 (1 H, m), 7.75 (1 H, s), 8.40 (1 H, s), and 8.80 (1 H, s).

The fractions enriched in more polar regioisomer B were combined, concentrated under reduced pressure, and the residue was recrystallized from acetonitrile-ethyl ether to give 50 mg of the 1-tetrazolyl isomer. MP: 148-149°C. ¹H NMR (CDCl₃) δ 0.86 (2 H, m), 1.02 (2 H, m), 1.71 (3 H, d, J = 6.9 Hz), 2.76 (1H, m), 4.87 (1H, dd, J = 14.4 and 4.8 Hz), 5.19 (1H, dd, J = 14.1 and 9.3 Hz), 5.33 (2 H, s), 5.76 (1H, m), 7.74 (1H, s), 8.53 (1H, s), and 8.80 (1 H, s).

### EXAMPLE 58

### 8-Cyclopropyl-3-[(2R)-1-(4-nitro-1H-pyrazol-1-yl)propan-2-yl]-7,8-dihydro-3H-[1,3]oxazino[6,5-g][1,2,3]benzotriazine-4,9-dione

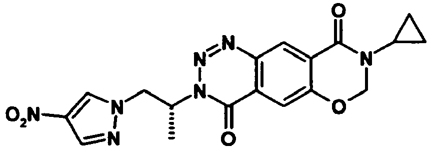

A solution of the mesylate from Example 57 (611 mg, 1.55 mmol) and 4-nitropyrazole (866 mg, 7.7 mmol) in DMF (10 mL) was treated with 60% NaH (160 mg, 4 mmol), and the obtained mixture was stirred under argon at 60°C for 16 h. The reaction mixture was poured onto crushed ice (50 g). The obtained crystalline material was filtered off, dried, and chromatographed (chloroform/ethyl acetate, 4:1).

Final purification by recrystallization from ethanol (15 mL) afforded 318 mg of the nitro pyrazole derivative. MP: 159 -160°C. ¹H NMR (CDCl₃) δ 0.85 (2 H, m), 1.02 (2 H, m), 1.67 (3 H, d, J = 6.9 Hz), 2.76 (1 H, m), 4.58 (1 H, dd, J = 13.9 and 5.1 Hz), 4.85 (1 H, dd, J = 13.9 and 9.5 Hz), 5.33 (2 H, s), 5.77 (1 H, m), 7.76 (1 H, s), 7.95 (1 H, s), 8.02 (1 H, s), and 8.80 (1 H, s).

### EXAMPLE 59

### 8-Cyclopropyl-3-[(2R)-1-(2H-tetrazol-2-yl)propan-2-yl]-3,8-dihydro[1,2,3]triazino[4,5-g]quinazoline-4,9-dione

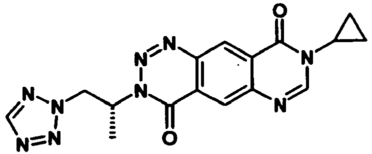

A mixture of formic acid (20 mL, 530 mmol) and acetic anhydride (20 mL, 211 mmol) was heated at 80°C for 1 h. Dimethyl aminoterephthalate (20.92 g, 100 mmol) was added, and the mixture was heated at 75°C for 18 h. The reaction mixture was poured into ethyl ether (300 mL), stirred for 15 min and cooled in a freezer for 1 h. The obtained precipitate was filtered off, washed with ether (100 mL) and dried in a vacuum desiccator to provide the formamide (23.0 g).

The above formamide (23.0 g, 97 mmol) was dissolved in 95% sulfuric acid (100 mL) and cooled (between -10 and -5°C). 90% Nitric acid (5.2 mL, 124 mmol) was added dropwise not allowing the temperature exceed 0°C. After the addition, the mixture was stirred for an additional 1 h with slow warming to 10°C, and then it was poured onto crushed ice (600 g). The precipitate was filtered off, then suspended in water (400 mL), treated with conc. ammonium hydroxide (to pH 8), acidified with acetic acid (to pH 5), filtered off, and washed with water (100 mL). Without drying, the obtained crude nitro derivative was suspended in a mixture of dichloromethane (200 mL) and methanol (200 mL), and hydrogenated over 10% Pd/C at 50 psi. The obtained solution was filtered through a pad of celite, and the solvent was evaporated. The residue was recrystallized from ethanol (120 mL) to give the amino-formamide (16.8 g).

A sample of the amino-formamide (1.26 g, 5 mmol) was treated with cyclopropylamine (0.7 mL, 10 mmol), sodium cyanide (49 mg,1 mmol), and ethanol (0.5 mL), sealed in a pressure tube, and heated at 110°C for 90 min. Column chromatography of the reaction mixture provided the quinazolinone (424 mg).

A solution of the quinazolinone (400 mg, 1.54 mmol) in methanol/THF (10 + 10 mL) was treated with 28% KOH (1.5 g, 7.6 mmol) and stirred at room temperature for 20 h. The mixture was acidified with conc. HCl, and the volatiles were removed under reduced pressure. DMF (20 mL) was added, and evaporation under reduced pressure was repeated to remove all moisture. Another portion of DMF (20 mL) was added followed by EDCI (1.15 g, 6 mmol), HOBT (135 mg, 1.0 mmol), DMAP (242 mg, 2.0 mmol), triethylamine (0.7 mL, 5.0 mmol) and (2R)-1-(2H-tetrazol-2-yl)-2-aminopropane (317 mg, 2.5 mmol, see example 53). The mixture was stirred at 40°C for 3 days. Acetic acid (1.0 mL) and isopentyl nitrite (2.0 mL, 14.9 mmol) were added. The final reaction mixture was stirred at room temperature overnight, then poured into water (100 mL), acidified to pH 3 with 1 N HCl, and extracted with chloroform (3 x 50 mL). The extract was dried over magnesium sulfate, and the volatiles were removed under reduced pressure. The residue was chromatographed (chloroform/ethyl acetate/ethanol/triethylamine, 50 : 45 : 3 : 2). The obtained product was recrystallized from ethanol (20 mL) to give 125 mg of a white solid. MP: 180-181°C. ¹H NMR (CDCl₃) δ 1.00 (2 H, m), 1.28 (2 H, m), 1.77 (3 H, d, J = 7.2 Hz), 3.31 (1 H, m), 5.13 (1 H, dd, J = 13.9 and 4.8 Hz), 5.35 (1 H, dd, J = 13.9 and 8.8 Hz), 5.81 (1 H, m), 8.24 (1 H, m), 8.40 (1 H, s), 8.55 (1 H, s), and 9.10 (1 H, s).

### EXAMPLE 60

### 3-[2-(3-Fluorophenyl)ethyl]-8,9-dihydro-3H-[1,3]oxazolo[2,3-b][1,2,3]triazino[4,5-g]quinazoline-4,11-dione

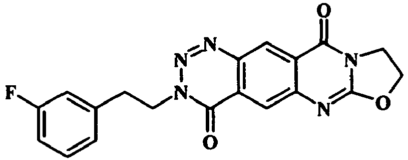

Dimethyl aminoterephthalate (3.5 g, 14.5 mmol) and 2-bromoethyl isocyanate (3.5 g, 23.3 mmol) were dissolved in 1,2-dichloroethane (50 mL). The mixture was heated to reflux (90 °C) for three hours. Triethylamine (5 mL) was added and the mixture was heated at reflux for another 24 hours. The mixture was evaporated onto silica gel (10 g) which was transferred to a silica gel column (100 g) and eluted using chloroform:THF, 80:20. The product fractions were combined and concentrated under vacuum to give 2.6 g (73%) of an off white solid.

The solid from the previous paragraph (1.56 g, 6.3 mmol) was dissolved in chloroform (150 mL). A mixture of nitric acid and sulfuric acid (1:1, 23 mL) was added dropwise. During 2 hr of stirring, the solution temperature increased to 40 °C. The reaction was poured into ice/water and the product was extracted with chloroform. The organic phase was washed with a saturated sodium bicarbonate solution and dried over magnesium sulfate. The solution was concentrated under vacuum to give 1.63 g of white solid.

The solid from the previous paragraph (1.63 g, 5.6 mmol) was dissolved in THF:MeOH (1:1, 100 mL) and the reduction of the nitro group (using freshly prepared Zn/Cu reagent) was carried out as in Example 3. The synthesis of the final product was carried out as in Example 2 substituting 3-fluorophenethylamine for 3-aminopropionitrile. The brown reaction mixture was diluted with chloroform (200 mL) and water (100 mL). The layers were separated and the aqueous layer was extracted with chloroform (2 × 200 mL). The organic layers were combined, dried over magnesium sulfate and concentrated under vacuum. The residue was triturated in methylene chloride (50 mL). The solid was filtered, washed with diethyl ether followed by ethyl acetate, and dried under high vacuum to give 0.92 g of a beige solid. The solid was triturated in methylene chloride to give a white solid with the following properties: MP: 226 - 228 °C; ¹H NMR (300 MHz, CDCl₃) δ 8.98 (1H, s), 8.41 (1H, s), 7.4 - 6.8 (4H, m), 4.87 (2H, m), 4.69 (2H, m), 4.46 (2H, m) and 3.23 ppm (2H, m).

### EXAMPLE 61

### 3-[(2S)-1-(3-Fluorophenyl)but-3-yn-2-yl]-8,9-dihydro-3H-[1,3]oxazolo[2,3-b][1,2,3] triazino[4,5-g]quinazoline-4,11-dione

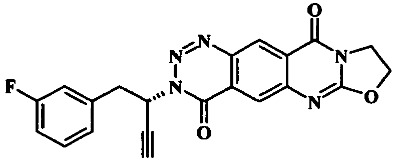

Methyl 7-nitro-5-oxo-2,3-dihydro-5*H*-[1,3]oxazolo[2,3-*b*]quinazoline-8-carboxylate (500 mg, 1.72 mmol, see example 60) was dissolved in THF/Methanol (100 and 50 mL). A solution of 2.0 g sodiumcarbonate in 50 ml water was added and the mixture was stirred at 60°C for 18 hours. The reaction was quenched with HCl (→ pH 3) and the solvent was evaporated. The water phase was extracted with 4x100 ml THF/CHCl₃ (2:1), and dried over sodium sulfate. The solution was concentrated under vacuum to give a yellow solid, which was dissolved in 100 ml DMF and the solvent was evaporated.

This material was dissolved in DMF (50 mL) to which DMAP (230 mg, 1.9 mmol), HOBT (250 mg, 1.9 mmol), triethylamine (1 mL, 7.2 mmol), (1S)-1-(3fluorobenzyl)prop-2-ynylamine (400 mg, 2.0 mmol, see example 17) and EDCI (1.5 g, 7.8 mmol) were added. The mixture was stirred at 25 °C for 18 hours. The DMF was removed under vacuum and ethyl acetate (100 mL) was added. The solution was washed with dilute sulfuric acid (pH 2, 100 mL) followed by sodium bicarbonate solution (100 mL). The aqueous phases were extracted with ethyl acetate (2x100 mL). The organic phases were combined, dried over sodium sulfate and concentrated under vacuum to give 600 mg of crude product, which was used in the next step without further purification.

The solid from the previous step (600 mg) was dissolved in a mixture of THF (40 mL) and methanol (20 mL). The solution was added to freshly prepared Zn/Cu reagent (5 g, see Example 3). Formic acid (0.5 ml) was added and the mixture was stirred at ambient temperature for 15 minutes after which the reduction was complete. The mixture was filtered and the filtrate was evaporated to dryness, then dissolved in DMF (60 mL). An excess of isoamyl nitrite (3 mL) was added and the mixture was stirred over night. The DMF was removed under vacuum and the crude product was purified by column chromatography (silica gel, ethyl acetate). The product fractions were combined and the solvent was removed under vacuum, to give 408 mg product.

This material was dissolved in 120 ml chloroform/20 ml THF, 787 mg Ph₃P and 534 mg NBS were added, and the mixture was stirred for 1 hour. The crude product was purified using flash chromatography (50 g silica gel, ethyl acetate:hexanes 35:65). The product fractions were combined and concentrated under vacuum to give 349 mg material, which was dissolved in 60 ml 1,2-dichloroethane, 2 ml triethylamine and 8 drops DBU were added, and the mixture was heated to 90°C for 18 hours. The crude product was purified using flash chromatography (50 g silica gel, ethyl acetate/chloroform/THF 75/25/0 → 40/25/35). The product fractions were combined and concentrated under vacuum to ~5 ml, which causes the product to crystallize. The off white powder (125 mg) had the following properties: MP: degradation above 230 °C; ¹H NMR (300 MHz, CDCl₃) δ 9.01 (1H, s), 8.38 (1H, s), 7.23-6.87 (4H, m), 6.13 (1H, dt, J = 7.8 and 2.4 Hz), 4.87 (2H, t, J = 8.1 Hz), 4.46 (2H, t, J = 8.1 Hz), 3.52 (2H, d, J = 7.8 Hz), 2.49 ppm (1H, d, J = 2.4 Hz).

### EXAMPLE 62

### 3-[(2R)-1-(2H-Tetrazol-2-yl)propan-2-yl]-8,9-dihydro-3H-[1,3]oxazolo[2,3-b][1,2,3] triazino[4,5-g]quinazoline-4,11-dione

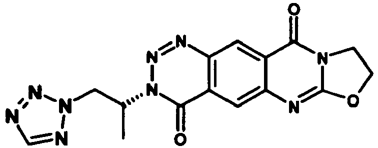

1.0 g (3.83 mmol, see example 60) amino ester was dissolved in 30 ml of THF, a solution of LiOH·H₂O (495 mg, 3.0 eq.) in water (10 mL) was added, and the mixture was stirred at room temperature for 2h. the reaction was quenched with 2N HCl (30 ml) and the solvents evaporated under vaccum which yielded a yellow solid. It was dissolved in 25 ml of 8:2 CH₂Cl₂/DMF, CDI (654 mg, 3.83 mmol) was added in portions and the mixture was stirred at room temperature for 2 h. (2R)-1-(2H-tetrazol-2-yl)-2-amino propane (513 mg, 3.83 mmol, see example 53) and TEA (2 ml) was added slowly to the reaction mixture and stirred at room temperature overnight (under N₂). The solvent was evaporated and the residue dissolved in 100 ml CHCl₃, washed with 2N HCl (2x50 ml), dried over Na₂SO₄ and concentrated under vaccum to obtain a yellow solid. The solids were dissolved in 30 mL DMF, 8 mL Isoamylnitrite was added and the mixture was stirred at 25°C over night to form the triazinones. The crude product was purified using flash chromatography (100 g silica gel, 20% THF in CHCl₃). The product fractions were combined and concentrated under vaccum to give 300 mg light yellow solid. This compound (300 mg) was dissolved in 50 ml of 1:1 THF/CHCl₃, N-bromosuccinimide (600 mg), triphenylphosphine (900 mg) were added in portions and stirred at room temperature overnight. The solvent was evaporated and the product was further purified by flash chromatography (20%THF in CHCl₃) to obtain 420 mg bromo derivative. The bromo derivative was dissolved in 50 ml of 1,2-dichloroethane, 2 ml of TEA and 10 drops of DBU were added, and the mixture was heated to reflux (90°C) overnight. The solvent was evaporated and the crude was purified using flash chromatography (100g silica gel, 30% THF in CHCl₃). The product fractions were combined and concentrated under vaccum to give the desired product which was crystallized from ether to obtain an off white solid (40 mg) with the following properties: MP: 216-218°C; ; is NMR (300 MHz, CDCl₃) δ 8.99 (1H, s), 8.43 (1H, s), 8.33 (1H, s), 5.86-5.75 (1H, m), 5.36 (1H, dd, J = 9.0 and 13.8 Hz), 5.14 (1H, dd, J = 4.8 and 13.8 Hz), 4.89 (2H, t, J=8.4 Hz), 4.46 (2H, t, J=8.4 Hz), 1.72 (3H, d, J = 6.9 Hz).

### EXAMPLE 63

### (4,11-Dioxo-0,8,9,11-tetrahydro-3H-[1,3]oxazolo[2,3-b][1,2,3]triazino[4,5-g]guinazolin-3-yl)acetonitrile

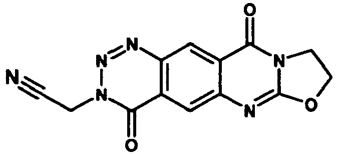

900 mg (3.44 mmol, see example 60) amino ester was dissolved in 30 ml of methanol, 1M NaOH (7 ml) was added and the mixture was stirred at room temperature overnight. The mixture was diluted with 10 ml of H₂O and acidified with 1N HCl (pH∼2) and evaporated under vaccum to obtain the amino acid as a yellow solid. The formation of the amide was carried out as in Example 62 using 900 mg (3.64 mmol) of the product from the previous reaction was treated with aminoacetonitrile bisulfate (648 mg 4.2 mmol) in DMF at room temperature for overnight. The reaction gave 1.0 g crude amide as a yellow solid and the following ring closure (using isoamyl nitrite) was carried out as in Example 62. The crude product was purified using flash chromatography (100 g silica gel, 20% THF in CHCl₃). The product fractions were combined and concentrated under vaccum to give 250 mg light yellow triazinone. This material (250 mg) was dissolved in 50 ml of 1:1 THF/CHCl₃, N-bromosuccinimide (427 mg, 3 eq) and triphenylphosphine (630 mg, 3 eq) were added in portions and stirred at room temperature overnight. The solvent was evaporated and the product was further purified by flash chromatography (20%THF in CHCl₃) to obtain 450 mg bromo derivative. The bromo derivative was dissolved in 50 ml of 1,2-dichloroethane, 2 ml of TEA and 10 drops of DBU were added and the mixture was heated to reflux (90°C) overnight. The solvent was evaporated and the crude was purified using flash chromatography (100g silica gel, 20% THF in CHCl₃). The product fractions were combined and concentrated under vaccum to give the desired product and it was crystallized from ether to obtain an off white solid (20 mg) with the following properties: MP: 240-242°C; ¹H NMR (300 MHz, CDCl₃) δ 9.17 (1H, s), 8.56 (1H, s), 5.37 (2H, s), 5.03 (2H, m), 4.58 (2H, m).

### EXAMPLE 64

### 3-[(2R)-1-(2H-Tetrazol-2-yl)propan-2-yl]-6a,7,9,10-tetrahydro-3H-[1,4]oxazino [3',4':2,3][1,3]oxazino[6,5-g][1,2,3]benzotriazine-4,12-dione

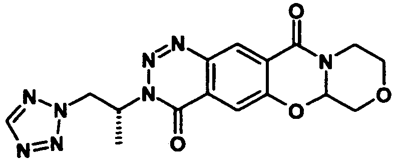

Sodium hydride (60%, 16 g, 400 mmol) was added portionwise to a solution of N-(2-hydroxyethyl)phthalimide (55.3 g, 289 mmol) in DMF (250 mL) stirred under argon. After evolution of hydrogen ceased, the mixture was preheated to 50°C, and bromoacetaldehyde diethyl acetal (70 mL, 466 mmol) was added dropwise in a rate to keep the reaction temperature in the range of 50-70°C. After the addition was accomplished, the mixture was stirred at 50°C for an additional 1 h, and then it was concentrated under reduced pressure. The residue was poured into ice-water (500 mL) and extracted with ethyl ether (3 x 200 mL). The extract was dried over magnesium sulfate, the solvent was evaporated, and the residue was purified by chromatography (chloroform/ethyl acetate, 9 : 1) to give *N*-[2-(2,2-diethoxyethoxy)ethyl]phthalimide (50.6 g).

A solution of the obtained phthalimide (15.2 g, 49.4 mmol) and hydrazine hydrate (5.0 g, 85 mmol) in ethanol (200 mL) was heated at 60°C for 16 h. The solid was filtered off and washed with ethanol (50 mL). After concentration of the filtrate under reduced pressure, the residue treated with ethyl ether (100 mL) to cause precipitation. The new solid was filtered off and washed with ethyl ether (50 mL). The ethereal solutions were combined, and the solvent was evaporated to give (2-aminoethoxy)acetaldehyde diethyl acetal as an oily substance (7,39 g).

A solution of 4-methylsalicylic acid (5.32 g, 35 mmol) and CDI (5.67 g, 35 mmol) in DMF (20 mL) was stirred under argon and heated at 40°C for 3 h. (2-Aminoethoxy)acetaldehyde diethyl acetal (6.45 g, 36.4 mmol) was added, and heating at 40°C was continued for 24 h. The reaction mixture was poured onto crushed ice (100 g), treated with concentrated HCl (100 mL), and stirred for 1 h. The mixture was diluted with brine and stirred for 1 h. The precipitate was filtered off, washed with water (100 mL) and dried to give (5.89 g) of the morpholino benzoxazine.

To 95% sulfuric acid (30 mL), stirred and cooled in an ice-methanol bath, was added portionwise to the morpholino benzoxazine (5.80 g, 23.8 mmol). After complete dissolution, 90% nitric acid (1.40 mL, 30 mmol) was added dropwise in a rate allowing the reaction temperature to be kept in the range of 0 - 5°C. The mixture was stirred then at 0 - 10°C for 30 min, poured onto crushed ice (500 g), and set aside at room temperature overnight. The precipitate was filtered off, washed with water (100 mL) and dried to give desired nitro (6.62 g).

A suspension of the nitro derivative (purity 90%, 35.2 g, 120 mmol) in DMF (200 mL) and Dimethylformamide dimethylacetal (80 mL, 566 mmol) was heated at 130°C for 18 h. The volatiles were evaporated under reduced pressure. A suspension of the obtained enamine in THF (500 mL) was added in portions to a solution of sodium periodate (64.1 g, 300) in water (500 mL) stirred vigorously at room temperature. After the addition, the mixture was stirred at 40-50°C for 2 h, then diluted with water (500 mL) and extracted with chloroform (3 x 500 mL). The extract was dried over magnesium sulfate and concentrated under reduced pressure. The residue was triturated with ethyl acetate (200 mL). The solid was separated by filtration and dried. Concentration of the filtrate gave an additional crop of the desired aldehyde (27.9 g).

A solution of the aldehyde (8.34 g, 30 mmol) in DMF (100 mL) was treated with OXONE (18.44 g, 30 mmol). The obtained mixture was stirred and heated at 40°C for 90 min, then concentrated under reduced pressure. The residue was suspended in water (200 mL) and stirred for 15 min. The precipitate was filtered off, washed with water (100 mL), and dried to give the desired nitroacid (8.58 g).

A solution of the nitroacid (2.35 g, 8 mmol) in DMF (20 mL) was treated with EDCI (2.87 g, 15 mmol), HOBT·H₂O (0.54 g, 4 mmol), DMAP (0.97 g, 8 mmol), triethylamine (1.4 mL, 10 mmol), and (2R)-1-(2H-tetrazol-2-yl)-2-aminopropane (660 mg, 5.2 mmol, see example 53). The reaction mixture was stirred at 40°C for 3 days, then poured into water (100 mL) acidified to pH 2 with 6 N HCl, and extracted with chloroform (2 x 100 mL). The solvent and other volatiles were removed under reduced pressure, and the residue was chromatographed (chloroform/ethyl acetate/ethanol, 50 : 45 :5) to give the desired nitro-amide (1.55 g).

A sample of the obtained nitro-amide (650 mg, 1.61 mmol) was dissolved in a mixture of dichloromethane (50 mL) and methanol (50 mmol) and hydrogenated over 10% Pd/C at 50 psi for 1h. The solution was filtered through a pad of celite, and the solvent was removed under reduced pressure. A solution of the obtained amino-amide in DMF (20 mL) was treated with isopentyl nitrite (3.0 mL, 22.3 mmol) and acetic acid (0.2 mL), and stirred at room temperature for 16 h. The volatiles were removed under reduced pressure, and the residue was chromatographed (chloroform/ethyl acetate/ethanol, 50 : 48 :2). Final purification of the obtained material by recrystallization from ethanol (20 mL) afforded the title compound (370 mg) as a mixture of two diastereomers, MP: 188-190°C. ¹H NMR (CDCl₃) δ 1.67 (3 H, 2 d), 3.20 (1H, m), 3.65 (2 H, m), 4.12 (1H, dd, J = 11.4 and 3.9 Hz), 4.31 (2 H, m), 5.11 (1 H, m), 5.33 (1 H, m), 5.45 (1 H, m), 5.78 (1 H, m), 7.73 (1 H, s), 8.41 (1 H, s), and 8.80 (1 H, s).

### EXAMPLE 65

### 3-[(2R)-1-(1H-Tetrazol-1-yl)propan-2-yl]-6a,7,9,10-tetrahydro-3H-[1,4]oxazino [3',4':2,3][1,3]oxazino[6,5-g][1,2,3]benzotriazine-4,12-dione

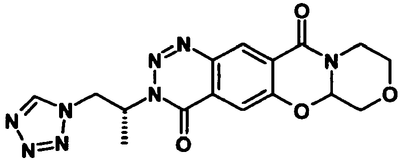

The nitro-acid (1.76 g, 6.0 mmol, see example 64) was dissolved in THF (20 mL) and treated with HOBT (135 mg, 1 mmol), diisopropylcarbodiimide (1.01 mL, 6.5 mmol), and (2R)-1-(1H-tetrazol-1-yl)-2-aminopropane (596 mg, 4.7 mmol, see example 53). The obtained mixture was stirred under argon at 30°C for 5 h and diluted with chloroform (50 mL). The solid was filtered off, washed with chloroform (20 mL), and dried to give the desired nitro-amide (1.17 g).

Reduction of the nitro-acid and cyclocondensation with isoamyl nitrite were carried out in analogy to the corresponding procedures given in Example 64. The crude final product was purified by chromatography (chloroform/ethyl acetate/ethanol, 50 : 45 : 5) and recrystallization from ethanol (25 mL) to afford a mixture of two diastereomers, 250 mg, MP: 200-203°C. ¹H NMR (CDCl₃) δ 1.70 (3 H, 2 d), 3.20 (1 H, m), 3.65 (2 H, m), 4.12 (1 H, dd, J = 11.7 and 3.6 Hz), 4.31 (2 H, m), 4.87 (1 H, dd, J = 14.4 and 4.8 Hz), 5.19 (1 H, m), 5.45 (1 H, m), 5.78 (1 H, m), 7.72 (1 H, s), 8.55 (1 H, s), and 8.79 (1 H, s).

### EXAMPLE 66

### 3-[2-(3-Fluorophenyl)ethyl]-7-(morpholin-4-ylcarbonyl)-1,2,3-benzotriazin-4(3H)-one

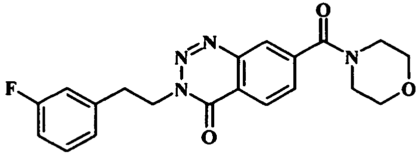

1-Methyl-2-aminoterephthalate (2.93 g, 15 mmol) was dissolved in DMF (60 mL). DMAP (1.8 g, 15 mmol), HOBT (1,7 g, 12.6 mmol), triethylamine (2 mL, 14 mmol) and morpholine (5 mL, 57 mmol) were added followed by EDCI (5 g, 26 mmol). The mixture was stirred at ambient temperature overnight. The DMF was removed under vacuum and water (150 mL) and 2M sulfuric acid were added to reach pH 2. The mixture was extracted with ethyl acetate (4 × 200 mL). The organic phases were combined, washed with a sodium bicarbonate solution (100 mL), dried over sodium sulfate and concentrated under vacuum to give an oil. The oil was further dried under high vacuum to form crystals of the amide derivative (2.8 g).

The amide derivative from the previous step (1.0 g, 3.8 mmol) was dissolved in a mixture of methanol and THF (30 mL each). Sodium carbonate (5 g, 47 mmol, in 50 mL water) was added. The mixture was stirred at ambient temperature for 5 days. 1N hydrochloric acid was added until pH reached 3. The mixture was extracted with ethyl acetate (6 × 150 mL) containing a trace of methanol (to improve solubility in the organic phase). The organic phases were combined, dried over sodium sulfate and concentrated under vacuum. The residue was dissolved in DMF (80 mL) and concentrated to remove remaining traces of water. The residue was dissolved in DMF (30 mL), DMAP (462 mg, 3.8 mmol), HOBT (511 mg, 3.8 mmol), triethyl amine (1 mL), 3-Fluorophenyl ethylamine (1 mL) and EDCI (1.5 g, 7.8 mmol) were added. The mixture was stirred at 45°C for 18 hours and then concentrated under high vacuum. Water (100 mL, pH∼2 with sulfuric acid) was added and the mixture was extracted with ethyl acetate (4 × 150 mL). The organic fractions were washed with saturated sodium bicarbonate solution (100 mL), dried over sodium sulfate and concentrated under vacuum. The product was purified using flash chromatography (AcOEt/Hexane 80/20 → 100/0), which yielded 700 mg yellow oil.

The material was dissolved in DMF (10 mL) and isoamyl nitrite (1 mL) was added. The mixture was stirred at ambient temperature for 18 h. The solvent was removed under vacuum and the residue was transferred to a column (100 g silica gel). The product was eluted with ethyl acetate:hexane (60:40 → 80:20), followed by a second chromatography (chloroform:THF, 95:5). The fractions containing the desired product were combined and concentrated under vacuum. The residue was recrystallized from dichloromethane/MTBE to give 110 mg of a yellowish solid with the following properties: MP: 175-177 °C; ¹H NMR (300 MHz, CDCl₃) δ 8.40 (1H, d, J=8.1 Hz), 8.13 (1H, d, J=1.2 Hz), 7.82 (1H, dd, J=8.1 and 1.5 Hz), 7.3 - 6.9 (4H, m), 4.71 (2H, m) 4.0-3.3 (8H, m) and 3.23 ppm (2H, m).

## Claims

1. A compound according to the formula: 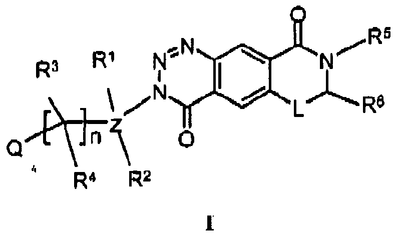 wherein;
R¹ and R² are independently hydrogen, alkyl, substituted alkyl, cycloalkyl, alkynyl, substituted alkynyl, cyano, alkoxy, carboxamido, substituted carboxamido, and if R¹ and R² are alkyl, R¹ and R² may be joined with a bond or-(CH₂)ₘ- to produce a cycloalkane ring,
R³ and R⁴ are independently hydrogen, alkyl, hydroxyl, alkoxy, cyano, fluoro, and if R³ and R⁴ are alkyl, R³ and R⁴ may be joined with a bond or -(CH₂)ₘ- to produce a cycloalkane ring, Q may be hydrogen, alkyl, cycloalkyl, cycloalkenyl, alkoxy, substituted alkoxy, substituted thio, cyano, thionitrile, sulfonamide, substituted sulfonamide, substituted sulfonyl, aromatic, substituted aromatic, heteroaromatic, substituted heteroaromatic, or bicycloheteroaromatic,
R⁵ is hydrogen, alkyl, cycloalkyl, or when R⁵ is alkyl, together with R⁶ may form a heterocycloalkane ring,
R⁶ may be hydrogen, alkyl, substituted alkyl, or together with R⁵, when R⁵ is alkyl, may form heterocycloalkane ring or -OR⁷,
R⁷ is alkyl or, when R⁵ is alkyl, together with R⁵ forms a 5-, 6-, or 7-membered ring,
L may be -O-, -S-, -N= or absent,
Z may be carbon or nitrogen or absent,
m = 1, 2 or 3,
n = 0, 1 or 2, and when n = 0,
Q may be directly bonded to Z;
with the provisos that when the compounds of the formula wherein R⁵ and R⁶ together form a morpholino ring and L is absent, then neither R¹, nor R² may be -C≡C-H; and
when the compounds of the formula wherein R⁵ is cyclopropyl, R¹, R², R³, R⁴, and R⁶ may not all be hydrogen, or Q may not be meta-fluorophenyl,
wherein the term "alkyl" is used herein to refer to a fully saturated monovalent radical containing up to 12 carbons and hydrogen, and which may be a straight chain or branched; the term "cycloalkyl" is used herein to refer to a fully saturated monovalent radical containing up to 8 carbons and hydrogen in a ring; and the term "alkenyl" is used herein to refer to a monovalent radical containing carbon and hydrogen that contains one or two sites of unsaturation, and which may be a straight chain, branched or cyclic;
or a pharmaceutically acceptable addition salt of an acid or base thereof.

2. A compound according to claim 1 above wherein:
R⁵ is hydrogen, alkyl, cycloalkyl or together with R⁶ may form a heterocycloalkane ring, and
L may be -O-, -S-, or -N=;
with the proviso that when R⁵ is cyclopropyl, R¹, R², R³, R⁴, and R⁶ may not all be hydrogen,
or Q may not be meta-fluorophenyl.

3. A compound according to claim 1 above wherein:
R⁵ is hydrogen, alkyl, cycloalkyl or together with R⁶ may form a heterocycloalkane ring, and
L is absent;
with the proviso that when R⁵ and R⁶ together form a morpholino ring, neither R¹, nor R² may be -C≡C-H.

4. A compound according to claim 2 above wherein:
L may be -O- or -N=.

5. A compound according to claim 4 above wherein:
Z is carbon, and
n = 1 or 2.

6. A compound according to claim 5 above wherein:
n = 1.

7. A compound according to claim 4 above wherein:
Z is carbon,
n = 0, and
Q is directly bonded to Z.

8. A compound according to claim 4 above wherein:
L is -O-,
Z is carbon, and
n = 1 or 2.

9. A compound according to claim 4 above wherein:
L is -N=,
Z is carbon, and
n = 1 or 2.

10. A compound according to claim 1 above wherein:
R¹ and R² are independently hydrogen, alkyl, substituted alkyl, cycloalky, alkynyl, substituted alkynyl, cyano, and if R¹ and R² are alkyl, R¹ and R² may be joined with a bond or -(CH₂)ₘ- to produce a cycloalkane ring,
R³ and R⁴ are independently hydrogen, alkyl, hydroxy, fluoro, and if R³ and R⁴ are alkyl, R³ and R⁴ may be joined with a bond or -(CH₂)ₘ- to produce a cycloalkane ring,
Q may be hydrogen, alkyl, cycloalkyl, cycloalkenyl, aromatic, substituted aromatic, heteroaromatic, substituted heteroaromatic, or bicycloheteroaromatic,
R⁵ is alkyl, cycloalkyl or together with R⁶ may form a heterocycloalkane ring,
R⁶ may be alkyl, substituted alkyl, or -OR⁷,
L may be -O-, -S- or -N=,
Z may be carbon or nitrogen,
m = 1, 2 or 3,
n = 0, 1 or 2, and when n = 0,
Q may be directly bonded to Z.

11. A compound according to claim 10 above wherein:
Z carbon, and
n = 1 or 2.

12. A compound according to claim 11 above wherein:
R⁵ and R⁶ together form a heterocycloalkane ring, and
L is -O- or -N=.

13. A compound according to claim 12 above wherein:
L is -O-.

14. A compound according to claim 11 above wherein:
R⁵ is alkyl
R⁶ is -OR⁷, and
R⁷ together with R⁵ forms a 5- or 6-membered ring, and
L is -O- or -N=.

15. A compound according to claim 12 above wherein:
R¹ is hydrogen,
R² is alkyl, substituted alkyl, cycloalkyl, alkynyl, substituted alkynyl, or cyano, and
Q is aromatic, substituted aromatic, heteroaromatic or substituted heteroaromatic.

16. A compound according to claim 15 above wherein:
Q is substituted aromatic, heteroaromatic or substituted heteroaromatic.

17. A compound according to claim 15 above wherein:
R² is alkyl, cycloalky, alkynyl, or cyano,
R³ is hydrogen,
R⁴ is alkyl, hydroxyl, fluoro, and
Q is aromatic, substituted aromatic, heteroaromatic or substituted heteroaromatic.

18. A compound according to claim 15 above wherein:
R² is alkyl, cycloalky, alkynyl, or cyano,
R³ and R⁴ are independently alkyl, hydroxy, fluoro, and if R³ and R⁴ are alkyl,
R³ and R⁴ may be joined with a bond or -(CH₂)ₘ- to produce a cycloalkane ring.

19. A compound according to claim 15 above wherein:
R² is alkyl, cycloalky, alkynyl, or cyano,
R³ and R⁴ are independently alkyl, hydroxyl, fluoro, and if R³ and R⁴ are alkyl,
R³ and R⁴ may be joined with a bond or -(CH₂)ₘ- to produce a cycloalkane ring.

20. A compound of claim 1 according to one of the following:
3-[2-(3-Fluorophenyl)ethyl]-6a,7,8,9-tetrahydro-3*H*-pyrrolo[2',1':2,3][1,3]oxazino[6,5-*g*][1,2,3]benzotriazine-4, 1-dione;
3-(4,11-Dioxo-4,6a,7,8,9,11-hexahydro-3*H*-pyrrolo[2',1':2,3][1,3]oxazino[6,5-*g*][1,2,3] benzotriazin-3 -yl)propanenitrile;
3-Cyclobutyl-6a,7,8,9-tetrahydro-3*H*-pyrrolo[2',1':2,3][1,3]oxazino[6,5-*g*][1,2,3] benzotriazine-4,11-dione;
3-Cyclopropyl-6a,7,8,9-tetrahydro-3*H*-pyrrolo[2',1':2,3][1,3]oxazino[6,5-*g*][1,2,3] benzotriazine-4,11-dione;
3-Ethyl-6a,7,8,9-tetrahydro-3*H*-pyrrolo[2',1':2,3][1,3]oxazino[6,5-*g*][1,2,3]benzotriazine-4,11-dione;
3-(Cyclopropylmethyl)-6a,7,8,9-tetrahydro-3*H*-pyrrolo[2',1':2,3][1,3]oxazino[6,5-*g*][1,2,3]benzotriazine-4,11-dione;
3-*tert*-Butyl-6a,7,8,9-tetrahydro-3*H*-pyrrolo[2',1':2,3][1,3]oxazino[6,5-*g*][1,2,3] benzotriazine-4,11-dione;
3-(Dimethylamino)-6a,7,8,9-tetrahydro-3*H*-pyrrolo[2',1':2,3][1,3]oxazino[6,5-*g*][1,2,3]benzotriazine-4,11-dione;
3-Prop-2-yn-1-yl-6a,7,8,9-tetrahydro-3*H*-pyrrolo[2',1':2,3][1,3]oxazino[6,5-*g*][1,2,3]benzotriazine-4,11-dione;
(4,11-Dioxo-4,6a,7,8,9,11-hexahydro-3*H*-pyrrolo[2',1':2,3][1,3]oxazino[6,5-*g*][1,2,3]benzotriazin-3-yl)acetonitrile;
2-(4,11-Dioxo-4,6a,7,8,9,11-hexahydro-3*H*-pyrrolo[2',1':2,3][1,3]oxazino[6,5-*g*][1,2,3]benzotriazin-3-yl)propanenitrile;
2-(4,11-Dioxo-4,6a,7,8,9,11-hexahydro-3*H*-pyrrolo[2',1':2,3][1,3]oxazino[6,5-*g*][1,2,3]benzotriazin-3-yl)-2-methylpropanenitrile;
3-[(2-Methyl-2*H*-tetrazol-5-yl)methyl]-6a,7,8,9-tetrahydro-3*H* pyrrolo [2',1':2,3] [1,3]oxazino[6,5-*g*][1,2,3]benzotriazine-4,11-dione and 3-[(1-methyl-1*H*-tetrazol-5-yl)methyl]-6a,7,8,9-tetrahydro-3*H*-pyrrolo[2',1':2,3][1,3]oxazino[6,5-*g*][1,2,3] benzotriazine-4,11-dione;
3-(2-Cyclohex-1-en-1-ylethyl)-6a,7,8,9-tetrahydro-3*H*-pyrrolo [2', 1':2,3 ] [1,3]oxazino[6,5-*g*][1,2,3]benzotriazine-4,11-dione;
3-(2-Cyclohexylethyl)-6a,7,8,9-tetrahydro-3*H*-pyrrolo[2',1':2,3][1,3]oxazino[6,5-*g*][1,2,3]benzotriazine-4,11-dione;
(6a*R*)-3-[(2*S*)-1-(3,5-Difluorophenyl)but-3-yn-2-yl]-6a,7,8,9-tetrahydro-3*H*-pyrrolo [2',1':2,3][1,3]oxazino[6,5-*g*][1,2,3]benzotriazine-4,11-dione;
3-[(2*S*)-1-(3-Fluoropheny-1)but-3-yn-2-yl]-6a,7,8,9-tetrahydro-3*H*-pyrrolo[2',1':2,3] [1,3]oxazino[6,5-*g*][1,2,3]benzotriazine-4,11-dione;
3-[(1*S*)-2-(3-Fluorophenyl)-1-isoxazol-3-ylethyl]-6a,7,8,9-tetrahydro-3*H*-pyrrolo [2',1':2,3][1,3]oxazino[6,5-*g*][1,2,3]benzotriazine-4,11-dione;
(2*S*)-2-[(6a*S*)-4,11-Dioxo-4,6a,7,8,9,11-hexahydro-3*H*-pyrrolo[2',1':2,3][1,3]oxazino[6,5-*g*][1,2,3]benzotriazin-3-yl]-3-(3-fluorophenyl)-N-methylpropanamide;
(2*S*)-2-[(6a*R*)-4,11-dioxo-4,6a,7,8,9,11-hexahydro-3*H*-pyrrolo[2', 1':2,3][1,3]oxazino[6,5-*g*][1,2,3]benzotriazin-3-yl]-3-(3-fluorophenyl)-N-methylpropanamide;
(2*S*)-2-[(6a*S*)-4,11-Dioxo-4,6a,7,8,9,11-hexahydro-3*H*-pyrrolo[2',1':2,3][1,3]oxazino[6,5-*g*][1,2,3]benzotriazin-3-yl]-3-(3-fluorophenyl)propanamide;
(2*S*)-2-[(6a*R*)-4,11-dioxo-4,6a,7,8,9,11-hexahydro-3*H*-pyrrolo[2',1':2,3][1,3]oxazino[6,5-*g*][1,2,3]benzotriazin-3-yl]-3-(3-fluorophenyl)propanamide;
(2*S*)-2-[(6a*R*)-4,11-Dioxo-4,6a,7,8,9,11-hexahydro-3*H*-pyrrolo[2',1':2,3][1,3]oxazino[6,5-*g*][1,2,3]benzotriazin-3-yl]-3-(3-fluorophenyl)propanenitrile;
(6a*S*)-3-[(2*S*)-1-(3-Fluorophenyl)-3-hydroxypropan-2-yl]-6a,7,8,9-tetrahydro-3*H-*pyrrolo[2',1':2,3][1,3]oxazino[5,5-*g*][1,2,3]benzotriazine-4,11-dione;
(6a*R*)-3-[(2*S*)-1-(3-fluorophenyl)-3-hydroxypropan-2-yl]-6a,7,8,9-tetrahydro-3*H-*pyrrolo[2',1':2,3][1,3]oxazino[6,5-*g*][1,2,3]benzotriazine-4,11-dione;
3-[1-(3-Nitrophenyl)but-3-yn-2-yl]-6a,7,8,9-tetrahydro-3*H*-pyrrolo[2',1':2,3][1,3] oxazino[6,5-*g*][1,2,3]benzotriazine-4,11-dione;
3-[(1S,2*R*)-1-*H*ydroxy-1-phenylpropan-2-yl]-6a,7,8,9-tetrahydro-3*H*-pyrrolo[2',1':2,3] [1,3]oxazino[6,5-*g*][1,2,3]benzotriazine-4,11-dione;
Erythro-3-[1-(3-fluorophenyl)-1-hydroxypropan-2-yl]-6a,7,8,9-tetrahydro-3*H*-pyrrolo [2',1':2,3][1,3]oxazino[6,5-*g*][1,2,3]benzotriazine-4,11-dione;
3-{[1-(3-Fluorophenyl)cyclopropyl]methyl}-6a,7,8,9-tetrahydro-3*H*-pyrrolo[2',1':2,3] [1,3]oxazino[6,5-*g*][1,2,3]benzotriazine-4,11-dione;
3-[(2*R*)-1-(3-Fluorophenyl)butan-2-yl]-6a,7,8,9-tetrahydro-3*H*-pyrrolo[2',1':2,3][1,3] oxazino[6,5-*g*][1,2,3]benzotriazine-4,11-dione;
3-[(1*R*)-1-Phenylethyl]-6a,7,8,9-tetrahydro-3*H*-pyrrolo[2',1':2,3][1,3]oxazino[5,5-*g*][1,2,3]benzotriazine-4,11-dione;
(6a*S*)-3-[(1*R*)-1-(3-Fluorophenyl)ethyl]-6a,7,8,9-tetrahydro-3*H*-pyrrolo[2',1':2,3] [1,3]oxazino[6,5-*g*][1,2,3]benzotriazine-4,11-dione;
(6a*R*)-3-[(1*R*)-1-(3-fluorophenyl)ethyl]-6a,7,8,9-tetrahydro-3*H-*pyrrolo[2',1':2,3][1,3]oxazino[6,5-*g*][1,2,3]benzotriazine-4, 11-dione;
(6a*S*)-3-[(1*R*)-1-(3,5-Difluorophenyl)ethyl]-6a,7,8,9-tetrahydro-3*H*-pyrrolo[2',1':2,3] [1,3]oxazino[6,5-*g*][1,2,3]benzotriazine-4,11-dione;
(6a*R*)-3-[(1*R*)-1-(3,5-difluorophenyl)ethyl]-6a,7,8,9-tetrahydro-3*H-*pyrrolo[2',1':2,3][1,3]oxazino[6,5-*g*][1,2,3]benzotriazine-4,11-dione;
3-(2,5-Difluorobenzyl)-6a,7,8,9-tetrahydro-3*H*-pyrrola[2',1':2,3][1,3]oxazino[6,5-*g*][1,2,3]benzotriazine-4,11-dione;
3-(1-Pyridin-3-ylethyl)-6a,7,8,9-tetrahydro-3*H*-pyrrolo[2',1':2,3][1,3]oxazino[6,5-*g*][1,2,3]benzotriazine-4,11-dione;
3-(1-Pyridin-4-ylethyl)-6a,7,8,9-tetrahydro-3*H*-pyrrolo[2',1':2,3][1,3]oxazino[6,5-*g*][1,2,3]benzotriazine-4,11-dione;
3-[1-(1,3-Thiazol-2-yl)ethyl]-6a,7,8,9-tetrahydro-3*H*-pyrrolo[2',1':2,3][1,3]oxazino[6,5-*g*][1,2,3]benzotriazine-4,11-dione;
(2*R*)-2-(4,11-Dioxo-4,6a,7,8,9,11-hexahydro-3*H*-pyrrolo[2',1':2,3][1,3]oxazino[6,5-*g*][1,2,3]benzotriazin-3-yl)propyl thiocyanate;
3-[2-(1*H*-Pyrazol-1-yl)ethyl]-6a,7,8,9-tetrahydro-3*H*-pyrrolo[2',1':2,3][1,3]oxazino[6,5-*g*][1,2,3]benzotriazole-4,11-dione;
3-[(2*R*)-1-(2*H*-Tetrazol-2-yl)propan-2-yl]-5a,7,8,9-tetrahydro-3*H*-pyrrolo[2',1':2,3] [1,3]oxazino[6,5-*g*][1,2,3]benzotriazine-4,11-dione;
3-[(2*R*)-1-(1*H*-tetrazol-1-yl)propan-2-yl]-6a,7,8,9-tetrahydro-3*H-*pyrrolo[2',1':2,3][1,3]oxazino[6,5-*g*][1,2,3]benzotriazine-4,11-dione
6a,7,8,9-Tetrahydro-3*H*-pyrrolo[2',1':2,3][1,3]oxazino[6,5-*g*][1,2,3]benzotriazine-4,11-dione; 3-(1-Phenylpent-3-yn-2-yl)-6a,7,8,9-tetrahydro-3*H*-pyrrolo[2',1':2,3][1,3]oxazino[6,5-*g*][1,2,3]benzotriazine-4,11-dione;
3-Methyl-6a,7,8,9-tetrahydro-3*H*-pyrrolo[2',1':2,3][1,3]oxazino[6,5-*g*][1,2,3] benzotriazine-4,11-dione;
3-(3-Fluorobenzyl)-6a,7,8,9-tetrahydro-3*H*-pyrrolo[2'1':2,3][1,3]oxazino[6,5-*g*][1,2,3] benzotriazine-4,11-dione;
3-[3-(3-Fluorophenyl)propyl]-6a,7,8,9-tetrahydro-3*H*-pyrrolo[2',1':2,3][1,3]oxazino[6,5-*g*][1,2,3]benzotriazine-4,11-dione;
3-(1,3-Benzothiazol-2-ylmethyl)-6a,7,8,9-tetrahydro-3*H*-pyrrolo[2',1':2,3] [1,3]oxazino [6,5-*g*][1,2,3]benzotriazine-4,11-dione;
3-(2,1,3-Benzoxadiazol-5-ylmethyl)-6a,7,8,9-tetrahydro-3*H*-pyrrolo[2',1':2,3] [1,3] oxazino[6,5-*g*][1,2,3]benzotriazine-4,11-dione;
3-[(4,11-Dioxo-4,6a,7,8,9,11-hexahydro-3*H*-pyrrolo[2',1':2,3][1,3]oxazino[6,5-*g*][1,2,3] benzotriazin-3-yl)methyl]benzonitrile;
2-[(4,11-Dioxo-4,6a,7,8,9,11-hexahydro-3*H*-pyrrolo[2',1':2,3][1,3]oxazino[6,5-*g*][1,2,3] benzotriazin-3-yl)methyl]benzonitrile;
3-(Pyridin-3-ylmethyl)-6a,7,8,9-tetrahydro-3*H*-pyrrolo[2',1':2,3][1,3]oxazino[6,5-*g*][1,2,3]benzotriazine-4,11-dione;
3-(Pyridin-2-ylmethyl)-6a,7,8,9-tetrahydro-3*H*-pyrrolo[2',1':2,3][1,3]oxazino[6,5-*g*][1,2,3]benzotriazine-4,11-dione;
3-(Pyrazin-2-ylmethyl)-6a,7,8,9-tetrahydro-3*H*-pyrrolo[2',1':2,3][1,3]oxazino[6,5-*g*][1,2,3]benzotriazine-4,11-dione;
3-[(4-Bromo-1*H*-pyrazol-1-yl)methyl]-6a,7,8,9-tetrahydro-3*H*-pyrrolo[2',1':2,3][1,3] oxazino[6,5-*g*][1,2,3]benzotriazine-4,11-dione;
3-[2-(3-Fluorophenyl)ethyl]-8,9-dihydro-3*H*-[1,3]oxazolo[2',3':2,3][1,3]oxazino[6,5-*g*][1,2,3]benzotriazine-4,11-dione;
8-Ethyl-3-[2-(3-fluorophenyl)ethyl]-7,8-dihydro-3*H*-[1,3]oxazino[6,5-*g*][1,2,3]benzotriazine-4,9-dione;
8-tert-Butyl-3-[(2*R*)-1-(2*H*-tetrazol-2-yl)propan-2-yl]-7,8-dihydro-3*H*-[1,3]oxazino[6,5-*g*][1,2,3]benzotriazine-4,9-dione;
8-tert-Butyl-3-[(2*R*)-1-(1*H*-tetrazol-1-yl)propan-2-yl]-7,8-dihydro-3*H*-[1,3]oxazino[6,5-*g*][1,2,3]benzotriazine-4,9-dione;
8-Cyclopropyl-3-(2-methoxyethyl)-7,8-dihydro-3*H*-[1,3]oxazino[6,5-*g*][1,2,3]benzo triazine-4,9-dione;
8-Cyclopropyl-3-[(2*S*)-1-(3-fluorophenyl)but-3-yn-2-yl]-7,8-dihydro-3*H*-[1,3]oxazino [6,5-*g*][1,2,3]benzotriazine-4,9-dione;
8-Cyclopropyl-3-[(2*R*)-1-(2*H*-tetrazol-2-yl)propan-2-yl]-7,8-dihydro-3*H*-[1,3] oxazino[6,5-*g*][1,2,3]benzotriazine-4,9-dione;
8-Cyclopropyl-3-[(2*R*)-1-(1*H*-tetrazol-1-yl)propan-2-yl]-7,8-dihydro-3*H*-[1,3]oxazino[6,5-*g*][1,2,3]benzotriazine-4,9-dione;
8-Cyclopropyl-3-[(2*R*)-1-(4-nitro-1*H*-pyrazol-1-yl)propan-2-yl]-7,8-dihydro-3*H-*[1,3]oxazino[6,5-*g*][1,2,3]benzotriazine-4,9-dione;
8-Cyclopropyl-3-[(2*R*)-1-(2*H*-tetrazol-2-yl)propan-2-yl]-3,8-dihydro[1,2,3]triazino[4,5-*g*]quinazoline-4,9-dione;
3-[2-(3-Fluorophenyl)ethyl]-8,9-dihydro-3*H*-[1,3]oxazolo[2,3-b][1,2,3]triazino[4,5-*g*]quinazoline-4,11-dione;
3-[(2*S*)-1-(3-Fluorophenyl)but-3-yn-2-yl]-8,9-dihydro-3*H*[1,3]oxazolo[2,3-b][1,2,3] triazino[4,5-*g*]quinazoline-4, 1-dione;
3-[(2*R*)-1-(2*H*-Tetrazol-2-yl)propan-2-yl]-8,9-dihydro-3*H*-[1,3]oxazolo[2,3-b][1,2,3] triazino[4,5-*g*]quinazoline-4,11-dione;
(4,11-Dioxo-4,8,9,11-tetrahydro-3*H*-[1,3]oxazolo[2,3-b][1,2,3]triazino[4,5-*g*]quinazolin-3-yl)acetonitrile;
3-[(2*R*)-1-(2*H*-Tetrazol-2-yl)propan-2-yl]-6a,7,9,10-tetrahydro-3*H*-[1,4]oxazino [3',4':2,3][1,3]oxazino [6,5-*g*][1,2,3]benzotriazine-4,12-dione;
3-[(2*R*)-1-(1*H*-Tetrazol-1-yl)propan-2-yl]-6a,7,9,10-tehrahydro-3*H*[1,4]oxazino [3',4':2,3][1,3]oxazino[6,5-*g*][1,2,3]benzotriazine-4,12-dione; or
3-[2-(3-Fluorophenyl)ethyl]-7-(morpholin-4-ylcarbonyl)-1,2,3-benzotriazin-4(3*H*)-one, or a pharmaceutically acceptable addition salt of an acid or base thereof.

21. A pharmaceutical composition comprising an effective amount of a compound according any of claims 1 -20 in combination with a pharmaceutically acceptable carrier, additive or excipient.

22. The composition according to claim 21 wherein said compound comprises about 0.5% to about 75% by weight of said composition and said carrier, additive or excipient comprises about 25% to about 95.5% of said composition.

23. Use of a compound according to any of claims 1-20 in the manufacture of a medicament for use in the treatment of a mammalian subject, wherein the subject suffers from a hypoglutamatergic condition or a deficiency in the number or strength of excitatory synapses or in the number of AMPA receptors, such that memory or other cognitive functions are impaired.

24. Use of a compound according to any of claims 1-20 in the manufacture of a medicament for use in the treatment of schizophrenia.

25. Use of a compound according to any of claims 1-20 in the manufacture of a medicament for use in the treatment of Parkinson's disease.

26. Use of a compound according to any of claims 1-20 in the manufacture of a medicament for use in the treatment of Attention Deficit Hyperactivity Disorder.

27. Use of a compound according to any of claims 1 -20 in the manufacture of a medicament for use in the treatment of Rett Syndrome.

28. Use of a compound according to any of claims 1-20 in the manufacture of a medicament for use in the treatment of Fragile-X Syndrome.

29. Use of a compound according to any of claims 1 -20 in the manufacture of a medicament for use in the treatment of Respiratory Depression.

30. Use of a compound according to any of claims 1-20 in the manufacture of a medicament for use in the treatment of Cognitive Disorders.

31. Use of a compound according to any of claims 1-20 in the manufacture of a medicament for the treatment of Alzheimer's disease.

32. Use of a compound according to any of claims 1-20 in the manufacture of a medicament for the treatment of a mammal subject wherein the subject suffers from a hypoglutamatergic condition or deficiencies in the number or strength of excitatory synapses or in the number of AMPA receptors such that a cortical/striatal imbalance occurs leading to schizophrenia or schizophreniform behavior.

## Patentansprüche

1. Verbindung gemäß der Formel: 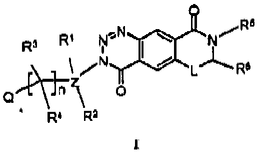 wobei:
R¹ und R² unabhängig voneinander Wasserstoff, Alkyl-, substituierte Alkyl-, Cycloalkyl-, Alkynyl-, substituierte Alkynyl-, Cyano-, Alkoxy-, Carboxamid-, substituierte Carboxamidgruppen sind und, falls R¹ und R² Alkylgruppen sind, R¹ und R² mittels einer Bindung oder -(CH₂)ₘ- vereinigt sein können, wobei ein Cycloalkanring entsteht,
R³ und R⁴ unabhängig voneinander Wasserstoff, Alkyl-, Hydroxy-, Alkoxy-, Cyano-, Fluorogruppen sind und, falls R³ und R⁴ Alkylgruppen sind, R³ und R⁴ mittels einer Bindung oder -(CH₂)ₘ- vereinigt sein können, wobei ein Cycloalkanring entsteht, Q Wasserstoff, eine Alkyl-, Cycloalkyl-, Cycloalkenyl-, Alkoxy-, substituierte Alkoxy-, substituierte Thio-, Cyano-, Thionitril-, Sulfonamid-, substituierte Sulfonamid-, substituierte Sulfonylgruppe, eine aromatische, substituierte aromatische, heteroaromatische, substituierte heteroaromatische oder bicyclische heteroaromatische Gruppe sein kann,
R⁵ Wasserstoff, eine Alkyl- oder Cycloalkylgruppe ist oder, wenn R⁵ eine Alkylgruppe ist, gemeinsam mit R⁶ einen Hetero-Cycloalkanring bilden kann,
R⁶ Wasserstoff, eine Alkyl- oder substituierte Alkylgruppe sein kann oder gemeinsam mit R⁵, wenn R⁵ eine Alkylgruppe ist, einen Hetero-Cycloalkanring oder -OR⁷ bilden kann,
R⁷ eine Alkylgruppe ist oder, wenn R⁵ eine Alkylgruppe ist, gemeinsam mit R⁵ einen 5-, 6- oder 7-gliedrigen Ring bildet,
L -O-, -S-, -N= oder nicht vorhanden sein kann,
Z Kohlenstoff oder Stickstoff oder nicht vorhanden sein kann,
m = 1, 2 oder 3 ist,
n = 0, 1 oder 2 ist, und, wenn n = 0 ist,
Q direkt an Z gebunden sein kann;
mit der Maßgabe, dass dann bei den Verbindungen der Formel, bei denen R⁵ und R⁶ gemeinsam einen Morpholin-Ring bilden und L nicht vorhanden ist,
weder R¹ noch R² -C≡C-H sein kann; und bei den Verbindungen der Formel, bei denen R⁵ Cyclopropyl ist, R¹, R², R³, R⁴ und R⁶ nicht alle Wasserstoff sein können bzw. Q nicht meta-Fluorphenyl sein kann,
wobei der Begriff "Alkyl" bzw. "Alkylgruppe" hier gebraucht wird, um eine vollständig abgesättigte, einwertige funktionelle Gruppe zu bezeichnen, die bis zu 12 Kohlenstoffatome und Wasserstoff umfasst und geradkettig oder verzweigt sein kann; der Begriff "Cycloalkyl" bzw. "Cycloalkylgruppe" hier gebraucht wird,
um eine vollständig abgesättigte, einwertige funktionelle Gruppe mit bis zu 8 Kohlenstoffatomen und Wasserstoff in einem Ring zu bezeichnen; und der Begriff "Alkenyl" bzw. "Alkenylgruppe" hier gebraucht wird, um eine einwertige funktionelle Gruppe zu bezeichnen, die Kohlenstoff und Wasserstoff umfasst,
eine Stelle oder zwei Stellen im ungesättigten Zustand aufweist und geradkettig,
verzweigt oder cyclisch sein kann;
oder ein pharmazeutisch verträgliches Additionssalz von einer Säure oder Base davon.

2. Verbindung nach Anspruch 1, wobei:
R⁵ Wasserstoff, eine Alkyl- oder Cycloalkylgruppe ist oder gemeinsam mit R⁶ einen Hetero-Cycloalkanring bilden kann, und
L -O-, -S- oder -N= sein kann;
mit der Maßgabe, dass dann, wenn R⁵ eine Cyclopropylgruppe ist, R¹, R², R³, R⁴ und R⁶ nicht alle Wasserstoff sein können bzw. Q nicht meta-Fluorphenyl sein kann.

3. Verbindung nach Anspruch 1, wobei:
R⁶ Wasserstoff, eine Alkyl- oder Cycloalkylgruppe ist oder gemeinsam mit R⁶ einen Hetero-Cycloalkanring bilden kann, und
L nicht vorhanden ist;
mit der Maßgabe, dass dann, wenn R⁶ und R⁶ gemeinsam einen Morpholin-Ring bilden, weder R¹ noch R² -C≡C-H sein kann.

4. Verbindung nach Anspruch 2, wobei:
L -O- oder -N= sein kann.

5. Verbindung nach Anspruch 4, wobei:
Z Kohlenstoff ist, und
n = 1 oder 2 ist.

6. Verbindung nach Anspruch 5, wobei:
n = 1 ist.

7. Verbindung nach Anspruch 4, wobei:
Z Kohlenstoff ist,
n = 0 ist und
Q direkt an Z gebunden ist.

8. Verbindung nach Anspruch 4, wobei:
L -O- ist,
Z Kohlenstoff ist, und
n = 1 oder 2 ist,

9. Verbindung nach Anspruch 4, wobei:
L -N= ist,
Z Kohlenstoff ist, und
n = 1 oder 2 ist.

10. Verbindung nach Anspruch 1, wobei:
R¹ und R² unabhängig voneinander Wasserstoff, Alkyl-, substituierte Alkyl-, Cycloalkyl-, Alkynyl-, substituierte Alkynyl-, Cyanogruppen sind und, falls R¹ und
R² Alkylgruppen sind, R¹ und R² mittels einer Bindung oder -(CH₂)ₘ- vereinigt sein können, wobei ein Cycloalkanring entsteht,
R³ und R⁴ unabhängig voneinander Wasserstoff, Alkyl-, Hydroxy-,
Fluorogruppen sind und, falls R³ und R⁴ Alkylgruppen sind, R³ und R⁴ mittels
einer Bindung oder -(CH₂)ₘ- vereinigt sein können, wobei ein Cycloalkanring entsteht,
Q Wasserstoff, eine Alkyl-, Cycloalkyl-, Cycloalkenylgruppe, eine aromatische, substituierte aromatische, heteroaromatische, substituierte heteroaromatische oder bicyclische heteroaromatische Gruppe sein kann,
R⁵ eine Alkyl- oder Cycloalkylgruppe ist oder gemeinsam mit R⁶ einen Hetero-Cycloalkanring bilden kann,
R⁶ eine Alkylgruppe, substituierte Alkylgruppe oder -OR⁷ sein kann,
L -O-, -S- oder -N= sein kann,
Z Kohlenstoff oder Stickstoff sein kann,
m = 1, 2 oder 3 ist,
n = 0, 1 oder 2 ist, und, wenn n = 0 ist,
Q direkt an Z gebunden sein kann.

11. Verbindung nach Anspruch 10, wobei:
Z Kohlenstoff ist, und
n = 1 oder 2 ist.

12. Verbindung nach Anspruch 11, wobei:
R⁵ und R⁵ gemeinsam einen Hetero-Cycloalkanring bilden, und
L -O- oder -N= ist.

13. Verbindung nach Anspruch 12, wobei:
L -O- ist.

14. Verbindung nach Anspruch 11, wobei:
R⁵ eine Alkylgruppe ist,
R⁶ -OR⁷ ist und
R⁷ gemeinsam mit R⁵ einen 5- oder 6-gliedrigen Ring bildet und
L -O- oder -N= ist.

15. Verbindung nach Anspruch 12, wobei:
R¹ Wasserstoff ist,
R² eine Alkyl-, substituierte Alkyl-, Cycloalkyl-, Alkynyl-, substituierte Alkynyl- oder Cyanogruppe ist
und
Q eine aromatische, substituierte aromatische, heteroaromatische oder substituierte heteroaromatische Gruppe ist.

16. Verbindung nach Anspruch 15, wobei:
Q eine substituierte aromatische, heteroaromatische oder substituierte heteroaromatische Gruppe ist.

17. Verbindung nach Anspruch 15, wobei:
R² eine Alkyl-, Cycloalkyl-, Alkynyl- oder Cyanogruppe ist,
R³ Wasserstoff ist,
R⁴ eine Alkyl-, Hydroxy- oder Fluorogruppe ist und
Q eine aromatische, substituierte aromatische, heteroaromatische oder substituierte heteroaromatische Gruppe ist.

18. Verbindung nach Anspruch 15, wobei:
R² eine Alkyl-, Cycloalkyl-, Alkynyl- oder Cyanogruppe ist,
R³ und R⁴ unabhängig voneinander Alkyl-, Hydroxy-, Fluorogruppen sind und, falls R³ und R⁴ Alkylgruppen sind,
R³ und R⁴ mittels einer Bindung oder -(CH₂)ₘ- vereinigt sein können, wobei ein Cycloalkanring entsteht.

19. Verbindung nach Anspruch 15, wobei:
R² eine Alkyl-, Cycloalkyl-, Alkynyl- oder Cyanogruppe ist,
R³ und R⁴ unabhängig voneinander Alkyl-, Hydroxy-, Fluorogruppen sind und, falls R³ und R⁴ Alkylgruppen sind,
R³ und R⁴ mittels einer Bindung oder -(CH₂)ₘ- vereinigt sein können, wobei ein Cycloalkanring entsteht.

20. Verbindung nach Anspruch 1, einer der folgenden Verbindungen entsprechend:
3-[2-(3-Fluorphenyl)ethyl]-5a,7,8,9.tetrahydro-3*H*-pyrrolo[2',1':2,3][1,3]oxazin[6, 5-g][1,2,3]benzotriazin-4,11-dion;
3-(4,11-Dioxo-4,5a,7,8,9,11-hexahydro-3*H*-pyrrolo[2',1':2,3][1,3]oxazin[5,5-g][1, 2,3]benzotriazin-3-yl)propannitril;
3-Cyclobutyl-6a,7,8,9,tetrahydro-3*H*-pyrrolo[2',1':2,3][1,3]oxazin[6,5-g][1,2, 3]benzotriazin-4,11-dion;
3-Cyclopropyl-6a,7,8,9-tetrahydro-3*H*-pyrrolo[2',1':2,3][1,3]oxazin[6,5-g][1,2, 3]benzotriazin-4,11-dion;
3-Ethyl-6a,7,8,9-tetrahydro-3*H*-pyrrolo[2',1':2,3][1,3]oxazin[6,5-g][1,2,3]benzotriazin-4,11-dion;
3-(Cyclopropylmethyl)-6a,7,8,9-tetrahydro-3*H*-pyrrolo[2',1':2,3][1,3]oxazin[6,5-g] [1,2,3]benzotriazin-4,11-dion;
3-*tert*-Butyl-6a,7,8,9-tetrahydro-3*H*-pyrrolo[2',1':2,3][1,3]oxazin[6,5-g][1,2, 3]benzotriazin-4,11-dion;
3-(Dimethylamino)-6a,7,8,9-tetrahydro-3*H*-pyrrolo[2',1':2,3][1,3]oxazin[6,5-g][1, 2,3]benzotriazin-4,11-dion;
3-Prop-2-yn-1-yl-6a,7,8,9-tetrahydro-3*H*-pyrrolo[2',1':2,3][1,3]oxazin[6,5-g][1,2, 3]benzotriazin-4,11-dion;
(4,11-Dioxo-4,7a,7,8,9,11-hexahydro-3*H*-pyrrolo[2',1':2,3][1,3]oxazin[6,5-g][1,2, 3]benzotriazin-3-yl)acetonitril;
2-(4,11-Dioxo-4,6a,7,8, 9,11-hexahydro-3*H*-pyrrolo[2',1':2,3][1,3]oxazin[6,5-g][1, 2,3]benzotriazin-3-yl)propannitril;
2-(4,11-Dioxo-4,6a-7,8,9,11-hexahydro-3*H*-pyrrolo(2',1':2,3][1,3]oxazin[6,5-g][1, 2,3]benzotriazin-3-yl)-2-methylpropannitril;
3-[(2-Methyl-2*H*-tetrazol-5-yl)methyl]-6a,7,8,9-tetrahydro-3*H*-pyrrolo[2',1':2,3][1, 3]oxazin[6,5-g][1,2,3]benzotriazin-4,11-dion und
3-[(1-Methyl-1*H*-tetrazol-5-yl)methyl]-6a,7,8,9-tetrahydra-3*H*-pyrrolo[2',1':2,3][1, 3]oxazin[6,5-g][1,2,3]benzotriazin-4,11-dion;
3-(2-Cyclohex-1-en-1-ylethyl)-6a,7,8,9,tetrahydro-3*H*-pyrrolo[2',1':2,3][1,3]oxazin[5,5-g][1,2,3]benzotriazin-4,1 1-dion;
3-(2-Cyclohexylethyl)-6a,7,8,9-tetrahydro-3*H*-pyrrolo[2',1':12,3][1,3]oxazin[6,5-g] [1,2,3]benzotriazin-4,11-dion:
(6a*R*)-3-[(2*S*)-1-(3,5-Difluorphenyl)but-3-yn-2-yl]-6a,7,8,9-tetrahydro-3*H*-pyrrolo [2',1':2,3][1,3]oxazin[6,5-g][1,2,3]benzotriazin-4,11-dion;
3-[(2*S*)-1-(3-Fluorphenyl)but-3-yn-2-yl]-5a,7,8,9-tetrahydro-3H-pyrrolo[2',1':2,3] [1,3]oxazin[6,5-g][1,2,3]benzotriazin-4,11-dion;
3-[(1*S*)-2-(3-Fluorphenyl)-1-isoxazol-3-ylethyl]-6a,7,8,9-tetrahydro-3*H*-pyrrolo [2',1':2,3][1,3]oxazin[5,5-g][1,2,3]benzotriazin-4,11-dion;
(2*S*)-2-[(6a*S*)-4,11-Dioxo-4,6a,7,8,9,11-hexahydro-3*H*-pyrrolo[2',1':2,3][1,3]oxazin[5,5-g][1,2,3]benzotriazin-3-yl]-3-(3-fluorphenyl)-*N*-methylpropanamid;
(2*S*)-2-[(6a*R*)-4,11-dioxo-4,6a,7,8,9,11-hexahydra-3*H*-pyrrolo[2',1':2,3][1,3]oxazin[6,5-g][1,2,3]benzotriazin-3-yl]-3-(3-fluorphenyl)-*N*-methylpropanamid;
(2*S*)-2-[(6a*S*)-4,11-Dloxo-4,6a,7,8,9,11-hexahydro-3*H*-pyrrolo[2',1':2,3][1,3]oxazin[6,5-g][1,2,3]benzatriazin-3-yl]-3-(3-fluorphenyl)propanamid;
(2*S*)-2-[(6a*R*)-4,11-dioxa-4,6a,7,8,9,11-hexahydro-3*H*-pyrrolo[2',1':2,3][1,3]oxazin[6,5-g][1,2,3]benzotriazin-3-yl]-3-(3-fluorphenyl)propanamid;
(2*S*)-2-[(6a*R*)-4,11-Dioxo-4,6a,7,8,9,1 1.hexahydro-3*H*-pyrrolo[2',1':2,3][1,3] oxazin[6,5 g][1,2,3]benzotriazin-3-yl]-3-(3-fluorphenyl)propannitril;
(6a*S*)-3-[(2*S*)-1-(3-Fluorphenyl)-3-hydroxypropan-2-yl]-Ba,7,B,9-tetrahydro-3*H-*pyrrolo[2',1':2,3][1,3]oxazin[6,5-g][1,2,3]benzotriazin-4,11-dion;
(6a*R*)-3-[(2*S*)-1-(3-Fluorphenyl)-3-hydxoxypropan-2-yl]-6a,7,8,9-tetra hydro-3*H-*pyrrolo[2',1':2,3][1,3]oxazin[6,5-g][1,2,3]benzotriazin-4,11-dion;
3-[1-(3-Nitrophenyl)but-3-yn-2-yl]-9a,7,8,9-tetrahydro-3*H*-pyrrolo[2',1':2,3][1,3] oxazin[6,5-g][1,2,3]benzotriazin-4,11-dion:
3-[(1*S*,2*R*)-1-Hydroxy-1-phenylpropan-2-yl]-5a,7,8,9-tetrahydro-3*H*-pyrrolo[2',1': 2,3][1,3]oxazin[6,5-g][1,2,3]benzotriazin-4,11-dion;
Erythro-3-[1-(3-fluorphenyl)-1-hydroxypropan-2-yl]-6a,7,8,9-tetrahydro-3*H*-pyrrolo[2',1':2,3][1,3]oxazin[6,5-g][1,2,3]benzotriazin-4,11-dion;
3-{[1-(3-Fiuorphenyl)cydopropyl]methyl}-5a,7,8,9-tetrahydro-3*H*-pyrrolo[2',1':2, 3][1,3]oxazin[6,5-g][1,2,3]benzotriazin-4,11-dion;
3-[(2*R*)-1-(3-Fluorphenyl)butan-2-yl]-6a,7,8,9-tetrahydro-3*H*-pyrrolo[2',1':2,3][1, 3]oxazin[6,5-g][1,2,3]benzotriazin,4,11-dion;
3-[(1*R*)-1-Phenylethyl]-6a,7,8,9-tetrahydro-3*H*-pyrrolo[2',1':2,3][1,3]oxazin[6,5-g] [1,2,3]benzotriazin-4,11-dion;
(6a*S*)-3-[(1*R*)-1-(3-Fluorphenyl)ethyl]-6a,7,8,9-tetrahydro-3*H*-pyrrolo[2',1':2,3][1, 3]oxazin[6,5-g][1,2,3]benzotriazin-4,11-dion:
(6a*R*)-3-[(1*R*)-1-(3-Fluorphenyl)ethyl]-6a,7,8,9-tetrahydro-3*H*-pyrrolo[2',1':2,3][1, 3]oxazin[6,5-g][1,2,3]benzotriazin-4,11-dion;
(6a*S*)-3-[(1*R*)-1-(3,5-Difluorphenyl)ethyl]-6a,7,8,9-tetrahydro-3*H*-pyrrolo[2',1':2, 3][1,3]oxazin[6,5-g][1,2,3]benzotriazin-4,11-dion;
(6a*R*)-3-[(1*R*)-1-(3,5-Difluorphenyl)ethyl]-6a,7,8,9-tetrahydro-3*H*-pyrrolo[2',1':2, 3][1,3]oxazin[6,5-g][1,2,3]benzotriazin-4,11-dion;
3-(2,5-Difluorbenzyl)-6a,7,8,9-tetrahydro-3*H*-pyrrolo[2',1':2,3][1,3]oxazin[6,5-g] [1,2,3]benzotriazin-4,11-dion;
3-(1-Pyridin-3-ylethyl)-6a,7,8,9-tetrahydro-3*H*-pyrralo[2',1':2,3][1,3]oxazin[6,5-g] [1,2,3]benzotriazin-4,11-dion;
3-(1-Pyridin-4-ylethyl)-5a,7,8,9-tetrahydro-3*H*-pyrrolo[2',1':2,3][1,3]oxazin[6,5-g] [1,2,3]benzotriazin-4,11-dion;
3-[1-(1,3-Thiazol-2-yl)ethyl]-6a,7,8,9-tetrahydro-3*H*-pyrrolo[2',1':2,3][1,3]oxazin [6,5-g)[1,2,3]benzotriazin-4,11-dion;
(2*R*)-2-(4,11-Dioxo-4,6a,7,8,9,11-hexahydro-3*H*-pyrrolo[2',1':2,3][1,3]oxazin[6,5 -g][1,2,3]benzotriazin-3-yl)propylthiocyanat;
3-[2-(1*H*-Pyrazol-1-yl)ethyl]-5a,7,8,9-tetrahydro-3*H*-pyrrolo[2',1':2,3][1,3]oxazin [6,5-g][1,2,3]benzotriazin-4,11-dion;
3-[(2*R*)-1-(2*H*-Tetrazol-2-yl)propan-2-yl]-6a,7,8,9-letrahydro-3*H*-pyrrolo[2',1':2,3 ][1,3]oxazin[6,5-g][1,2,3]benzotriazin-4,11-dion;
3-[(2*R*)-1-(1*H*-tetrazol-1-yl)propan-2-yl]-6a,7,8,9-tetrahydro-3*H*-pyrrolo[2',1':2,3] [1,3]oxazin[6,5-g]benzotriazin-4.11-dion;
6a,7,8,9-Tetrahydro-3*H*-pyrrolo[2',1':2,3][1,3]oxazin[6,5-g][1,2,3]benzotriazin-4, 11-dion;
3-(1-Phenylpent-3-yn-2-yl)-6a,7,8,9-tetrahydro-3*H*-pyrroio[2',1':2,3][1,3]oxazin [6,5-g][1,2,3]benzotriazin-4,11-dion;
3-Methyl-6a,7,8,9-tetrahydro-3*H*-pyrrolo[2',1':2,3][1,3]oxazin[6,5-g][1,2,3]benzotriazin-4.11-dion;
3-(3-Fluorbenzyl)-6a,7,8,9-tetrahydro-3*H*-pyrrolo[2',1':2,3][1,3]oxazin[6,5-g][1,2, 3]benzotriazin-4,11-dion;
3-[3-(3-Fluorphenyl)propyl]-5a,7,8,9-tetrahydro-3*H*-pyrrolo[2',1';2,3][1,3]oxazin [6,5-g][1,2,3]benzotriazin-4,11-dion;
3-(1,3-Benzothiazol-2-ylmethyl)-6a,7,8,9-tetrahydro-3*H*-pyrrolo[2',1':2,3][1,3] oxazin[6,5-g][1,2,3]benzotriazin-4,11-dion;
3-(2,1,3-Benzoxadiazol-5-ylmethyl)-6a,7,8,9-tetrahydro-3*H*-pyrrolo[2',1':2,3][1,3] oxazin[6,5-g][1,2,3]benzotriazin-4,11-dion;
3-[(4,11-Dioxo-4,6a,7,8,9,11-hexahydro-3*H*-pyrrolo[2',1':2,3][1,3]oxazin[6,5-g] [1,2,3]benzotriazin-3-yl)methyl]benzonitril;
2-[(4,11-Dioxo-4,6a,7,8,9,11-hexahydro-3*H*-pyrrolo[2',1':2,3][1,3]oxazin[6,5-g] [1,2,3]benzotriazin-3-yl)methyl]benzonitril;
3-(Pyridin-3-ylmethyl)-6a,7,8,9-tetrahydro-3*H*-pyrrolo[2',1':2,3][1,3]oxazin[6,5-g] [1,2,3]benzotriazin-4,11-dion;
3-(Pyridin-2-ylmethyl)-6a,7,8,9-tetrahydro-3*H*-pyrrolo[2',1':2,3][1,3]oxazimo[6,5-g][1,2,3]benzotriazin-4,11-dion;
3-(Pyrazin-2-ylmethyl)-6a,7,6,9-tetrahydro-3*H*-pyrrolo[2',1':2,3][1,3]oxazin[6,5-g] [1,2,3]benzotriazin-4,11-dion;
3-[(4-Brom-1*H*-pyrazol-1-yl)methyl]-6a,7,8,9-tetrahydro-3*H*-pyrrolo[2',1':2,3][1,3] oxazin[6,5-g][1,2,3]benzotriazin-4,11-dion;
3-[2-(3-Fluorphenyl)ethyl]-8,9-dihydro-3*H*-[1,3]oxazolo[2',3':2,3][1,3]oxazin[6,5-g][1,2,3]benzotriazin-4,11-dion;
8-Ethyl-3-[2-(3-fluorphenyl)ethyl]-7,8-dihydro-3*H*-[1,3]oxazin[6,5-g][1,2,3]benzotriazin-4,9-dion;
8-*tert*-Butyl-3-[(2*R*)-1-(2*H*-tetrazol-2-yl)propan-2-yl]-7,8-d ihydro-3*H*-[1,3]oxazin [6,5-g][1,2,3]benzotriazin-4,9-dion;
8-*tert*-Butyl-3-[(2*R*)-1-(1*H*-tetrazol-1-yl)propan-2-yl]-7,8-diydro-3*H*-[1,3]oxazin[6, 5-g][1,2,3]benzotriazin-4,9-dion;
8-Cyclopropyl-3-(2-methoxyethyl)-7,8-dihydro-3*H*-[1,3]oxazin[6,5-g][1,2,3]benzotriazin-4,9-dion;
8-Cyclopropyl-3-[(2*S*)-1-(3-fluorphenyl)but-3-yn-2-yl]-7,8-dihydro-3*H*-[1,3]oxazin [6,5-g][1,2,3]benzotriazin-4,9-dion;
8-Cyclopropyl-3-[(2*R*)-1-(2*H*-tetrazol-2-yl)propan-2-yl]-7,8-dihydro-3*H*-[1,3]oxazin[6,5-g][1,2,3]benzotriazin-4,9-dion;
8-Cyclopfopyl-3-[(2R)-1-(1*H*-tetrazol-1-yl)propan-2-yl]-7,8-dihydro-3*H*-[1,3]oxazin[6,5-g][1,2,3]benzotriazin-4,9-dion;
8-Cydopropyl-3-[(2*R*)-1-(4-nitro-1*H*-pyrazol-1-yl)propan-2-yl]-7,8-dihydro-3*H*-[1, 3]oxazin[6,5-g][1,2,3]benzotriazin-4,9-dion;
8-Cyclopropyl-3-[(2*R*)-1-(2*H*-tetrazol-2-yl)propan-2-yl]-3,6-dihydro[1,2,3]triazin [4,5-g]chinazolin-4,9-dion;
3-[2-(3-Fluorphenyl)ethyl]-8,9-dihydro-3*H*-[1,3]oxazol[2,3-b][1,2,3]triazin[4,5-g] chinazolin-4,11-dion;
3-[(2*S*)-1-(3-Fluorphenyl)but-3-yn-2-yl)-8,9-dihydro-3*H*-[1,3]oxazol[2,3-*b*][1,2,3] triazin[4,5-g]chinazolin-4,11-dion;
3-[(2*R*)-1-(2*H*-Tetrazol-2-yl)propan-2-yl]-8,9-dihydro-3*H*-[1,3]oxazol[2,3-*b*][1,2,3] triazin[4,5-g]chinazolin-4,11-dion;
(4,11-Dioxo-4,8,9,11-tetrahydro-3*H*-[1,3]oxazol[2,3-*b*][1,2,3]triazin[4,5-g]chinazolin-3-yl)acetonitril;
3-[(2*R*)-1-(2*H*-Tetrazol-2-yl)propan-2-yl]-6a,7,9,10-tetrahydro-3*H*-[1,4]oxazin[3', 4':2,3][1,3]oxazin[6,5-g][1,2,3]benzotriazin-4,12-dion;
3-[(2*R*)-1-(1*H*-Tetrazol-1-yl)propan-2-yl]-6a,7,9,10-tetrahydro-3*H*-[1,4]oxazin[3', 4':2,3][1,3]oxazin[6,5-g](1,2,3]benzotriazin-4,12-dion; oder
3-[2-(3-Fluorphenyl)ethyl]-7-(morpholin-4-ylcarbonyl)-1,2,3-benzotriazin-4(3*H*) -on
oder ein pharmazeutisch verträgliches Additionssalz einer Säure oder Base davon.

21. Pharmazeutische Zusammensetzung, die eine wirksame Menge einer Verbindung nach einem der Ansprüche 1 bis 20 in Kombination mit einem pharmazeutisch verträgliche Träger-, Zusatz- oder Hilfsstoff umfasst,

22. Zusammensetzung nach Anspruch 21, wobei die Verbindung etwa 0,5 Gew.-% bis etwa 75 Gew.-% der Zusammensetzung ausmacht und der Träger-, Zusatz- oder Hilfsstoff etwa 25 % bis etwa 95,5 % der Zusammensetzung ausmacht.

23. Verwendung einer Zusammensetzung nach einem der Ansprüche 1 bis 20 zur Herstellung eines Medikaments zur Verwendung bei der Behandlung eines Säugetiers, das an einem Mangel am Neurotransmitter Glutamat oder an einem Defizit bei der Anzahl oder Reizstärke der erregenden Synapsen oder bei der Anzahl der AMPA-Rezeptoren leidet, derart, dass Gedächtnis- und andere kognitive Funktionen beeinträchtigt sind.

24. Verwendung einer Verbindung nach einem der Ansprüche 1 bis 20 zur Herstellung eines Medikaments zur Verwendung bei der Behandlung von Schizophrenie.

25. Verwendung einer Verbindung nach einem der Ansprüche 1 bis 20 zur Herstellung eines Medikaments zur Verwendung bei der Behandlung der Parkinsonkrankheit.

26. Verwendung einer Verbindung nach einem der Ansprüche 1 bis 20 zur Herstellung eines Medikaments zur Verwendung bei der Behandlung des Aufmerksamkeits-Defizit-Syndroms mit Hyperaktivität.

27. Verwendung einer Verbindung nach einem der Ansprüche 1 bis 20 zur Herstellung eines Medikaments zur Verwendung bei der Behandlung des Rett-Syndroms.

28. Verwendung einer Verbindung nach einem der Ansprüche 1 bis 20 zur Herstellung eines Medikaments zur Verwendung bei der Behandlung des Fragilen-X-Syndroms.

29. Verwendung einer Verbindung nach einem der Ansprüche 1 bis 20 zur Herstellung eines Medikaments zur Verwendung bei der Behandlung von Atemdepression.

30. Verwendung einer Verbindung nach einem der Ansprüche 1 bis 20 zur Herstellung eines Medikaments zur Verwendung bei der Behandlung von kognitiven Störungen.

31. Verwendung einer Verbindung nach einem der Ansprüche 1 bis 20 zur Herstellung eines Medikaments zur Behandlung der Alzheimerkrankheit.

32. Verwendung einer Zusammensetzung nach einem der Ansprüche 1 bis 20 zur Herstellung eines Medikaments zur Behandlung eines Säugetiers, das an einem Mangel am Neurotransmitter Glutamat oder an einem Defizit bei der Anzahl oder Reizstärke der erregenden Synapsen oder der Anzahl der AMPA-Rezeptoren leidet, derart, dass ein Llngleichgewicht zwischen kortikaler und striataler Aktivität auftritt, das zu Schizophrenie oder schizophreniformem Verhalten führt.

## Revendications

1. Composé selon la formule : 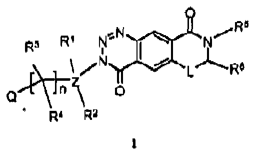 où :
R¹ et R² sont indépendamment hydrogène, alkyle, alkyle substitué, cycloalkyle, alkynyle, alkynyle substitué, cyano, alkoxy, carboxamido, carboxamido substitué, et si R¹ et R² sont alkyle, R¹ et R² peuvent être unis avec une liaison ou -(CH₂)ₘ- pour produire un cycle cycloalcane,
R³ et R⁴ sont indépendamment hydrogène, alkyle, hydroxyle, alkoxy, cyano, fluoro, et si R³ et R⁴ sont alkyle, R³ et R⁴ peuvent être unis avec une liaison ou -(CH₂)ₘ- pour produire un cycle cycloalcane, Q peut être hydrogène, alkyle, cycloalkyle, cycloalcényle, alkoxy, alkoxy substitué, thio substitué, cyano, thionitrile, sulforiamide, sulfonamide substitué, sulfonyle substitué, aromatique, aromatique substitué, hétéroaromatique, hétéroaromatique substitué, ou bicyclohétéroaromatique,
R⁵ est hydrogène, alkyle, cycloalkyle, ou, lorsque R⁵ est alkyle, peut former avec
R⁶ un cycle hétérocycloalcane,
R⁶ peut être hydrogène, alkyle, alkyle substitué, ou avec R⁵, lorsque R⁵ est alkyle, peut former un cycle hétérocycloalcane ou -OR⁷,
R⁷ est alkyle ou, lorsque R⁵ est alkyle, peut former avec R⁵ un cycle à 5, 6 ou 7 chaînons,
L peut être -O-, -S-, -N= ou absent,
Z peut être carbone ou azote ou absent,
m= 1, 2 ou 3,
n = 0, 1 ou 2, et lorsque n = 0,
Q peut être directement lié à Z ;
à condition que, lorsque les composés de la formule où R⁵ et R⁶ forment ensemble un cycle morpholino et L est absent, ni R¹ ni R² ne puisse alors être -C=C-H ; et
lorsque les composés de la formule où R⁵ est cyclopropyle, R¹, R², R³, R⁴ et R⁶ puissent ne pas tous être hydrogène, ou Q puisse ne pas être méta-fluorophényle,
où le terme « alkyle » est utilisé ici pour désigner un radical monovalent entièrement saturé contenant jusqu'à 12 carbones et un hydrogène, et qui peut être une chaîne droite ou ramifiée ; le terme « cycloalkyle » est utilisé ici pour désigner un radical monovalent entièrement saturé contenant jusqu'à 8 carbones et un hydrogène en cycle ; et le terme « alcényle" est utilisé ici pour désigner un radical monovalent contenant un carbone et un hydrogène qui contient un ou deux sites d'insaturation, et qui peut être une chaîne droite, ramifiée ou cyclique ; ou un sel d'addition pharmaceutiquement acceptable d'un acide ou d'une base de celui-ci.

2. Composé selon la revendication 1 ci-dessus, dans lequel :
R⁵ est hydrogène, alkyle, cycloalkyle ou peut former, avec R⁶, un cycle hétérocycloalcane, et L peut être -O-, -S-, ou -N= ;
à condition que, lorsque R⁵ est cyclopropyle, R¹, R², R³, R⁴ et R⁶ puissent ne pas tous être hydrogène, ou Q puisse ne pas être méta-fluorophényle.

3. Composé selon la revendication 1 ci-dessus, dans lequel :
R⁵ est hydrogène, alkyle, cycloalkyle ou peut former, avec R⁶, un cycle hétérocycloalcane, et L est absent ;
à condition que, lorsque R⁵ et R⁶ forment ensemble un cycle morpholino, ni R¹ ni R² ne puisse être -C=C-H.

4. Composé selon la revendication 2 ci-dessus, dans lequel :
L peut être -O- ou -N=.

5. Composé selon la revendication 4 ci-dessus, dans lequel :
Z est carbone, et
n = 1 ou 2.

6. Composé selon la revendication 5 ci-dessus, dans lequel:
n = 1.

7. Composé selon la revendication 4 ci-dessus, dans lequel:
Z est carbone,
n = 0, et
Q est directement lié à Z.

8. Composé selon la revendication 4 ci-dessus, dans lequel :
L est -O-,
Z est carbone, et
n = 1 ou 2.

9. Composé selon la revendication 4 ci-dessus, dans lequel:
L est -N=
Z est carbone, et
n = 1 ou 2.

10. Composé selon la revendication 1 ci-dessus, dans lequel:
R¹ et R² sont indépendamment hydrogène, alkyle, alkyle substitué, cycloalky alkynyle, alkynyle substitué, cyano, et si R¹ et R² sont alkyle, R¹ et R² peuvent être unis avec une liaison ou -(CH₂)ₘ- pour produire un cycle cycloalcane,
R³ et R⁴ sont indépendamment hydrogène, alkyle, hydroxyle, fluoro, et si R³ et R⁴ sont alkyle, R³ et R⁴ peuvent être unis avec une liaison ou -(CH₂)ₘ- pour produire un cycle cycloalcane,
Q peut être hydrogène, alkyle, cycloalkyle, cycloalcényle, aromatique,
aromatique substitué, hétéroaromatique, hétéroaromatique substitué, ou
bicyclohétéroaromatique,
R⁵ est alkyle, cycloalkyle ou peut former, avec R⁶, un cycle hétérocycloalcane,
R⁶ peut être alkyle, alkyle substitué, ou -OR⁷,
L peut être -O-, -S- ou -N=,
Z peut être carbone ou azote,
m = 1, 2 ou 3,
n = 0, 1 ou 2, et lorsque n = 0,
Q peut être directement lié avec Z.

11. Composé selon la revendication 10 ci-dessus, dans lequel :
Z est carbone, et
n = 1 ou 2.

12. Composé selon la revendication 11 ci-dessus, dans lequel :
R⁵ et R⁶ forment ensemble un cycle hétérocycloalcane, et
L est -O- ou -N=.

13. Composé selon la revendication 12 ci-dessus, dans lequel :
L est -O-.

14. Composé selon la revendication 11 ci-dessus, dans lequel:
R⁵ est alkyle
R⁶ est -OR⁷, et
R⁷, avec R⁵, forme un cycle à 5 ou 6 chaînons, et
L est -O- ou -N=.

15. Composé selon la revendication 12 ci-dessus, dans lequel :
R¹ est hydrogène,
R² est alkyle, alkyle substitué, cycloalky, alkynyle, alkynyle substitué, ou cyano, et
Q est aromatique, aromatique substitué, hétéroaromatique ou hétéroaromatique substitué.

16. Composé selon la revendication 15 ci-dessus, dans lequel:
Q est aromatique substitué, hétéroaromatique ou hétéroaromatique substitué.

17. Composé selon la revendication 15 ci-dessus, dans lequel:
R² est alkyle, cycloalky, alkynyle, ou cyano,
R³ est hydrogène.
R⁴ est alkyle, hydroxyle, fluoro, et
Q est aromatique, aromatique substitué, hétéroaromatique ou hétéroaromatique substitué.

18. Composé selon la revendication 15 ci-dessus, dans lequel :
R² est alkyle, cycloalky, alkynyle, ou cyano,
R³ et R⁴ sont indépendamment alkyle, hydroxyle, fluoro, et si R³ et R⁴ sont alkyle,
R³ et R⁴ peuvent être unis avec une liaison ou -(CH₂)ₘ- pour produire un cycle cycloalcane.

19. Composé selon la revendication 15 ci-dessus, dans lequel :
R² est alkyle, cycloalky, alkynyle, ou cyano,
R³ et R⁴ sont indépendamment alkyle, hydroxyle, fluoro, et si R³ et R⁴ sont alkyle,
R³ et R⁴ peuvent être unis avec une liaison ou -(CH₂)ₘ- pour produire un cycle cycloalcane,

20. Composé selon la revendication 1, selon une des structures suivantes :
3-[2-(3-Fluorophényl)éthyl]-6a,7,8,9-tétrahydro-3*H*-pyrrolo[2',1':2,3][1,3]oxazino [6,5-g][1,2,3]benzotriazine-4,11-dione ;
3-(4,11-Dioxo-4,6a,7,8,9,11-hexahydro-3*H*-pyrrolo[2',1',2,3][1,3]oxazino[6,5-g] [1,2,3]benzotrïazin-3-yl)propanenitrile ;
3-Cyclobutyl-6a,7,8,9-tétrahydro-3*H*-pyrrolo[2',1':2,3][1,3]oxazino[6,5-g][1,2,3] benzotriazine-4,11-dione;
3-Cyclopropyl-6a,7,8,9-tétrahydro-3*H*-pyrrolo[2',1':2,3][1,3]oxazino[6,5-g][1,2,3] benzotriazine-4,11-dione ;
3-Éthyl-6a,7,8,9-tétrahydro-3*H*-pyrrolo[2',1':2,3][1,3]oxazino[6,5-g] [1,2,3]benzotriazine-4,11-dione :
3-(Cyclopropylméthyl)-6a,7,8,9-tétrahydro-3*H*-pyrrolo[2',1':2,3][1,3]oxazino [6,5-g][1,2,3]benzotriazine-4,11-dione ;
3-*tert*-Butyl-6a,7,8,9-tétrahydro-3*H*-pyrrolo[2',1':2,3][1,3]oxazino[6,5-g][1,2,3] benzotriazine-4,11-dione ;
3-(Diméthylamino)-6a,7,8,9-tétrahydro-3*H*-pyrrolo[2',1':2,3][1,3]oxazino[6,5-g] [1,2,3]benzotriazine-4,11-dione ;
3-Prop-2-yn-1-yl-6a,7,8,9-tétrahydro-3*H*-pyrrolo[2',1':2,3][1,3]oxazino[6,5-g] [1,2,3]benxotriaxine-4,11-dione ;
(4,11-Dioxo-4,6a,7,6,9,11-hexahydro-3*H*-pyrrolo[2',1':2,3][1,3]oxazino[6,5-g] [1,2,3]benzotriazin-3-yl)acétonitrile ;
2-(4,11-Dioxo-4,6a7,8,9,11-hexahydro-3*H*-pyrrolo[2',1':2,3][1,3]oxazino[6,5-g] [1,2,3]benzotriazin-3-yl)propanenitrile ;
2-(4,11-Dioxo-4,8a,7,8,9,11-hexahydro-3*H*-pyrrolo(2',1':2,3][1,3]oxazino[6,5-g] [1,2,3]benzotriazin-3-yl)-2-méthylpropanenitrile ;
3-[(2-Méthyl-2*H*-tétrazol-5-yl)méthyl]-6a,7,8,9-tétrahydro-3*H*pyrrolo[2',1':2,3] [1,3]oxazino[6,5-g][1,2,3]benzytriazine-4,11-dione et
3-[(1-méthyl-1*H*-tétrazol-5-yl)méthyl]-6a,7,8,9-tétrahydro-3*H*-pyrrolo[2',1':2,3] [1,3]oxazino[6,5-g][1,2,3]benzotriazine-4,11-dione ;
3-(2-Cyclohex-1-en-1-yléthyl)-6a,7,8,9-tétrahydro-3*H*-pyrroly[2',1':2,3][1,3] oxazino[6,5-g][1,2,3]benzotriazirie-4,11-dione ;
3-(2-Cyclohexyléthyl)-6a,7,8,9,tétrahydro-3*H*-pyrrolo[2',1':2,3][1,3]oxazino[6,5-g] [1,2,3]benzotriazine-4,11-dione ;
(6a*R*)-3-[(2*S*)-1-(3,5-Difluorophényl)but-3-yn-2-yl]-8a,7,8,9-tétrahydro-3*H-*pyrrolo[2',1':2,3][1,3]oxazin[6,5-g][1,2,3]benzotriazine-4,11-dione ;
3-[(2*S*)-1-(3-Fluorophényl)but-3-yn,2-yl]-6a,7,8,9-tétrahydro-3*H*-pyrrolo[2',1':2,3] [1,3]oxazino[6,5-g][1,2,3]benzotriazine-4,11-dione ;
3-[(1*S*)-2-(3-Fluorophényl)-1-isoxazol-3-yléthyl]-6a,7,8,9-tétrahydro-3*H*-pyrrolo [2',1':2,3][1,3]oxazino[6,5-g][1,2,3]benzotriazine-4,11-dione ;
(2*S*)-2-[(6a*S*)-4,11-Dioxo-4,6a,7,8,9,11-hexahydro-3*H*-pyrrolo[2',1':2,3][1,3] oxazino[6,5-g][1,2,3]benzotriazin-3-yl]-3-(3-fluorophényl)-*N-*méthylpropanamide ;
(2S)-2-[(8a*R*)-4,11-dioxo-4,6a,7,8,9,11-hexahydro-3*H*-pyrrolo[2',1':2,3][1,3] oxazino[6,5-g][1,2,3]benzotriazin-3-yl]-3-(3-fluorophényl)-*N-*méthylpropanamide :
(2*S*)-2-[(6a*S*)-4,11-Dioxo-4,6a,7,8,9,11-hexahydro-3*H*-pyrrolo[2',1':2,3][1,3] oxazino[6,5-g][1,2,3]benzotriazin-3-yl]-3-(3-fluorophényl)propanamide ;
(2*S*)-2-[(6a*R*)-4,11-dioxo-4,8a,7,8,9,11-hexahydro-3*H*-pyrrolo[2',1':2,3][1,3] oxazino[6,5-g][1,2,3]benzotriazin-3-yl]-3-(3-fluorophényl)propanamide ;
(2*S*)-2-[(6a*R*)-4,11-Dioxo-4,6a,7,8,9,11-hexahydro-3*H*-pyrralo[2',1';2,3][1,3] oxazino[6,5 g][1,2,3]benzotriazin-3-yl]-3(3-fluorophényl)propanenitrile ;
(6a*S*)-3[(2*S*)-1-(3-Fluorophényl)-3-hydroxypropan-2-yl)-6a,7,8,9-tétrahydro-3*H-*pyrrolo[2',1':2,3][1,3]oxazirio[6,5-g][1,2,3]benzotriazine-4,11-dione ;
(6aR)-3-[(2S)-1-(3-fluorophényl)-3-hydroxypropan-2-yl]-6a,7,8,9-tétrahydro-3H-pyrrolo[2',1':2,3][1,3]oxazino[6,5-g][1,2,3]benzotriazine-4,11-dione ;
3-[1-(3-Nitrophényl)but-3-yn-2-yl}-6a,7,8,9-tétrahydro-3*H*-pyrrolo[2',1';2,3][1,3] oxazino[6,5-g][1,2,3]benzotriazine-4,11-dione ;
3-[(1*S*,2*R*)-1-Hydroxy-1-phénylpropan-2-yl]-6a,7,8,9-tétrahydro-3*H*-pyrrolo[2',1': 2,3][1,3]oxazino[6,5-g][1,2,3]benzotriazine-4,11-dione ;
Érythro-3-[1-(3-fluorophényl)-1-hydroxypropan-2-yl]-6a,7,8,9-tétrahydro-3*H-*pyrrolo[2',1':2,3][1,3]oxazino[6,5-g][1,2,3]benzotriazine-4,11-dione ;
3-{[1-(3-Fluorophényl)cyclopropyl]méthyl}-6a,7,8,9-tétrahydre-3*H*-pyrrolo [2',1':2,3][1,3]oxazino[6,5-g][1,2,3]benzotriazine-4,11-dione ;
3-[(2*R*)-1-(3-Fluorophényl)butan-2-yl]-6a,7,8,9-tétrahydro-3*H*-pyrrolo[2',1':2,3] [1,3]oxazino[6,5-g][1,2,3]benzotriazine-4,11-dione ;
3-[(1*R*)-1-Phényléthyl]-6a,7,8,9-tétrahydro-3*H*-pyrrolo[2',1':2,3][1,3]oxazino [6,5-g][1,2,3]benzotriazine-4,11-dione ;
(6a*S*)-3-[(1*R*)-1-(3-Fluorophényl)éthyl]-6a,7,8,9-tétrahydro-3*H*-pyrrolo[2',1';2,3] [1,3]oxazino[6,5-g][1,2,3]benzotriazine-4,11-dione ;
(6a*R*)-3-[(1*R*)-1-(3-fluorophényl)éthyl]-6a,7,8,9-tétrahydro-3*H*-pyrrolo[2',1':2,3] [1,3]oxazino[6,5-g][1,2,3]benzotriazine-4,11-dione :
(6a*S*)-3-[(1*R*)-1-(3,5-Difluorophényl)éthyl]-6a,7,8,9-tétrahydro-3*H*-pyrrolo [2',1':2,3][1,3]oxazino[6,5-g][1,2,3]benzotriazine-4,11-dione ;
(6a*R*)-3-[(1*R*)-1-(3,5-difluorophényl)éthyl]-6a,7,8,9-tétrahydro-3*H*-pyrrolo [2',1':2,3][1,3]oxazino[6,5-g][1,2,3]benzotriazine-4,11-dione ;
3-(2,5-Difluorobenzyl)-6a,7,8,9-tétrahydro-3*H*-pyrrolo[2',1':2,3][1,3]oxazino [6,5-g][1,2,3]benzotriazine-4,11-dione ;
3-(1-Pyridin-3-yléthyl)-6a,7,8,9-tétrahydro-3*H*-pyrrolo[2',1':2,3][1,3]oxazino [6,5-g][1,2,3]benzotriazine-4,11-dione ;
3-(1-Pyridin-4-yléthyi)-6a,7,8,9-tétrahydro-3*H*-pyrrolo[2',1':2,3][1,3]oxazino [6,5-g][1,2,3]benzotriazine-4,11-dione ;
3-[1-(1,3-Thiazol-2-yl)éthyl]-6a,7,8,9-tétrahydro-3*H*-pyrrolo[2',1':2,3][1,3] oxazino[6,5-g][1,2,3]benzotriazine-4,11-dione ;
(2*R*)-2-(4,11-Dioxo-4,6a,7,8,9,11-hexahydro-3*H*-pyrrolo[2',1':2,3][1,3]oxazino [6,5-g][1,2,3]benzotriazin-3-yl)propyl thiocyanate ;
3-[2-(1*H*-Pyrazol-1-yl)éthyl]-6a,7,8,9-tétrahydro-3*H*-pyrrolo[2',1':2,3][1,3]oxazino [6,5-g][1,2,3]benzotriazine-4,11-dione ;
3-[(2*R*)-1-(2*H*-Tétrazol-2-yl)propan-2-yl]-6a,7,8,9-tétrahydro-3*H*-pyrrolo [2',1':2,3][1,3]oxazino[6,5-g][1,2,3]benzotriazine-4,11-dione ;
3-[(2*R*)-1-(1*H*-tétrazol-1-yl)propan-2-yl]-6a,7,8,9-tétrahydro-3*H*-pyrrolo[2',1':2,3] [1,3]oxazino[6,5-g]benzotriazine-4-11-dione
6a,7,8,9-Tétrahydro-3*H*-pyrrolo[2',1':2,3][1,3]oxazino[6,5-g][1,2,3] benzotriazine-4,11-dione ;
3-(1-Phénylpent-3-yn-2-yl)-6a,7,8,9-tétrahydro-3*H*-pyrrolo[2',1':2,3][1,3]oxazino [6,5-g][1,2,3]benzotriazine-4,11-dione ;
3-Méthyl-6a,7,8,9-tétrahydro-3*H*-pyrrolo[2',1':2,3][1,3]oxazino[6,5-g][1,2,3] benzotriazine-4,11-dione ;
3-(3-Fluorobenzyl)-6a,7,8,9-tétrahydro-3*H*-pyrrolo[2',1':2,3][1,3]oxazino[6,5-g] [1,2,3]benzotriazine-4,11-dione ;
3-[3-(3-Fluorophényl)propyl]-6a,7,8,9-tétrahydro-3*H*-pyrrolo[2',1':2.3][1,3] oxazino[6,5-g][1,2,3]benzotriazine-4,11-dione ;
3-(1,3-Benzothiazol-2-ylméthyl)-6a,7,8,9-tétrahydro-3*H*-pyrrolo[2',1':2,3][1,3] oxazino[6,5-g][1,2,3]benzothazine-4,11-dione ;
3-(2,1,3-Benzoxadiazol-5-ylméthyl)-6a,7,8,9-tétrahydro-3*H*-pyrrolo[2',1':2,3][1,3] oxazino[6,5-g][1,2,3]benzotriazine-4,11-dione ;
3-[(4,11-Dioxo-4,6a,7,8,9,11-hexahydro-3*H*-pyrrolo[2',1':2,3][1,3]oxaxino[6,5-g] [1,2,3]benzotriazin-3-yl)méthyl]benzonitrile ;
2-[(4,11-Dioxo-4,6a,7,8,9,11-hexahydro-3*H*-pyrrolo[2',1':2,3][1,3]oxazino[6,5-g] [1,2,3]benzotriazin-3-yl)méthyl]benzonitrile;
3-(Pyridin-3-ylméthyl)-6a,7,8,9-tétrahydro-3*H*-pyrrolo[2',1':2,3][1,3]oxazino [6,5-g][1,2,3]benzotriazine-4,11-dione ;
3-(Pyridin-2-ylméthyl)-6a,7,8,9-tétrahydro-3*H*-pyrrolo[2',1':2,3][1,3]oxazino [6,5-g][1,2,3]benzotriazine-4,11-dione ;
3-(Pyrazin-2-ylméthyl)-6a,7,8,9-tétrahydro-3*H*-pyrrolo[2',1':2,3][1,3]oxazino [6,5-g][1,2,3]benzotriazine-4,11-dione ;
3-[(4-6romo-1*H*-pyraxol-1-yl)méthyl]-6a,7,8,9-tétrahydro-3*H*-pyrrolo[2',1':2,3] [1,3]oxazino[6,5-g][1,2,3]benzotriazine-4,11-dione ;
3-[2-(3-Fluorophényl)éthyl]-8,9-dihydro-3*H*-[1,3]oxazolo[2',3':2,3][1,3]oxazino [6,5-g][1,2,3]benzotriazine-4,11-dione ;
8-Éthyl-3-[2-(3-fluorophényl)éthyl]-7,8-dihydro-3*H*-[1,3]oxazino[6,5-g][1,2,3] benzotriazine-4,9-dione ;
8-*tert*-Butyl-3-[(2*R*)-1-(2*H*-tetrazol-2-yl)propan-2-yl]-7,8-dihydro-3*H*-[1,3]oxazino [6,5-g][1,2,3]benzotriazine-4,9-dione ;
8-*tert*-Butyl-3-[(2*R*)-1-(1*H*-tétrazol-1-yl)propan-2-yl]-7,8-dihydro-3*H*-[1,3]oxazino [6,5-g][1,2,3]benzotriazine-4,9-dione ;
8-Cyclopropyl-3-(2-méthoxyéthyl)-7,8-dihydro-3*H*-[1,3]oxazino[6,5-g][1,2,3] benzotriazine-4,9-dione ;
8-Cyclopropyl-3-[(2*S*)-1-(3-fluorophényl)but-3-yn-2-yl]-7,8-dihydro-3*H*-[1,3] oxazino[6,5-g][1,2,3]benzotriazine-4,9-dione ;
8-Cyclopropyl-3-[(2*R*)-1-(2*H*-tétrazol-2-yl)propan-2-yl]-7,8-dihydro-3*H*-[1,3] oxazino[6,5-g][1,2,3]benzotriazine-4,9-diona ;
8-Cyclopropyl-3-[(2*R*)-1-(1*H*-tétrazol-1-yl)propan-2-yl]-7,8-dihydro-3*H*-[1,3] oxazino[6,5-g][1,2,3]benzotriazine-4,9-dione ;
8-Cyclopropyl-3-[(2*R*)-1-(4-nitro-1*H*-pyrazol-1-yl)propan-2-yl]-7,8-dihydro-3*H-*[1,3]oxazino[6,5-g][1,2,3]benzotriazine-4,9-dione ;
8-Cyclopropyl-3-[(2*R*)-1-(2*H*-tétrazol-2-yl)propan-2-yl]-3,8-dihydro [1,2,3]triazino[4,5-g]quinazoline-4,9-dione ;
3-[2-(3-Fluorophényl)éthyl]-8,9-dihydro-3*H*-[1,3]oxazolo[2,3-*b*][1,2,3]triazino [4,5-g]quinazolïne-4,11-dione ;
3-[(2*S*)-1-(3-Fluorophényl)but-3-yn-2-yl)-8,9-dihydro-3*H*-[1,3]oxazolo[2,3-*b*] [1,2,3]triazino[4,5-g]quinazoline-4,11-dione ;
3-[(2R)-1-(2H-Tétrazol-2-yl)propan-2-yl]-8,9-dihydro-3H-[1,3]oxazolo[2,3-b] [1,2,3]triazino[4,5-g]quinazoline-4,11-dione ;
(4,11-Dioxo-4,8,9,11-tétrahydro-3*H*-[1,3]oxazolo[2,3-*b*][1,2,3]triazino[4,5-g] quinazolin-3-yl)acétoriitrile :
3-[(2*R*)-1-(2*H*-Tétrazol-2-yl)propan-2-yl]-6a,7,9,10-tétrahydro-3*H*-[1,4]oxazino [3',4':2,3][1,3]oxazino[6,5-g][1,2,3]benzotriazine-4,12-dione ;
3-[(2*R*)-1-(1*H*-Tétrazol-1-yl)propan-2-yl]-6a,7,9,10-tétrahydro-3*H*-[1,4]oxazino [3',4':2,3][1,3]oxazino[6,5-g][1,2,3]benzotriazine-4,12-dione ; ou
3-[2-(3-Fluorophényl)éthyl]-7-(morpholin-4-ylcarbonyl)-1,2,3-benzotriazin-4(3*H*)-one, ou un sel d'addition pharmaceutiquement acceptable d'un acide ou d'une base de celui-ci.

21. Composition pharmaceutique comprenant une quantité efficace d'un composé selon l'une quelconque des revendications de 1 à 20 en association avec un porteur, additif ou excipient pharmaceutiquement acceptable.

22. Composition selon la revendication 21, dans laquelle ledit composé comprend entre 0,5 % et 75 % environ en poids de ladite composition et ledit porteur, additif ou excipient comprend entre 25 % et 95,5 % environ de ladite composition.

23. Utilisation d'un composé selon l'une quelconque des revendications de 1 à 20 dans la fabrication d'un médicament destiné à être utilisé dans le traitement d'un sujet mammifère, où le sujet souffre d'un état hypoglutamatergique ou d'une déficience du nombre ou de la force des synapses excitatrices ou du nombre de récepteurs AMPA, de sorte que la mémoire ou d'autres fonctions cognitives sont détériorées.

24. Utilisation d'un composé selon l'une quelconque des revendications de 1 à 20 dans la fabrication d'un médicament destiné à être utilisé dans le traitement de la schizophrénie.

25. Utilisation d'un composé selon l'une quelconque des revendications de 1 à 20 dans la fabrication d'un médicament destiné à être utilisé dans le traitement de la maladie de Parkinson.

26. Utilisation d'un composé selon l'une quelconque des revendications de 1 à 20 dans la fabrication d'un médicament destiné à être utilisé dans le traitement du trouble déficitaire de l'attention avec hyperactivité.

27. Utilisation d'un composé selon l'une quelconque des revendications de 1 à 20 dans la fabrication d'un médicament destiné à être utilisé dans le traitement du syndrome de Rett.

28. Utilisation d'un composé selon l'une quelconque des revendications de 1 à 20 dans la fabrication d'un médicament destiné à être utilisé dans le traitement du syndrome du X firagile.

29. Utilisation d'un composé selon l'une quelconque des revendications de 1 à 20 dans la fabrication d'un médicament destiné à être utilisé dans le traitement de la dépression respiratoire.

30. Utilisation d'un composé selon l'une quelconque des revendications de 1 à 20 dans la fabrication d'un médicament destiné à être utilisé dans le traitement des troubles cognitifs.

31. Utilisation d'un composé selon l'une quelconque des revendications de 1 à 20 dans la fabrication d'un médicament pour le traitement de la maladie d'Alzheimer.

32. Utilisation d'un composé selon l'une quelconque des revendications de 1 à 20 dans la fabrication d'un médicament pour le traitement d'un sujet mammifère, où le sujet souffre d'un état hypoglutamatergique ou de déficiences du nombre ou de la force des synapses excitatrices ou du nombre de récepteurs VAMPA, de sorte qu'un déséquilibre cortical/striatal apparaît, conduisant à la schizophrénie ou à un comportement schizophrénifiorme.
